# EUROPEAN PATENT APPLICATION

(11) **EP 3 756 687 A2**
(43) Date of publication of application: **30.12.2020**
(21) Application number: 20177089.8
(22) Date of filing: 13.12.2013
(51) Int. Cl.: A61K 39/395, A61K 39/00

(54) **TARGET PEPTIDES FOR OVARIAN CANCER THERAPY AND DIAGNOSTICS**

(30) Priority: 13.12.2012 US 201261736815 P
(62) Document number(s) of the earlier application(s) in accordance with Art. 76 EPC: 13862491.1 / 2 931 312
(71) Applicant: University Of Virginia Patent Foundation, Charlottesville, VA 22902 (US); The Board of Regents of the University of Oklahoma, Oklahoma City OK 73019 (US)
(72) Inventor: HUNT, Donald F., Charlottesville, VA 22901 (US); SHABANOWITZ, Jeffrey, Charlottesville, VA 22904-4319 (US); ABELIN, Jennifer G., Sommers, CT 06071 (US); HILDEBRAND, William H., Edmond, OK 73034 (US); PATTERSON, Andrea M., Indianapolis, IN 46202 (US)
(74) Representative: Isarpatent

(57) **Abstract**

A set of target peptides are presented by HLA A*0201 on the surface of ovarian cancer cells. They are envisioned to among other things (a) stimulate an immune response to the proliferative disease, e.g., ovarian cancer, (b) function as immunotherapeutics in adoptive T-cell therapy or as a vaccine, (c) facilitate antibody recognition of tumor boundaries in surgical pathology samples, (d) act as biomarkers for early detection and/or diagnosis of the disease, and (e) act as targets in the generation antibody-like molecules which recognize the target-peptide/MHC complex.

SEQUENCE LISTING

<110>  University of Virginia Patent Foundation
       The Board of Regents of the University of Oklahoma

<120>  TARGET PEPTIDES FOR OVARIAN CANCER THERAPY AND DIAGNOSTICS

<130>  P38789-EP01

<150>  US 61/736,815
<151>  2012-12-13

<160>  243

<170>  PatentIn version 3.5

<210>  1
<211>  9
<212>  PRT
<213>  Homo sapiens

<220>
<221>  MISC_FEATURE
<222>  (4)..(4)
<223>  The amino acid at this position is optionally phosphorylated

<400>  1

Ala Ile Leu Ser Pro Ala Phe Lys Val
1               5

<210>  2
<211>  9
<212>  PRT
<213>  Homo sapiens

<220>
<221>  MISC_FEATURE
<222>  (5)..(5)
<223>  The amino acid at this position is optionally phosphorylated

<400>  2

Ala Ile Met Arg Ser Pro Gln Met Val
1               5

<210>  3
<211>  11
<212>  PRT
<213>  Homo sapiens

<220>
<221>  MISC_FEATURE
<222>  (4)..(4)
<223>  The amino acid at this position is optionally phosphorylated

<400>  3

Ala Leu Asp Ser Gly Ala Ser Leu Leu His Leu
1               5                   10

<210>  4
<211>  9
<212>  PRT
<213>  Homo sapiens

<220>
<221>  MISC_FEATURE
<222>  (5)..(5)
<223>  The amino acid at this position is optionally phosphorylated

<400>  4

Ala Leu Gly Asn Thr Pro Pro Phe Leu
1               5

<210>  5
<211>  9
<212>  PRT
<213>  Homo sapiens

<220>
<221>  MISC_FEATURE
<222>  (4)..(4)
<223>  The amino acid at this position is optionally phosphorylated

<400>  5

Ala Leu Leu Ser Leu Leu Lys Arg Val
1               5

<210>  6
<211>  9
<212>  PRT
<213>  Homo sapiens

<220>
<221>  MISC_FEATURE
<222>  (5)..(5)
<223>  The amino acid at this position is optionally phosphorylated

<400>  6

Ala Met Ala Ala Ser Pro His Ala Val
1               5

<210>  7
<211>  13
<212>  PRT
<213>  Homo sapiens

<220>
<221>  MISC_FEATURE
<222>  (7)..(7)
<223>  The amino acid at this position is optionally phosphorylated

<400>  7

Ala Met Leu Gly Ser Lys Ser Pro Asp Pro Tyr Arg Leu
1               5                   10

<210>  8
<211>  10
<212>  PRT
<213>  Homo sapiens

<220>
<221>  MISC_FEATURE
<222>  (4)..(4)
<223>  The amino acid at this position is optionally phosphorylated

<400>  8

Ala Thr Trp Ser Gly Ser Glu Phe Glu Val
1               5                   10

<210>  9
<211>  11
<212>  PRT
<213>  Homo sapiens

<220>
<221>  MISC_FEATURE
<222>  (4)..(4)
<223>  The amino acid at this position is optionally phosphorylated

<400>  9

Ala Val Val Ser Pro Pro Ala Leu His Asn Ala
1               5                   10

<210>  10
<211>  9
<212>  PRT
<213>  Homo sapiens

<220>
<221>  MISC_FEATURE
<222>  (4)..(4)
<223>  The amino acid at this position is optionally phosphorylated

<400>  10

Asp Leu Arg Thr Val Glu Lys Glu Leu
1               5

<210>  11
<211>  10
<212>  PRT
<213>  Homo sapiens

<220>
<221>  MISC_FEATURE
<222>  (6)..(6)
<223>  The amino acid at this position is optionally phosphorylated

<400>  11

Asp Leu Trp Lys Ile Thr Lys Val Met Asp
1               5                   10

<210>  12
<211>  9
<212>  PRT
<213>  Homo sapiens

<220>
<221>  MISC_FEATURE
<222>  (5)..(5)
<223>  The amino acid at this position is optionally phosphorylated

<400>  12

Glu Leu Phe Ser Ser Pro Pro Ala Val
1               5

<210>  13
<211>  10
<212>  PRT
<213>  Homo sapiens

<220>
<221>  MISC_FEATURE
<222>  (7)..(7)
<223>  The amino acid at this position is optionally phosphorylated

<400>  13

Glu Leu Arg Ile Ser Gly Ser Val Gln Leu
1               5                   10

<210>  14
<211>  11
<212>  PRT
<213>  Homo sapiens

<220>
<221>  MISC_FEATURE
<222>  (4)..(4)
<223>  The amino acid at this position is optionally phosphorylated

<400>  14

Phe Ile Gly Ser Pro Thr Thr Pro Ala Gly Leu
1               5                   10

<210>  15
<211>  9
<212>  PRT
<213>  Homo sapiens

<220>
<221>  MISC_FEATURE
<222>  (5)..(5)
<223>  The amino acid at this position is optionally phosphorylated

<400>  15

Phe Leu Asp Asn Ser Phe Glu Lys Val
1               5

<210>  16
<211>  11
<212>  PRT
<213>  Homo sapiens

<220>
<221>  MISC_FEATURE
<222>  (7)..(7)
<223>  The amino acid at this position is optionally phosphorylated

<400>  16

Phe Leu Asp Arg Pro Pro Thr Pro Leu Phe Ile
1               5                   10

<210>  17
<211>  9
<212>  PRT
<213>  Homo sapiens

<220>
<221>  MISC_FEATURE
<222>  (4)..(4)
<223>  The amino acid at this position is optionally phosphorylated

<400>  17

Phe Leu Asp Ser Leu Arg Asp Leu Ile
1               5

<210>  18
<211>  12
<212>  PRT
<213>  Homo sapiens

<220>
<221>  MISC_FEATURE
<222>  (8)..(8)
<223>  The amino acid at this position is optionally phosphorylated

<400>  18

Phe Leu Phe Asp Lys Pro Val Ser Pro Leu Leu Leu
1               5                   10

<210>  19
<211>  9
<212>  PRT
<213>  Homo sapiens

<220>
<221>  MISC_FEATURE
<222>  (8)..(8)
<223>  The amino acid at this position is optionally phosphorylated

<400>  19

Phe Leu Gly Val Arg Pro Lys Ser Ala
1               5

<210>  20
<211>  14
<212>  PRT
<213>  Homo sapiens

<220>
<221>  MISC_FEATURE
<222>  (10)..(10)
<223>  The amino acid at this position is optionally phosphorylated

<400>  20

Phe Leu Ile Thr Gly Gly Gly Lys Gly Ser Gly Phe Ser Leu
1               5                   10

<210>  21
<211>  10
<212>  PRT
<213>  Homo sapiens

<220>
<221>  MISC_FEATURE
<222>  (4)..(4)
<223>  The amino acid at this position is optionally phosphorylated

<400>  21

Phe Leu Leu Ser Gln Asn Phe Asp Asp Glu
1               5                   10

<210>  22
<211>  11
<212>  PRT
<213>  Homo sapiens

<220>
<221>  MISC_FEATURE
<222>  (4)..(4)
<223>  The amino acid at this position is optionally phosphorylated

<400>  22

Gly Ala Leu Ser Pro Ser Leu Leu His Ser Leu
1               5                   10

<210>  23
<211>  9
<212>  PRT
<213>  Homo sapiens

<220>
<221>  MISC_FEATURE
<222>  (5)..(5)
<223>  The amino acid at this position is optionally phosphorylated

<400>  23

Gly Leu Ala Pro Thr Pro Pro Ser Met
1               5

<210>  24
<211>  10
<212>  PRT
<213>  Homo sapiens

<220>
<221>  MISC_FEATURE
<222>  (4)..(4)
<223>  The amino acid at this position is optionally phosphorylated

<400>  24

Gly Leu Asp Ser Leu Asp Gln Val Glu Ile
1               5                   10


<210>  25
<211>  9
<212>  PRT
<213>  Homo sapiens


<220>
<221>  MISC_FEATURE
<222>  (8)..(8)
<223>  The amino acid at this position is optionally phosphorylated


<400>  25

Gly Leu Gly Glu Leu Leu Arg Ser Leu
1               5


<210>  26
<211>  10
<212>  PRT
<213>  Homo sapiens


<220>
<221>  MISC_FEATURE
<222>  (4)..(4)
<223>  The amino acid at this position is optionally phosphorylated


<400>  26

Gly Leu Ile Ser Pro Glu Leu Arg His Leu
1               5                   10


<210>  27
<211>  9
<212>  PRT
<213>  Homo sapiens


<220>
<221>  MISC_FEATURE
<222>  (4)..(4)
<223>  The amino acid at this position is optionally phosphorylated


<400>  27

Gly Leu Ile Ser Pro Asn Val Gln Leu
1               5


<210>  28
<211>  9
<212>  PRT
<213>  Homo sapiens

<220>
<221>  MISC_FEATURE
<222>  (4)..(4)
<223>  The amino acid at this position is optionally phosphorylated

<400>  28

Gly Leu Ile Ser Pro Val Trp Gly Ala
1               5

<210>  29
<211>  11
<212>  PRT
<213>  Homo sapiens

<220>
<221>  MISC_FEATURE
<222>  (4)..(4)
<223>  The amino acid at this position is optionally phosphorylated

<400>  29

Gly Leu Ile Thr Pro Gly Gly Phe Ser Ser Val
1               5                   10

<210>  30
<211>  9
<212>  PRT
<213>  Homo sapiens

<220>
<221>  MISC_FEATURE
<222>  (5)..(5)
<223>  The amino acid at this position is optionally phosphorylated

<400>  30

Gly Leu Leu Asp Ser Pro Thr Ser Ile
1               5

<210>  31
<211>  9
<212>  PRT
<213>  Homo sapiens

<220>
<221>  MISC_FEATURE
<222>  (5)..(5)
<223>  The amino acid at this position is optionally phosphorylated

<400>  31

Gly Leu Leu Gly Ser Pro Ala Arg Leu
1               5


<210>  32
<211>  9
<212>  PRT
<213>  Homo sapiens


<220>
<221>  MISC_FEATURE
<222>  (5)..(5)
<223>  The amino acid at this position is optionally phosphorylated


<400>  32

Gly Leu Leu Gly Ser Pro Val Arg Ala
1               5


<210>  33
<211>  10
<212>  PRT
<213>  Homo sapiens


<220>
<221>  MISC_FEATURE
<222>  (4)..(4)
<223>  The amino acid at this position is optionally phosphorylated


<400>  33

Gly Leu Leu Ser Pro Arg Phe Val Asp Val
1               5                   10


<210>  34
<211>  9
<212>  PRT
<213>  Homo sapiens


<220>
<221>  MISC_FEATURE
<222>  (4)..(4)
<223>  The amino acid at this position is optionally phosphorylated


<400>  34

Gly Leu Leu Ser Pro Arg His Ser Leu
1               5


<210>  35
<211>  11
<212>  PRT
<213>  Homo sapiens

<220>
<221>  MISC_FEATURE
<222>  (4)..(4)
<223>  The amino acid at this position is optionally phosphorylated

<400>  35

Gly Met Leu Ser Pro Gly Lys Ser Ile Glu Val
1               5                   10

<210>  36
<211>  10
<212>  PRT
<213>  Homo sapiens

<220>
<221>  MISC_FEATURE
<222>  (2)..(2)
<223>  The amino acid at this position is optionally phosphorylated

<400>  36

Gly Ser Gln Leu Ala Val Met Met Tyr Leu
1               5                   10

<210>  37
<211>  9
<212>  PRT
<213>  Homo sapiens

<220>
<221>  MISC_FEATURE
<222>  (4)..(4)
<223>  The amino acid at this position is optionally phosphorylated

<400>  37

Gly Val Ala Ser Pro Thr Ile Thr Val
1               5

<210>  38
<211>  9
<212>  PRT
<213>  Homo sapiens

<220>
<221>  MISC_FEATURE
<222>  (4)..(4)
<223>  The amino acid at this position is optionally phosphorylated

<400>  38

Gly Val Val Ser Pro Thr Phe Glu Leu
1               5

<210>  39
<211>  9
<212>  PRT
<213>  Homo sapiens

<220>
<221>  MISC_FEATURE
<222>  (4)..(4)
<223>  The amino acid at this position is optionally phosphorylated

<400>  39

His Leu His Ser Pro Gln His Lys Leu
1               5

<210>  40
<211>  9
<212>  PRT
<213>  Homo sapiens

<220>
<221>  MISC_FEATURE
<222>  (4)..(4)
<223>  The amino acid at this position is optionally phosphorylated

<400>  40

Ile Leu Gln Thr Pro Gln Phe Gln Met
1               5

<210>  41
<211>  9
<212>  PRT
<213>  Homo sapiens

<220>
<221>  MISC_FEATURE
<222>  (5)..(5)
<223>  The amino acid at this position is optionally phosphorylated

<400>  41

Ile Leu Gln Val Ser Ile Pro Ser Leu
1               5

<210>  42
<211>  11
<212>  PRT
<213>  Homo sapiens

<220>
<221>  MISC_FEATURE
<222>  (4)..(4)
<223>  The amino acid at this position is optionally phosphorylated

<400>  42

Ile Val Leu Ser Asp Ser Glu Val Ile Gln Leu
1               5                   10

<210>  43
<211>  9
<212>  PRT
<213>  Homo sapiens

<220>
<221>  MISC_FEATURE
<222>  (4)..(4)
<223>  The amino acid at this position is optionally phosphorylated

<400>  43

Lys Ala Phe Ser Pro Val Arg Ser Val
1               5

<210>  44
<211>  9
<212>  PRT
<213>  Homo sapiens

<220>
<221>  MISC_FEATURE
<222>  (4)..(4)
<223>  The amino acid at this position is optionally phosphorylated

<400>  44

Lys Ile Ala Ser Glu Ile Ala Gln Leu
1               5

<210>  45
<211>  10
<212>  PRT
<213>  Homo sapiens

<220>
<221>  MISC_FEATURE
<222>  (4)..(4)
<223>  The amino acid at this position is optionally phosphorylated

<400>  45

Lys Ile Glu Ser Leu Glu Asn Leu Tyr Leu
1               5                   10

<210>  46
<211>  9
<212>  PRT
<213>  Homo sapiens

<220>
<221>  MISC_FEATURE
<222>  (4)..(4)
<223>  The amino acid at this position is optionally phosphorylated

<400>  46

Lys Ile Gly Ser Ile Ile Phe Gln Val
1               5

<210>  47
<211>  11
<212>  PRT
<213>  Homo sapiens

<220>
<221>  MISC_FEATURE
<222>  (4)..(4)
<223>  The amino acid at this position is optionally phosphorylated

<400>  47

Lys Leu Ala Ser Leu Glu Arg Glu Ala Ser Val
1               5                   10

<210>  48
<211>  11
<212>  PRT
<213>  Homo sapiens

<220>
<221>  MISC_FEATURE
<222>  (4)..(4)
<223>  The amino acid at this position is optionally phosphorylated

<400>  48

Lys Leu Ala Ser Pro Glu Lys Leu Ala Gly Leu
1               5                   10

<210>  49
<211>  10
<212>  PRT
<213>  Homo sapiens

<220>
<221>  MISC_FEATURE
<222>  (4)..(4)
<223>  The amino acid at this position is optionally phosphorylated

<400>  49

Lys Leu Ala Ser Pro Glu Leu Glu Arg Leu
1               5                   10

<210>  50
<211>  10
<212>  PRT
<213>  Homo sapiens

<220>
<221>  MISC_FEATURE
<222>  (6)..(6)
<223>  The amino acid at this position is optionally phosphorylated

<400>  50

Lys Leu Phe Pro Asp Thr Pro Leu Ala Leu
1               5                   10

<210>  51
<211>  11
<212>  PRT
<213>  Homo sapiens

<220>
<221>  MISC_FEATURE
<222>  (4)..(4)
<223>  The amino acid at this position is optionally phosphorylated

<400>  51

Lys Leu Phe Ser Pro Ser Lys Glu Ala Glu Leu
1               5                   10

<210>  52
<211>  11
<212>  PRT
<213>  Homo sapiens

<220>
<221>  MISC_FEATURE
<222>  (7)..(7)
<223>  The amino acid at this position is optionally phosphorylated

<400>  52

Lys Leu Ile Asp Ile Val Ser Ser Gln Lys Val
1               5                   10

<210>  53
<211>  9
<212>  PRT
<213>  Homo sapiens

<220>
<221>  MISC_FEATURE
<222>  (4)..(4)
<223>  The amino acid at this position is optionally phosphorylated

<400>  53

Lys Leu Lys Ser Gln Glu Ile Phe Leu
1               5

<210>  54
<211>  10
<212>  PRT
<213>  Homo sapiens

<220>
<221>  MISC_FEATURE
<222>  (4)..(4)
<223>  The amino acid at this position is optionally phosphorylated

<400>  54

Lys Leu Leu Ser Pro Ser Asp Glu Lys Leu
1               5                   10

<210>  55
<211>  10
<212>  PRT
<213>  Homo sapiens

<220>
<221>  MISC_FEATURE
<222>  (4)..(4)
<223>  The amino acid at this position is optionally phosphorylated

<400>  55

Lys Leu Leu Ser Pro Ser Asn Glu Lys Leu
1               5                   10

<210>  56
<211>  9
<212>  PRT
<213>  Homo sapiens

<220>
<221>  MISC_FEATURE
<222>  (8)..(8)
<223>  The amino acid at this position is optionally phosphorylated

<400>  56

Lys Leu Met Ala Pro Asp Ile Ser Leu
1               5

<210>  57
<211>  9
<212>  PRT
<213>  Homo sapiens

<220>
<221>  MISC_FEATURE
<222>  (4)..(4)
<223>  The amino acid at this position is optionally phosphorylated

<400>  57

Lys Leu Met Ser Pro Lys Ala Asp Val
1               5

<210>  58
<211>  11
<212>  PRT
<213>  Homo sapiens

<220>
<221>  MISC_FEATURE
<222>  (4)..(4)
<223>  The amino acid at this position is optionally phosphorylated

<400>  58

Lys Leu Met Ser Pro Lys Ala Asp Val Lys Leu
1               5                   10

<210>  59
<211>  9
<212>  PRT
<213>  Homo sapiens

<220>
<221>  MISC_FEATURE
<222>  (8)..(8)
<223>  The amino acid at this position is optionally phosphorylated

<400>  59

Lys Leu Gln Glu Phe Leu Gln Thr Leu
1               5

<210>  60
<211>  9
<212>  PRT
<213>  Homo sapiens

<220>
<221>  MISC_FEATURE
<222>  (4)..(4)
<223>  The amino acid at this position is optionally phosphorylated

<400>  60

Lys Gln Asp Ser Leu Val Ile Asn Leu
1               5

<210>  61
<211>  10
<212>  PRT
<213>  Homo sapiens

<220>
<221>  MISC_FEATURE
<222>  (4)..(4)
<223>  The amino acid at this position is optionally phosphorylated

<400>  61

Lys Arg Leu Ser Thr Ser Pro Val Arg Leu
1               5                   10

<210>  62
<211>  9
<212>  PRT
<213>  Homo sapiens

<220>
<221>  MISC_FEATURE
<222>  (4)..(4)
<223>  The amino acid at this position is optionally phosphorylated

<400>  62

Lys Thr Met Ser Gly Thr Phe Leu Leu
1               5

<210>  63
<211>  9
<212>  PRT
<213>  Homo sapiens

<220>
<221>  MISC_FEATURE
<222>  (6)..(6)
<223>  The amino acid at this position is optionally phosphorylated

<400>  63

Lys Thr Trp Lys Gly Ser Ile Gly Leu
1               5

<210>  64
<211>  9
<212>  PRT
<213>  Homo sapiens

<220>
<221>  MISC_FEATURE
<222>  (4)..(4)
<223>  The amino acid at this position is optionally phosphorylated

<400>  64

Lys Val Leu Ser Lys Glu Phe His Leu
1               5

<210>  65
<211>  10
<212>  PRT
<213>  Homo sapiens

<220>
<221>  MISC_FEATURE
<222>  (4)..(4)
<223>  The amino acid at this position is optionally phosphorylated

<400>  65

Lys Val Leu Ser Thr Glu Glu Met Glu Leu
1               5                   10

<210>  66
<211>  10
<212>  PRT
<213>  Homo sapiens

<220>
<221>  MISC_FEATURE
<222>  (5)..(5)
<223>  The amino acid at this position is optionally phosphorylated

<400>  66

Lys Val Leu Ser Thr Glu Glu Met Glu Leu
1               5                   10

<210>  67
<211>  10
<212>  PRT
<213>  Homo sapiens

<220>
<221>  MISC_FEATURE
<222>  (4)..(4)
<223>  The amino acid at this position is optionally phosphorylated

<400>  67

Leu Leu Ala Ser Pro Gly His Ile Ser Val
1               5                   10

<210>  68
<211>  11
<212>  PRT
<213>  Homo sapiens

<220>
<221>  MISC_FEATURE
<222>  (4)..(4)
<223>  The amino acid at this position is optionally phosphorylated

<400>  68

Leu Gln Leu Ser Pro Leu Lys Gly Leu Ser Leu
1               5                   10

<210>  69
<211>  9
<212>  PRT
<213>  Homo sapiens

<220>
<221>  MISC_FEATURE
<222>  (5)..(5)
<223>  The amino acid at this position is optionally phosphorylated

<400>  69

Leu Gln Asn Ile Thr Glu Asn Gln Leu
1               5

<210>  70
<211>  11
<212>  PRT
<213>  Homo sapiens

<220>
<221>  MISC_FEATURE
<222>  (4)..(4)
<223>  The amino acid at this position is optionally phosphorylated

<400>  70

Asn Leu Gly Ser Arg Asn His Val His Gln Leu
1               5                   10

<210>  71
<211>  12
<212>  PRT
<213>  Homo sapiens

<220>
<221>  MISC_FEATURE
<222>  (4)..(4)
<223>  The amino acid at this position is optionally phosphorylated

<400>  71

Asn Leu Leu Ser Pro Asp Gly Lys Met Ile Ser Val
1               5                   10

<210>  72
<211>  9
<212>  PRT
<213>  Homo sapiens

<220>
<221>  MISC_FEATURE
<222>  (4)..(4)
<223>  The amino acid at this position is optionally phosphorylated

<400>  72

Arg Ala Ser Ser Leu Ser Ile Thr Val
1               5

<210>  73
<211>  11
<212>  PRT
<213>  Homo sapiens

<220>
<221>  MISC_FEATURE
<222>  (4)..(4)
<223>  The amino acid at this position is optionally phosphorylated

<400>  73

Arg Glu Asp Ser Thr Pro Gly Lys Val Phe Leu
1               5                   10

<210>  74
<211>  11
<212>  PRT
<213>  Homo sapiens

<220>
<221>  MISC_FEATURE
<222>  (4)..(4)
<223>  The amino acid at this position is optionally phosphorylated

<400>  74

Arg Ile Asp Ser Lys Asp Ser Ala Ser Glu Leu
1               5                   10

<210>  75
<211>  9
<212>  PRT
<213>  Homo sapiens

<220>
<221>  MISC_FEATURE
<222>  (4)..(4)
<223>  The amino acid at this position is optionally phosphorylated

<400>  75

Arg Ile Asn Ser Phe Glu Glu His Val
1               5

<210>  76
<211>  9
<212>  PRT
<213>  Homo sapiens

<220>
<221>  MISC_FEATURE
<222>  (4)..(4)
<223>  The amino acid at this position is optionally phosphorylated

<400>  76

Arg Ile Gln Ser Lys Leu Tyr Arg Ala
1               5

<210>  77
<211>  9
<212>  PRT
<213>  Homo sapiens

<220>
<221>  MISC_FEATURE
<222>  (4)..(4)
<223>  The amino acid at this position is optionally phosphorylated

<400>  77

Arg Ile Thr Ser Leu Ile Val His Val
1               5

<210>  78
<211>  9
<212>  PRT
<213>  Homo sapiens

<220>
<221>  MISC_FEATURE
<222>  (4)..(4)
<223>  The amino acid at this position is optionally phosphorylated

<400>  78

Arg Leu Ala Ser Ala Ser Arg Ala Leu
1               5

<210>  79
<211>  10
<212>  PRT
<213>  Homo sapiens

<220>
<221>  MISC_FEATURE
<222>  (4)..(4)
<223>  The amino acid at this position is optionally phosphorylated

<400>  79

Arg Leu Ala Ser Leu Asn Ala Glu Ala Leu
1               5                   10

<210>  80
<211>  9
<212>  PRT
<213>  Homo sapiens

<220>
<221>  MISC_FEATURE
<222>  (4)..(4)
<223>  The amino acid at this position is optionally phosphorylated

<400>  80

Arg Leu Ala Ser Arg Pro Leu Leu Leu
1               5

<210>  81
<211>  9
<212>  PRT
<213>  Homo sapiens

<220>
<221>  MISC_FEATURE
<222>  (4)..(4)
<223>  The amino acid at this position is optionally phosphorylated

<400>  81

Arg Leu Asp Ser Tyr Leu Arg Ala Pro
1               5

<210>  82
<211>  7
<212>  PRT
<213>  Homo sapiens

<220>
<221>  MISC_FEATURE
<222>  (4)..(4)
<223>  The amino acid at this position is optionally phosphorylated

<400>  82

Arg Leu Asp Ser Tyr Val Arg
1               5

<210>  83
<211>  9
<212>  PRT
<213>  Homo sapiens

<220>
<221>  MISC_FEATURE
<222>  (4)..(4)
<223>  The amino acid at this position is optionally phosphorylated

<400>  83

Arg Leu Asp Ser Tyr Val Arg Ser Leu
1               5

<210>  84
<211>  9
<212>  PRT
<213>  Homo sapiens

<220>
<221>  MISC_FEATURE
<222>  (4)..(4)
<223>  The amino acid at this position is optionally phosphorylated

<400>  84

Arg Leu Asp Thr Gly Pro Gln Ser Leu
1               5

<210>  85
<211>  9
<212>  PRT
<213>  Homo sapiens

<220>
<221>  MISC_FEATURE
<222>  (4)..(4)
<223>  The amino acid at this position is optionally phosphorylated

<400>  85

Arg Leu Glu Ser Ala Asn Arg Arg Leu
1               5

<210>  86
<211>  9
<212>  PRT
<213>  Homo sapiens

<220>
<221>  MISC_FEATURE
<222>  (4)..(4)
<223>  The amino acid at this position is optionally phosphorylated

<400>  86

Arg Leu Phe Ser Lys Glu Leu Arg Cys
1               5

<210>  87
<211>  10
<212>  PRT
<213>  Homo sapiens

<220>
<221>  MISC_FEATURE
<222>  (6)..(6)
<223>  The amino acid at this position is optionally phosphorylated

<400>  87

Arg Leu Phe Ser Leu Ser Asn Pro Ser Leu
1               5                   10


<210>  88
<211>  9
<212>  PRT
<213>  Homo sapiens


<220>
<221>  MISC_FEATURE
<222>  (4)..(4)
<223>  The amino acid at this position is optionally phosphorylated


<400>  88

Arg Leu Phe Ser Gln Gly Gln Asp Val
1               5


<210>  89
<211>  10
<212>  PRT
<213>  Homo sapiens


<220>
<221>  MISC_FEATURE
<222>  (4)..(4)
<223>  The amino acid at this position is optionally phosphorylated


<400>  89

Arg Leu Gly Ser Phe His Glu Leu Leu Leu
1               5                   10


<210>  90
<211>  11
<212>  PRT
<213>  Homo sapiens


<220>
<221>  MISC_FEATURE
<222>  (4)..(4)
<223>  The amino acid at this position is optionally phosphorylated


<400>  90

Arg Leu Lys Ser Asp Glu Arg Pro Val His Ile
1               5                   10


<210>  91
<211>  10
<212>  PRT
<213>  Homo sapiens

<220>
<221>  MISC_FEATURE
<222>  (4)..(4)
<223>  The amino acid at this position is optionally phosphorylated

<400>  91

Arg Leu Leu Ser Asp Gly Gln Gln His Leu
1               5                   10

<210>  92
<211>  9
<212>  PRT
<213>  Homo sapiens

<220>
<221>  MISC_FEATURE
<222>  (4)..(4)
<223>  The amino acid at this position is optionally phosphorylated

<400>  92

Arg Leu Leu Ser Asp Leu Glu Glu Leu
1               5

<210>  93
<211>  9
<212>  PRT
<213>  Homo sapiens

<220>
<221>  MISC_FEATURE
<222>  (4)..(4)
<223>  The amino acid at this position is optionally phosphorylated

<400>  93

Arg Leu Leu Ser Asp Gln Thr Arg Leu
1               5

<210>  94
<211>  9
<212>  PRT
<213>  Homo sapiens

<220>
<221>  MISC_FEATURE
<222>  (4)..(4)
<223>  The amino acid at this position is optionally phosphorylated

<400>  94

Arg Leu Leu Ser Phe Gln Arg Tyr Leu
1               5

<210>  95
<211>  9
<212>  PRT
<213>  Homo sapiens

<220>
<221>  MISC_FEATURE
<222>  (4)..(4)
<223>  The amino acid at this position is optionally phosphorylated

<400>  95

Arg Leu Leu Ser Pro Leu Ser Ser Ala
1               5

<210>  96
<211>  11
<212>  PRT
<213>  Homo sapiens

<220>
<221>  MISC_FEATURE
<222>  (4)..(4)
<223>  The amino acid at this position is optionally phosphorylated

<400>  96

Arg Leu Leu Ser Pro Leu Ser Ser Ala Arg Leu
1               5                   10

<210>  97
<211>  9
<212>  PRT
<213>  Homo sapiens

<220>
<221>  MISC_FEATURE
<222>  (4)..(4)
<223>  The amino acid at this position is optionally phosphorylated

<400>  97

Arg Leu Leu Ser Pro Arg Pro Ser Leu
1               5

<210>  98
<211>  10
<212>  PRT
<213>  Homo sapiens

<220>
<221>  MISC_FEATURE
<222>  (4)..(4)
<223>  The amino acid at this position is optionally phosphorylated

<400>  98

Arg Leu Leu Ser Pro Arg Pro Ser Leu Leu
1               5                   10

<210>  99
<211>  10
<212>  PRT
<213>  Homo sapiens

<220>
<221>  MISC_FEATURE
<222>  (4)..(4)
<223>  The amino acid at this position is optionally phosphorylated

<400>  99

Arg Leu Leu Ser Val His Asp Phe Asp Phe
1               5                   10

<210>  100
<211>  10
<212>  PRT
<213>  Homo sapiens

<220>
<221>  MISC_FEATURE
<222>  (4)..(4)
<223>  The amino acid at this position is optionally phosphorylated

<400>  100

Arg Leu Asn Thr Ser Asp Phe Gln Lys Leu
1               5                   10

<210>  101
<211>  9
<212>  PRT
<213>  Homo sapiens

<220>
<221>  MISC_FEATURE
<222>  (8)..(8)
<223>  The amino acid at this position is optionally phosphorylated

<400>  101

Arg Leu Pro Asn Arg Ile Pro Ser Leu
1               5

<210>  102
<211>  9
<212>  PRT
<213>  Homo sapiens

<220>
<221>  MISC_FEATURE
<222>  (4)..(4)
<223>  The amino acid at this position is optionally phosphorylated

<400>  102

Arg Leu Gln Ser Leu Ile Lys Asn Ile
1               5

<210>  103
<211>  9
<212>  PRT
<213>  Homo sapiens

<220>
<221>  MISC_FEATURE
<222>  (4)..(4)
<223>  The amino acid at this position is optionally phosphorylated

<400>  103

Arg Leu Gln Ser Thr Ser Glu Arg Leu
1               5

<210>  104
<211>  9
<212>  PRT
<213>  Homo sapiens

<220>
<221>  MISC_FEATURE
<222>  (4)..(4)
<223>  The amino acid at this position is optionally phosphorylated

<400>  104

Arg Leu Arg Ser Tyr Glu Asp Met Ile
1               5

<210>  105
<211>  9
<212>  PRT
<213>  Homo sapiens

<220>
<221>  MISC_FEATURE
<222>  (4)..(4)
<223>  The amino acid at this position is optionally phosphorylated

<400>  105

Arg Leu Ser Ser Pro Leu His Phe Val
1               5

<210>  106
<211>  9
<212>  PRT
<213>  Homo sapiens

<220>
<221>  MISC_FEATURE
<222>  (5)..(5)
<223>  The amino acid at this position is optionally phosphorylated

<400>  106

Arg Met Phe Pro Thr Pro Pro Ser Leu
1               5

<210>  107
<211>  12
<212>  PRT
<213>  Homo sapiens

<220>
<221>  MISC_FEATURE
<222>  (4)..(4)
<223>  The amino acid at this position is optionally phosphorylated

<400>  107

Arg Met Phe Ser Pro Met Glu Glu Lys Glu Leu Leu
1               5                   10

<210>  108
<211>  9
<212>  PRT
<213>  Homo sapiens

<220>
<221>  MISC_FEATURE
<222>  (4)..(4)
<223>  The amino acid at this position is optionally phosphorylated

<400>  108

Arg Met Ile Ser Thr Gly Ser Glu Leu
1               5

<210>  109
<211>  10
<212>  PRT
<213>  Homo sapiens

<220>
<221>  MISC_FEATURE
<222>  (4)..(4)
<223>  The amino acid at this position is optionally phosphorylated

<400>  109

Arg Met Leu Ser Leu Arg Asp Gln Arg Leu
1               5                   10

<210>  110
<211>  9
<212>  PRT
<213>  Homo sapiens

<220>
<221>  MISC_FEATURE
<222>  (4)..(4)
<223>  The amino acid at this position is optionally phosphorylated

<400>  110

Arg Met Tyr Ser Phe Asp Asp Val Leu
1               5

<210>  111
<211>  10
<212>  PRT
<213>  Homo sapiens

<220>
<221>  MISC_FEATURE
<222>  (4)..(4)
<223>  The amino acid at this position is optionally phosphorylated

<400>  111

Arg Met Tyr Ser Pro Ile Ile Tyr Gln Ala
1               5                   10

<210>  112
<211>  11
<212>  PRT
<213>  Homo sapiens

<220>
<221>  MISC_FEATURE
<222>  (4)..(4)
<223>  The amino acid at this position is optionally phosphorylated

<400>  112

Arg Gln Asp Ser Thr Pro Gly Lys Val Phe Leu
1               5                   10

<210>  113
<211>  9
<212>  PRT
<213>  Homo sapiens

<220>
<221>  MISC_FEATURE
<222>  (4)..(4)
<223>  The amino acid at this position is optionally phosphorylated

<400>  113

Arg Gln Ile Ser Phe Lys Ala Glu Val
1               5

<210>  114
<211>  9
<212>  PRT
<213>  Homo sapiens

<220>
<221>  MISC_FEATURE
<222>  (4)..(4)
<223>  The amino acid at this position is optionally phosphorylated

<400>  114

Arg Gln Ile Ser Gln Asp Val Lys Leu
1               5

<210>  115
<211>  9
<212>  PRT
<213>  Homo sapiens

<220>
<221>  MISC_FEATURE
<222>  (4)..(4)
<223>  The amino acid at this position is optionally phosphorylated

<400>  115

Arg Gln Leu Ser Ala Leu His Arg Ala
1               5

<210>  116
<211>  12
<212>  PRT
<213>  Homo sapiens

<220>
<221>  MISC_FEATURE
<222>  (4)..(4)
<223>  The amino acid at this position is optionally phosphorylated

<400>  116

Arg Gln Leu Ser Leu Glu Gly Ser Gly Leu Gly Val
1               5                   10

<210>  117
<211>  10
<212>  PRT
<213>  Homo sapiens

<220>
<221>  MISC_FEATURE
<222>  (4)..(4)
<223>  The amino acid at this position is optionally phosphorylated

<400>  117

Arg Gln Leu Ser Ser Gly Val Ser Glu Ile
1               5                   10

<210>  118
<211>  9
<212>  PRT
<213>  Homo sapiens

<220>
<221>  MISC_FEATURE
<222>  (4)..(4)
<223>  The amino acid at this position is optionally phosphorylated

<400>  118

Arg Gln Ser Ser Ser Arg Phe Asn Leu
1               5

<210>  119
<211>  9
<212>  PRT
<213>  Homo sapiens

<220>
<221>  MISC_FEATURE
<222>  (4)..(4)
<223>  The amino acid at this position is optionally phosphorylated

<400>  119

Arg Arg Leu Ser Glu Arg Glu Thr Arg
1               5

<210>  120
<211>  13
<212>  PRT
<213>  Homo sapiens

<220>
<221>  MISC_FEATURE
<222>  (4)..(4)
<223>  The amino acid at this position is optionally phosphorylated

<400>  120

Arg Ser Ala Ser Pro Asp Asp Asp Leu Gly Ser Ser Asn
1               5                   10

<210>  121
<211>  9
<212>  PRT
<213>  Homo sapiens

<220>
<221>  MISC_FEATURE
<222>  (4)..(4)
<223>  The amino acid at this position is optionally phosphorylated

<400>  121

Arg Ser Phe Ser Pro Thr Met Lys Val
1               5

<210>  122
<211>  10
<212>  PRT
<213>  Homo sapiens

<220>
<221>  MISC_FEATURE
<222>  (4)..(4)
<223>  The amino acid at this position is optionally phosphorylated

<400>  122

Arg Ser Leu Ser Gln Glu Leu Val Gly Val
1               5                   10

<210>  123
<211>  9
<212>  PRT
<213>  Homo sapiens

<220>
<221>  MISC_FEATURE
<222>  (4)..(4)
<223>  The amino acid at this position is optionally phosphorylated

<400>  123

Arg Thr Ala Ser Leu Ile Ile Lys Val
1               5

<210>  124
<211>  9
<212>  PRT
<213>  Homo sapiens

<220>
<221>  MISC_FEATURE
<222>  (4)..(4)
<223>  The amino acid at this position is optionally phosphorylated

<400>  124

Arg Thr Phe Ser Leu Asp Thr Ile Leu
1               5

<210>  125
<211>  9
<212>  PRT
<213>  Homo sapiens

<220>
<221>  MISC_FEATURE
<222>  (4)..(4)
<223>  The amino acid at this position is optionally phosphorylated

<400>  125

Arg Thr Phe Ser Pro Thr Tyr Gly Leu
1               5

<210>  126
<211>  9
<212>  PRT
<213>  Homo sapiens

<220>
<221>  MISC_FEATURE
<222>  (4)..(4)
<223>  The amino acid at this position is optionally phosphorylated

<400>  126

Arg Thr His Ser Leu Leu Leu Leu Leu
1               5

<210>  127
<211>  9
<212>  PRT
<213>  Homo sapiens

<220>
<221>  MISC_FEATURE
<222>  (4)..(4)
<223>  The amino acid at this position is optionally phosphorylated

<400>  127

Arg Thr Leu Ser His Ile Ser Glu Ala
1               5

<210>  128
<211>  9
<212>  PRT
<213>  Homo sapiens

<220>
<221>  MISC_FEATURE
<222>  (4)..(4)
<223>  The amino acid at this position is optionally phosphorylated

<400>  128

Arg Thr Ser Ser Phe Thr Glu Gln Leu
1               5

<210>  129
<211>  9
<212>  PRT
<213>  Homo sapiens

<220>
<221>  MISC_FEATURE
<222>  (4)..(4)
<223>  The amino acid at this position is optionally phosphorylated

<400>  129

Arg Val Ala Ser Pro Thr Ser Gly Val
1               5

<210>  130
<211>  9
<212>  PRT
<213>  Homo sapiens

<220>
<221>  MISC_FEATURE
<222>  (4)..(4)
<223>  The amino acid at this position is optionally phosphorylated

<400>  130

Arg Val Asp Ser Pro Ser His Gly Leu
1               5

<210>  131
<211>  9
<212>  PRT
<213>  Homo sapiens

<220>
<221>  MISC_FEATURE
<222>  (4)..(4)
<223>  The amino acid at this position is optionally phosphorylated

<400>  131

Arg Val Gly Ser Leu Val Leu Asn Leu
1               5

<210>  132
<211>  9
<212>  PRT
<213>  Homo sapiens

<220>
<221>  MISC_FEATURE
<222>  (4)..(4)
<223>  The amino acid at this position is optionally phosphorylated

<400>  132

Arg Val Ile Ser Gly Val Leu Gln Leu
1               5

<210>  133
<211>  9
<212>  PRT
<213>  Homo sapiens

<220>
<221>  MISC_FEATURE
<222>  (5)..(5)
<223>  The amino acid at this position is optionally phosphorylated

<400>  133

Arg Val Leu His Ser Pro Pro Ala Val
1               5

<210>  134
<211>  9
<212>  PRT
<213>  Homo sapiens

<220>
<221>  MISC_FEATURE
<222>  (4)..(4)
<223>  The amino acid at this position is optionally phosphorylated

<400>  134

Arg Val Pro Ser Leu Leu Val Leu Leu
1               5

<210>  135
<211>  9
<212>  PRT
<213>  Homo sapiens

<220>
<221>  MISC_FEATURE
<222>  (4)..(4)
<223>  The amino acid at this position is optionally phosphorylated

<400>  135

Arg Val Thr Ser Ala Glu Ile Lys Leu
1               5

<210>  136
<211>  11
<212>  PRT
<213>  Homo sapiens

<220>
<221>  MISC_FEATURE
<222>  (4)..(4)
<223>  The amino acid at this position is optionally phosphorylated

<400>  136

Arg Val Trp Ser Pro Pro Arg Val His Lys Val
1               5                   10

<210>  137
<211>  14
<212>  PRT
<213>  Homo sapiens

<220>
<221>  MISC_FEATURE
<222>  (5)..(5)
<223>  The amino acid at this position is optionally phosphorylated

<400>  137

Ser Ala Arg Gly Ser Pro Thr Arg Pro Asn Pro Pro Val Arg
1               5                   10

<210>  138
<211>  9
<212>  PRT
<213>  Homo sapiens

<220>
<221>  MISC_FEATURE
<222>  (4)..(4)
<223>  The amino acid at this position is optionally phosphorylated

<400>  138

Ser Ile Leu Ser Phe Val Ser Gly Leu
1               5

<210>  139
<211>  9
<212>  PRT
<213>  Homo sapiens

<220>
<221>  MISC_FEATURE
<222>  (4)..(4)
<223>  The amino acid at this position is optionally phosphorylated

<400>  139

Ser Ile Met Ser Phe His Ile Asp Leu
1               5

<210>  140
<211>  9
<212>  PRT
<213>  Homo sapiens

<220>
<221>  MISC_FEATURE
<222>  (4)..(4)
<223>  The amino acid at this position is optionally phosphorylated

<400>  140

Ser Ile Met Ser Pro Glu Ile Gln Leu
1               5

<210>  141
<211>  9
<212>  PRT
<213>  Homo sapiens

<220>
<221>  MISC_FEATURE
<222>  (5)..(5)
<223>  The amino acid at this position is optionally phosphorylated

<400>  141

Ser Ile Ser Ser Thr Pro Pro Ala Val
1               5

<210>  142
<211>  9
<212>  PRT
<213>  Homo sapiens

<220>
<221>  MISC_FEATURE
<222>  (3)..(3)
<223>  The amino acid at this position is optionally phosphorylated

<400>  142

Ser Lys Thr Val Ala Thr Phe Ile Leu
1               5

<210>  143
<211>  9
<212>  PRT
<213>  Homo sapiens

<220>
<221>  MISC_FEATURE
<222>  (4)..(4)
<223>  The amino acid at this position is optionally phosphorylated

<400>  143

Ser Leu Ala Ser Leu Thr Glu Lys Ile
1               5

<210>  144
<211>  9
<212>  PRT
<213>  Homo sapiens

<220>
<221>  MISC_FEATURE
<222>  (8)..(8)
<223>  The amino acid at this position is optionally phosphorylated

<400>  144

Ser Leu Asp Ser Glu Asp Tyr Ser Leu
1               5

<210>  145
<211>  10
<212>  PRT
<213>  Homo sapiens

<220>
<221>  MISC_FEATURE
<222>  (4)..(4)
<223>  The amino acid at this position is optionally phosphorylated

<400>  145

Ser Leu Asp Ser Leu Gly Asp Val Phe Leu
1               5                   10

<210>  146
<211>  9
<212>  PRT
<213>  Homo sapiens

<220>
<221>  MISC_FEATURE
<222>  (6)..(6)
<223>  The amino acid at this position is optionally phosphorylated

<400>  146

Ser Leu Phe Gly Gly Ser Val Lys Leu
1               5

<210>  147
<211>  9
<212>  PRT
<213>  Homo sapiens

<220>
<221>  MISC_FEATURE
<222>  (8)..(8)
<223>  The amino acid at this position is optionally phosphorylated

<400>  147

Ser Leu Phe Lys Arg Leu Tyr Ser Leu
1               5

<210>  148
<211>  12
<212>  PRT
<213>  Homo sapiens

<220>
<221>  MISC_FEATURE
<222>  (4)..(4)
<223>  The amino acid at this position is optionally phosphorylated

<400>  148

Ser Leu Phe Ser Ser Glu Glu Ser Asn Leu Gly Ala
1               5                   10

<210>  149
<211>  10
<212>  PRT
<213>  Homo sapiens

<220>
<221>  MISC_FEATURE
<222>  (4)..(4)
<223>  The amino acid at this position is optionally phosphorylated

<400>  149

Ser Leu Phe Ser Gly Asp Glu Glu Asn Ala
1               5                   10

<210>  150
<211>  10
<212>  PRT
<213>  Homo sapiens

<220>
<221>  MISC_FEATURE
<222>  (4)..(4)
<223>  The amino acid at this position is optionally phosphorylated

<400>  150

Ser Leu Phe Ser Gly Ser Tyr Ser Ser Leu
1               5                   10

<210>  151
<211>  11
<212>  PRT
<213>  Homo sapiens

<220>
<221>  MISC_FEATURE
<222>  (5)..(5)
<223>  The amino acid at this position is optionally phosphorylated

<400>  151

Ser Leu Leu Ala Ser Pro Gly His Ile Ser Val
1               5                   10

<210>  152
<211>  9
<212>  PRT
<213>  Homo sapiens

<220>
<221>  MISC_FEATURE
<222>  (8)..(8)
<223>  The amino acid at this position is optionally phosphorylated

<400>  152

Ser Leu Leu His Thr Ser Arg Ser Leu
1               5

<210>  153
<211>  9
<212>  PRT
<213>  Homo sapiens

<220>
<221>  MISC_FEATURE
<222>  (4)..(4)
<223>  The amino acid at this position is optionally phosphorylated

<400>  153

Ser Leu Leu Ser Leu His Val Asp Leu
1               5

<210>  154
<211>  10
<212>  PRT
<213>  Homo sapiens

<220>
<221>  MISC_FEATURE
<222>  (4)..(4)
<223>  The amino acid at this position is optionally phosphorylated

<400>  154

Ser Leu Met Ser Gly Thr Leu Glu Ser Leu
1               5                   10

<210>  155
<211>  9
<212>  PRT
<213>  Homo sapiens

<220>
<221>  MISC_FEATURE
<222>  (8)..(8)
<223>  The amino acid at this position is optionally phosphorylated

<400>  155

Ser Leu Gln Pro Arg Ser His Ser Val
1               5

<210>  156
<211>  9
<212>  PRT
<213>  Homo sapiens

<220>
<221>  MISC_FEATURE
<222>  (4)..(4)
<223>  The amino acid at this position is optionally phosphorylated

<400>  156

Ser Leu Gln Ser Leu Glu Thr Ser Val
1               5

<210>  157
<211>  9
<212>  PRT
<213>  Homo sapiens

<220>
<221>  MISC_FEATURE
<222>  (4)..(4)
<223>  The amino acid at this position is optionally phosphorylated

<400>  157

Ser Leu Ser Ser Leu Leu Val Lys Leu
1               5

<210>  158
<211>  9
<212>  PRT
<213>  Homo sapiens

<220>
<221>  MISC_FEATURE
<222>  (6)..(6)
<223>  The amino acid at this position is optionally phosphorylated

<400>  158

Ser Leu Val Asp Gly Tyr Phe Arg Leu
1               5

<210>  159
<211>  10
<212>  PRT
<213>  Homo sapiens

<220>
<221>  MISC_FEATURE
<222>  (4)..(4)
<223>  The amino acid at this position is optionally phosphorylated

<400>  159

Ser Met Leu Ser Gln Glu Ile Gln Thr Leu
1               5                   10

<210>  160
<211>  9
<212>  PRT
<213>  Homo sapiens

<220>
<221>  MISC_FEATURE
<222>  (4)..(4)
<223>  The amino acid at this position is optionally phosphorylated

<400>  160

Ser Met Ser Ser Leu Ser Arg Glu Val
1               5

<210>  161
<211>  9
<212>  PRT
<213>  Homo sapiens

<220>
<221>  MISC_FEATURE
<222>  (5)..(5)
<223>  The amino acid at this position is optionally phosphorylated

<400>  161

Ser Met Thr Arg Ser Pro Pro Arg Val
1               5

<210>  162
<211>  8
<212>  PRT
<213>  Homo sapiens

<220>
<221>  MISC_FEATURE
<222>  (4)..(5)
<223>  The amino acids at these position are optionally phosphorylated

<400>  162

Ser Pro Arg Ser Ser Gln Leu Val
1               5

<210>  163
<211>  13
<212>  PRT
<213>  Homo sapiens

<220>
<221>  MISC_FEATURE
<222>  (1)..(1)
<223>  The amino acid at this position is optionally phosphorylated

<400>  163

Ser Pro Thr Arg Pro Asn Pro Pro Val Arg Asn Leu His
1               5                   10

<210>  164
<211>  10
<212>  PRT
<213>  Homo sapiens

<220>
<221>  MISC_FEATURE
<222>  (4)..(4)
<223>  The amino acid at this position is optionally phosphorylated

<400>  164

Ser Gln Ile Ser Pro Lys Ser Trp Gly Val
1               5                   10

<210>  165
<211>  10
<212>  PRT
<213>  Homo sapiens

<220>
<221>  MISC_FEATURE
<222>  (4)..(4)
<223>  The amino acid at this position is optionally phosphorylated

<400>  165

Ser Thr Met Ser Leu Asn Ile Ile Thr Val
1               5                   10

<210>  166
<211>  10
<212>  PRT
<213>  Homo sapiens

<220>
<221>  MISC_FEATURE
<222>  (1)..(1)
<223>  The amino acid at this position is optionally phosphorylated

<400>  166

Ser Thr Met Ser Leu Asn Ile Ile Thr Val
1               5                   10

<210>  167
<211>  9
<212>  PRT
<213>  Homo sapiens

<220>
<221>  MISC_FEATURE
<222>  (4)..(4)
<223>  The amino acid at this position is optionally phosphorylated

<400>  167

Ser Val Phe Ser Pro Ser Phe Gly Leu
1               5

<210>  168
<211>  10
<212>  PRT
<213>  Homo sapiens

<220>
<221>  MISC_FEATURE
<222>  (4)..(4)
<223>  The amino acid at this position is optionally phosphorylated

<400>  168

Ser Val Gly Ser Asp Tyr Tyr Ile Gln Leu
1               5                   10

<210>  169
<211>  9
<212>  PRT
<213>  Homo sapiens

<220>
<221>  MISC_FEATURE
<222>  (4)..(4)
<223>  The amino acid at this position is optionally phosphorylated

<400>  169

Ser Val Leu Ser Pro Ser Phe Gln Leu
1               5

<210>  170
<211>  9
<212>  PRT
<213>  Homo sapiens

<220>
<221>  MISC_FEATURE
<222>  (5)..(5)
<223>  The amino acid at this position is optionally phosphorylated

<400>  170

Ser Val Met Asp Ser Pro Lys Lys Leu
1               5

<210>  171
<211>  12
<212>  PRT
<213>  Homo sapiens

<220>
<221>  MISC_FEATURE
<222>  (4)..(4)
<223>  The amino acid at this position is optionally phosphorylated

<400>  171

Ser Val Tyr Ser Gly Asp Phe Gly Asn Leu Glu Val
1               5                   10


<210>  172
<211>  9
<212>  PRT
<213>  Homo sapiens


<220>
<221>  MISC_FEATURE
<222>  (4)..(4)
<223>  The amino acid at this position is optionally phosphorylated


<400>  172

Thr Leu Ser Ser Pro Pro Pro Gly Leu
1               5


<210>  173
<211>  9
<212>  PRT
<213>  Homo sapiens


<220>
<221>  MISC_FEATURE
<222>  (4)..(4)
<223>  The amino acid at this position is optionally phosphorylated


<400>  173

Thr Met Met Ser Pro Ser Gln Phe Leu
1               5


<210>  174
<211>  11
<212>  PRT
<213>  Homo sapiens


<220>
<221>  MISC_FEATURE
<222>  (4)..(4)
<223>  The amino acid at this position is optionally phosphorylated


<400>  174

Thr Val Met Ser Asn Ser Ser Val Ile His Leu
1               5                   10


<210>  175
<211>  10
<212>  PRT
<213>  Homo sapiens

<220>
<221>  MISC_FEATURE
<222>  (4)..(4)
<223>  The amino acid at this position is optionally phosphorylated

<400>  175

Val Ile Asp Ser Gln Glu Leu Ser Lys Val
1               5                   10

<210>  176
<211>  9
<212>  PRT
<213>  Homo sapiens

<220>
<221>  MISC_FEATURE
<222>  (4)..(4)
<223>  The amino acid at this position is optionally phosphorylated

<400>  176

Val Leu Phe Ser Ser Pro Pro Gln Met
1               5

<210>  177
<211>  9
<212>  PRT
<213>  Homo sapiens

<220>
<221>  MISC_FEATURE
<222>  (5)..(5)
<223>  The amino acid at this position is optionally phosphorylated

<400>  177

Val Leu Phe Ser Ser Pro Pro Gln Met
1               5

<210>  178
<211>  10
<212>  PRT
<213>  Homo sapiens

<220>
<221>  MISC_FEATURE
<222>  (6)..(6)
<223>  The amino acid at this position is optionally phosphorylated

<400>  178

Val Leu Ser Ser Leu Thr Pro Ala Lys Val
1               5                   10


<210>  179
<211>  10
<212>  PRT
<213>  Homo sapiens


<220>
<221>  MISC_FEATURE
<222>  (5)..(5)
<223>  The amino acid at this position is optionally phosphorylated


<400>  179

Val Met Phe Arg Thr Pro Leu Ala Ser Val
1               5                   10


<210>  180
<211>  9
<212>  PRT
<213>  Homo sapiens


<220>
<221>  MISC_FEATURE
<222>  (5)..(5)
<223>  The amino acid at this position is optionally phosphorylated


<400>  180

Val Met Ile Gly Ser Pro Lys Lys Val
1               5


<210>  181
<211>  9
<212>  PRT
<213>  Homo sapiens


<220>
<221>  MISC_FEATURE
<222>  (8)..(8)
<223>  The amino acid at this position is optionally phosphorylated


<400>  181

Tyr Ala Tyr Asp Gly Lys Asp Tyr Ile
1               5


<210>  182
<211>  9
<212>  PRT
<213>  Homo sapiens

<220>
<221>  MISC_FEATURE
<222>  (4)..(4)
<223>  The amino acid at this position is optionally phosphorylated

<400>  182

Tyr Leu Ala Ser Leu Glu Lys Lys Leu
1               5

<210>  183
<211>  9
<212>  PRT
<213>  Homo sapiens

<220>
<221>  MISC_FEATURE
<222>  (4)..(4)
<223>  The amino acid at this position is optionally phosphorylated

<400>  183

Tyr Leu Asp Ser Gly Ile His Ser Gly
1               5

<210>  184
<211>  10
<212>  PRT
<213>  Homo sapiens

<220>
<221>  MISC_FEATURE
<222>  (4)..(4)
<223>  The amino acid at this position is optionally phosphorylated

<400>  184

Tyr Leu Asp Ser Gly Ile His Ser Gly Ala
1               5                   10

<210>  185
<211>  8
<212>  PRT
<213>  Homo sapiens

<220>
<221>  MISC_FEATURE
<222>  (1)..(1)
<223>  The amino acid at this position is optionally phosphorylated

<400>  185

Tyr Leu Gly Leu Asp Val Pro Val
1               5


<210>  186
<211>  11
<212>  PRT
<213>  Homo sapiens


<220>
<221>  MISC_FEATURE
<222>  (4)..(4)
<223>  The amino acid at this position is optionally phosphorylated


<400>  186

Tyr Leu Gly Ser Ile Ser Thr Leu Val Thr Leu
1               5                   10


<210>  187
<211>  9
<212>  PRT
<213>  Homo sapiens


<220>
<221>  MISC_FEATURE
<222>  (5)..(5)
<223>  The amino acid at this position is optionally phosphorylated


<400>  187

Tyr Leu Ile His Ser Pro Met Ser Leu
1               5


<210>  188
<211>  9
<212>  PRT
<213>  Homo sapiens


<220>
<221>  MISC_FEATURE
<222>  (4)..(4)
<223>  The amino acid at this position is optionally phosphorylated


<400>  188

Tyr Leu Leu Ser Pro Leu Asn Thr Leu
1               5


<210>  189
<211>  9
<212>  PRT
<213>  Homo sapiens

<220>
<221>  MISC_FEATURE
<222>  (1)..(1)
<223>  The amino acid at this position is optionally phosphorylated

<400>  189

Tyr Leu Gln Ser Arg Tyr Tyr Arg Ala
1               5

<210>  190
<211>  9
<212>  PRT
<213>  Homo sapiens

<220>
<221>  MISC_FEATURE
<222>  (4)..(4)
<223>  The amino acid at this position is optionally phosphorylated

<400>  190

Tyr Leu Gln Ser Arg Tyr Tyr Arg Ala
1               5

<210>  191
<211>  11
<212>  PRT
<213>  Homo sapiens

<220>
<221>  MISC_FEATURE
<222>  (5)..(5)
<223>  The amino acid at this position is optionally phosphorylated

<400>  191

Tyr Leu Ser Asp Ser Asp Thr Glu Ala Lys Leu
1               5                   10

<210>  192
<211>  11
<212>  PRT
<213>  Homo sapiens

<220>
<221>  MISC_FEATURE
<222>  (4)..(4)
<223>  The amino acid at this position is optionally phosphorylated

<400>  192

Tyr Gln Leu Ser Pro Thr Lys Leu Pro Ser Ile
1               5                   10

<210>  193
<211>  9
<212>  PRT
<213>  Homo sapiens

<220>
<221>  MISC_FEATURE
<222>  (5)..(5)
<223>  The amino acid at this position is optionally phosphorylated

<400>  193

Tyr Thr Ala Gly Thr Pro Tyr Lys Val
1               5

<210>  194
<211>  9
<212>  PRT
<213>  Homo sapiens

<400>  194

His Leu Phe Gly Tyr Ser Trp Tyr Lys
1               5

<210>  195
<211>  9
<212>  PRT
<213>  Homo sapiens

<400>  195

Tyr Leu Ser Gly Ala Asp Leu Asn Leu
1               5

<210>  196
<211>  9
<212>  PRT
<213>  Homo sapiens

<400>  196

Glu Ile Trp Thr His Ser Tyr Lys Val
1               5

<210>  197
<211>  9
<212>  PRT
<213>  Homo sapiens

<400>  197

Ala Leu Leu Ala Val Gly Ala Thr Lys
1               5


<210>  198
<211>  16
<212>  PRT
<213>  Homo sapiens


<400>  198

Trp Asn Arg Gln Leu Tyr Pro Glu Trp Thr Glu Ala Gln Arg Leu Asp
1               5                   10                  15


<210>  199
<211>  9
<212>  PRT
<213>  Homo sapiens


<400>  199

Ala Leu Asn Phe Pro Gly Ser Gln Lys
1               5


<210>  200
<211>  9
<212>  PRT
<213>  Homo sapiens


<400>  200

Ser Gln Asn Phe Pro Gly Ser Gln Lys
1               5


<210>  201
<211>  9
<212>  PRT
<213>  Homo sapiens


<400>  201

Lys Thr Trp Gly Gln Tyr Trp Gln Val
1               5


<210>  202
<211>  9
<212>  PRT
<213>  Homo sapiens


<400>  202

Ile Thr Asp Gln Val Pro Phe Ser Val

1                   5


<210>  203
<211>  9
<212>  PRT
<213>  Homo sapiens


<400>  203

Ile Met Asp Gln Val Pro Phe Ser Val
1                   5


<210>  204
<211>  9
<212>  PRT
<213>  Homo sapiens


<400>  204

Tyr Leu Glu Pro Gly Pro Val Thr Ala
1                   5


<210>  205
<211>  10
<212>  PRT
<213>  Homo sapiens


<400>  205

Val Leu Tyr Arg Tyr Gly Ser Phe Ser Val
1                   5                   10


<210>  206
<211>  9
<212>  PRT
<213>  Homo sapiens


<400>  206

Leu Ile Tyr Arg Arg Arg Leu Met Lys
1                   5


<210>  207
<211>  9
<212>  PRT
<213>  Homo sapiens


<400>  207

Lys Ile Phe Gly Ser Leu Ala Phe Leu
1                   5

<210>  208
<211>  9
<212>  PRT
<213>  Homo sapiens

<400>  208

Val Leu Arg Glu Asn Thr Ser Pro Lys
1               5

<210>  209
<211>  14
<212>  PRT
<213>  Homo sapiens

<400>  209

Leu Leu Lys Tyr Arg Ala Arg Glu Pro Val Thr Lys Ala Glu
1               5                   10

<210>  210
<211>  9
<212>  PRT
<213>  Homo sapiens

<400>  210

Ser Leu Phe Arg Ala Val Ile Thr Lys
1               5

<210>  211
<211>  9
<212>  PRT
<213>  Homo sapiens

<400>  211

Glu Ala Asp Pro Thr Gly His Ser Tyr
1               5

<210>  212
<211>  9
<212>  PRT
<213>  Homo sapiens

<400>  212

Glu Val Asp Pro Ile Gly His Leu Tyr
1               5

<210>  213
<211>  15
<212>  PRT

<213>  Homo sapiens

<400>  213

Thr Ser Tyr Val Lys Val Leu His His Met Val Lys Ile Ser Gly
1               5                   10                  15

<210>  214
<211>  9
<212>  PRT
<213>  Homo sapiens

<400>  214

Gly Leu Tyr Asp Gly Met Glu His Leu
1               5

<210>  215
<211>  9
<212>  PRT
<213>  Homo sapiens

<400>  215

Ala Ala Gly Ile Gly Ile Leu Thr Val
1               5

<210>  216
<211>  23
<212>  PRT
<213>  Homo sapiens

<400>  216

Arg Asn Gly Tyr Arg Ala Leu Met Asp Lys Ser Leu His Val Gly Thr
1               5                   10                  15

Gln Cys Ala Leu Thr Arg Arg
            20

<210>  217
<211>  20
<212>  PRT
<213>  Homo sapiens

<220>
<221>  MISC_FEATURE
<222>  (12)..(12)
<223>  The amino acid at this position is optionally phosphorylated

<400>  217

Val Pro Asn Ala Pro Pro Ala Tyr Glu Lys Leu Ser Ala Glu Gln Ser
1               5                   10                  15

Pro Pro Pro Tyr
            20

<210>  218
<211>  12
<212>  PRT
<213>  Homo sapiens

<220>
<221>  MISC_FEATURE
<222>  (11)..(11)
<223>  The amino acid at this position is optionally phosphorylated

<400>  218

Pro Asn Ala Pro Pro Ala Tyr Glu Lys Leu Ser Ala
1               5                   10

<210>  219
<211>  12
<212>  PRT
<213>  Homo sapiens

<220>
<221>  MISC_FEATURE
<222>  (11)..(11)
<223>  The amino acid at this position is optionally phosphorylated

<400>  219

Pro Asn Ala Pro Pro Ala Tyr Glu Lys Leu Ser Ala
1               5                   10

<210>  220
<211>  9
<212>  PRT
<213>  Homo sapiens

<220>
<221>  MISC_FEATURE
<222>  (9)..(9)
<223>  The amino acid at this position is optionally phosphorylated

<400>  220

Ala Pro Pro Ala Tyr Glu Lys Leu Ser
1               5

<210>  221

<211>  12
<212>  PRT
<213>  Homo sapiens

<220>
<221>  MISC_FEATURE
<222>  (9)..(9)
<223>  The amino acid at this position is optionally phosphorylated

<400>  221

Ala Pro Pro Ala Tyr Glu Lys Leu Ser Ala Glu Gln
1               5                   10

<210>  222
<211>  15
<212>  PRT
<213>  Homo sapiens

<220>
<221>  MISC_FEATURE
<222>  (9)..(9)
<223>  The amino acid at this position is optionally phosphorylated

<400>  222

Ala Pro Pro Ala Tyr Glu Lys Leu Ser Ala Glu Gln Ser Pro Pro
1               5                   10                  15

<210>  223
<211>  16
<212>  PRT
<213>  Homo sapiens

<220>
<221>  MISC_FEATURE
<222>  (9)..(9)
<223>  The amino acid at this position is optionally phosphorylated

<400>  223

Ala Pro Pro Ala Tyr Glu Lys Leu Ser Ala Glu Gln Ser Pro Pro Pro
1               5                   10                  15

<210>  224
<211>  17
<212>  PRT
<213>  Homo sapiens

<220>
<221>  MISC_FEATURE
<222>  (9)..(9)
<223>  The amino acid at this position is optionally phosphorylated

<400>  224

Ala Pro Pro Ala Tyr Glu Lys Leu Ser Ala Glu Gln Ser Pro Pro Pro
1               5                   10                  15

Tyr

<210>  225
<211>  9
<212>  PRT
<213>  Homo sapiens

<220>
<221>  MISC_FEATURE
<222>  (8)..(8)
<223>  The amino acid at this position is optionally phosphorylated

<400>  225

Pro Pro Ala Tyr Glu Lys Leu Ser Ala
1               5

<210>  226
<211>  12
<212>  PRT
<213>  Homo sapiens

<220>
<221>  MISC_FEATURE
<222>  (8)..(8)
<223>  The amino acid at this position is optionally phosphorylated

<400>  226

Pro Pro Ala Tyr Glu Lys Leu Ser Ala Glu Gln Ser
1               5                   10

<210>  227
<211>  9
<212>  PRT
<213>  Homo sapiens

<220>
<221>  MISC_FEATURE
<222>  (7)..(7)
<223>  The amino acid at this position is optionally phosphorylated

<400>  227

Pro Ala Tyr Glu Lys Leu Ser Ala Glu

1               5


<210>  228
<211>  12
<212>  PRT
<213>  Homo sapiens


<220>
<221>  MISC_FEATURE
<222>  (7)..(7)
<223>  The amino acid at this position is optionally phosphorylated


<400>  228

Pro Ala Tyr Glu Lys Leu Ser Ala Glu Gln Ser Pro
1               5                   10


<210>  229
<211>  12
<212>  PRT
<213>  Homo sapiens


<220>
<221>  MISC_FEATURE
<222>  (6)..(6)
<223>  The amino acid at this position is optionally phosphorylated


<400>  229

Ala Tyr Glu Lys Leu Ser Ala Glu Gln Ser Pro Pro
1               5                   10


<210>  230
<211>  12
<212>  PRT
<213>  Homo sapiens


<220>
<221>  MISC_FEATURE
<222>  (5)..(5)
<223>  The amino acid at this position is optionally phosphorylated


<400>  230

Tyr Glu Lys Leu Ser Ala Glu Gln Ser Pro Pro Pro
1               5                   10


<210>  231
<211>  9
<212>  PRT
<213>  Homo sapiens

<220>
<221>  MISC_FEATURE
<222>  (4)..(4)
<223>  The amino acid at this position is optionally phosphorylated


<400>  231

Ala Ala Gln Glu Arg Arg Val Pro Arg
1               5


<210>  232
<211>  9
<212>  PRT
<213>  Homo sapiens


<220>
<221>  MISC_FEATURE
<222>  (4)..(4)
<223>  The amino acid at this position is optionally phosphorylated


<400>  232

Leu Leu Gly Pro Gly Arg Pro Tyr Arg
1               5


<210>  233
<211>  10
<212>  PRT
<213>  Homo sapiens


<220>
<221>  MISC_FEATURE
<222>  (4)..(4)
<223>  The amino acid at this position is optionally phosphorylated


<400>  233

Ala Ser Gly Pro Gly Gly Gly Ala Pro Arg
1               5                   10


<210>  234
<211>  16
<212>  PRT
<213>  Colstridium tetani

<400>  234

Ala Gln Tyr Ile Lys Ala Asn Ser Lys Phe Ile Gly Ile Thr Glu Leu
1               5                   10                  15


<210>  235
<211>  9
<212>  PRT
<213>  Homo sapiens

<220>
<221>  MISC_FEATURE
<222>  (4)..(4)
<223>  The amino acid at this position is optionally phosphorylated

<400>  235

Arg Leu Ser Asn Arg Leu Leu Leu Arg
1               5

<210>  236
<211>  16
<212>  PRT
<213>  Homo sapiens

<220>
<221>  MISC_FEATURE
<222>  (4)..(4)
<223>  The amino acid at this position is optionally phosphorylated

<400>  236

Ala Gln Asn Ile Leu Leu Ser Asn Ala Pro Leu Gly Pro Gln Phe Pro
1               5                   10                  15

<210>  237
<211>  11
<212>  PRT
<213>  Homo sapiens

<220>
<221>  MISC_FEATURE
<222>  (4)..(4)
<223>  The amino acid at this position is optionally phosphorylated

<400>  237

Ser Ser Asp Tyr Val Ile Pro Ile Gly Thr Tyr
1               5                   10

<210>  238
<211>  13
<212>  PRT
<213>  Homo sapiens

<220>
<221>  MISC_FEATURE
<222>  (4)..(4)
<223>  The amino acid at this position is optionally phosphorylated

<400>  238

Ser Asp Ala Glu Lys Ser Asp Ile Cys Thr Asp Glu Tyr
1               5                   10

<210>  239
<211>  9
<212>  PRT
<213>  Homo sapiens

<220>
<221>  MISC_FEATURE
<222>  (4)..(4)
<223>  The amino acid at this position is optionally phosphorylated

<400>  239

Lys Cys Asp Ile Cys Thr Asp Glu Tyr
1               5

<210>  240
<211>  9
<212>  PRT
<213>  Homo sapiens

<220>
<221>  MISC_FEATURE
<222>  (4)..(4)
<223>  The amino acid at this position is optionally phosphorylated

<400>  240

Tyr Met Asp Gly Thr Met Ser Gln Val
1               5

<210>  241
<211>  21
<212>  PRT
<213>  Homo sapiens

<220>
<221>  MISC_FEATURE
<222>  (4)..(4)
<223>  The amino acid at this position is optionally phosphorylated

<400>  241

<210>  242
<211>  15
<212>  PRT
<213>  Colstridium tetani

<400>  242

Gln Tyr Ile Lys Ala Asn Ser Lys Phe Ile Gly Ile Thr Glu Leu
1               5                   10                  15


<210>  243
<211>  12
<212>  PRT
<213>  Artificial Sequence

<220>
<223>  Artificially synthesized PADRE peptide


<220>
<221>  misc_feature
<222>  (3)..(3)
<223>  Xaa can be any naturally occurring amino acid

<400>  243

Ala Lys Xaa Val Ala Ala Trp Thr Leu Lys Ala Ala
1               5                   10

## Description

### CROSS-REFERENCE TO RELATED APPLICATION

This application embodiments the benefit of U.S. Provisional Patent Application Serial No. 61/736,815, filed December 13, 2012, the disclosure of which is incorporated herein by reference in its entirety.

### REFERENCE TO SEQUENCE LISTING

The Sequence Listing associated with the instant disclosure has been electronically submitted to the United States Patent and Trademark Office as International Receiving Office as a 64 kilobyte ASCII text file created on December 13, 2013 and entitled "3062_11_PCT_ST25.txt". The Sequence Listing submitted via EFS-Web is hereby incorporated by reference in its entirety.

### GRANT STATEMENT

This invention was made with government support under Grant No. AI 033993 awarded by National Institutes of Health. The Government has certain rights in the invention.

### TECHNICAL FIELD

The presently disclosed subject matter relates to diagnostics and therapeutics. In particular, it relates to immunotherapies and diagnostics in the context of proliferative diseases such as cancer.

### BACKGROUND

The mammalian immune system has evolved a variety of mechanisms to protect the host from cancerous cells. An important component of this response is mediated by cells referred to as T cells. Cytotoxic T lymphocytes (CTL) are specialized T cells that primarily function by recognizing and killing cancerous cells or infected cells, but they can also function by secreting soluble molecules referred to as cytokines that can mediate a variety of effects on the immune system. T helper cells primarily function by recognizing antigen on specialized antigen presenting cells, and in turn secreting cytokines that activate B cells, T cells, and macrophages. A variety of evidence suggests that immunotherapy designed to stimulate a tumor-specific CTL response would be effective in controlling cancer. For example, it has been shown that human CTL recognize sarcomas (Slovin et al. (1986) J Immunol 137:3042-3048), renal cell carcinomas (Schendel et al. (1993) J Immunol 151:4209-4220), colorectal carcinomas (Jacob et al. (1997) Int J Cancer 71:325-332), ovarian carcinomas (Peoples et al. (1993) Surgery 114:227-234), pancreatic carcinomas (Peiper et al. (1997) Eur J Immunol 27:1115-1123), squamous tumors of the head and neck (Yasumura et al. (1993) Cancer Res 53:1461-1468), and squamous carcinomas of the lung (Slingluff et al. (1994) Cancer Res 54:2731-2737; Yoshino et al. (1994) Cancer Res 54:3387-3390). The largest number of reports of human tumor-reactive CTLs, however, has concerned melanomas (Boon et al. (1994) Annu Rev Immunol 12:337-365). The ability of tumor-specific CTL to mediate tumor regression, in both human (Parmiani et al. (2002) J Natl Cancer Inst 94:805-818; Weber (2002) Cancer Invest 20:208-221) and animal models, suggests that methods directed at increasing CTL activity would likely have a beneficial effect with respect to tumor treatment.

Ovarian cancer is a cancer that starts in the ovaries, the female reproductive organ that produces eggs. It is the ninth most common cancer among women and causes more deaths than any other type of female reproductive cancer. Ovarian cancer accounts for 3% of all cancers in women. While the cause of ovarian cancer is unknown, several factors appear to affect a woman's risk for developing ovarian cancer. Age, obesity, estrogen therapy, family histories of ovarian, breast or colorectal cancer, among other factors have been found to increase a woman's chance for ovarian cancer. Also, some gene defects, such as BRCA1 and BRCA2, appear to be responsible for a small number of ovarian cancer cases. On the other hand, some factors appear to decrease the risk including, taking birth control pills and having children. Symptoms of ovarian cancer are usually vague, but can include tiredness, back pain, upset stomach, menstrual changes, pelvic discomfort or pain, and constipation. Screening can include pelvic examinations, imaging including CT scans, MRI, or ultrasound of the pelvis, blood tests including CA125 blood test, and pelvic laparoscopy or exploratory laparotomy. Surgery is used to treat all stages of ovarian cancer. Additionally, chemotherapy has also been used to treat any remaining disease after surgery or if the cancer comes back.

According to the American Cancer Society, only about 20% of ovarian cancers are found at an early stage. Among those women, about 9 out of 10 women treated for early ovarian cancer will longer than 5 years after the cancer is found. The survival rates differ among different types of ovarian cancer. For example, for invasive epithelial ovarian cancer, the American Cancer Society reports the following 5 year survival rates: Stage I: 89%; IA, 94%; Stage IB: 91%; IC: 80%; Stage II: 66%; IIB: 67%; IIC: 57%; III: 34%; IIIA: 45%; IIIB: 39%; IIIC: 35%; IV: 18%. For ovarian tumors of low malignant potential, the 5 year survival rates are reported to be as follows: Stage I: 99%; II: 98%; III: 96%; and IV: 77%. Nevertheless, additional therapeutics which are safer and more effective than current therapies are in high demand.

In order for CTL to kill or secrete cytokines in response to a cancer cell, the CTL must first recognize the cancer cell (Townsend & Bodmer (1989) Ann Rev Immunol 7:601-624). This process involves the interaction of the T cell receptor, located on the surface of the CTL, with what is generically referred to as an MHC-peptide complex which is located on the surface of the cancerous cell. MHC (major histocompatibility-complex)-encoded molecules have been subdivided into two types, and are referred to as class I and class II MHC-encoded molecules. In the human immune system, MHC molecules are referred to as human leukocyte antigens (HLA). Within the MHC complex, located on chromosome six, are three different loci that encode for class I MHC molecules. MHC molecules encoded at these loci are referred to as HLA-A, HLA-B, and HLA-C. The genes that can be encoded at each of these loci are extremely polymorphic, and thus, different individuals within the population express different class I MHC molecules on the surface of their cells. HLA-A1, HLA-A2, HLA-A3, HLA-B7, HLA-B 14, HLA-B27, and HLA-B44 are examples of different class I MHC molecules that can be expressed from these loci.

The peptides which associate with the MHC molecules can either be derived from proteins made within the cell, in which case they typically associate with class I MHC molecules (Rock & Goldberg (1999) Annu Rev Immunol 17:739-779); or they can be derived from proteins which are acquired from outside of the cell, in which case they typically associate with class II MHC molecules (Watts (1997) Annu Rev Immunol 15:821-850). The peptides that evoke a cancer-specific CTL response most typically associate with class I MHC molecules. The peptides themselves are typically nine amino acids in length, but can vary from a minimum length of eight amino acids to a maximum of fourteen amino acids in length. Tumor antigens can also bind to class II MHC molecules on antigen presenting cells and provoke a T helper cell response. The peptides that bind to class II MHC molecules are generally twelve to nineteen amino acids in length, but can be as short as ten amino acids and as long as thirty amino acids.

The process by which intact proteins are degraded into peptides is referred to as antigen processing. Two major pathways of antigen processing occur within cells (Rock & Goldberg (1999) Annu Rev Immunol 17:739-779). One pathway, which is largely restricted to professional antigen presenting cells such as dendritic cells, macrophages, and B cells, degrades proteins that are typically phagocytosed or endocytosed into the cell. Peptides derived from this pathway can be presented on either class I or to class II MHC molecules. A second pathway of antigen processing is present in essentially all cells of the body. This second pathway primarily degrades proteins that are made within the cells, and the peptides derived from this pathway primarily bind to class I MHC molecules. Antigen processing by this latter pathway involves polypeptide synthesis and proteolysis in the cytoplasm, followed by transport of peptides to the plasma membrane for presentation. These peptides, initially being transported into the endoplasmic reticulum of the cell, become associated with newly synthesized class I MHC molecules and the resulting complexes are then transported to the cell surface. Peptides derived from membrane and secreted proteins have also been identified. In some cases these peptides correspond to the signal sequence of the proteins which is cleaved from the protein by the signal peptidase. In other cases, it is thought that some fraction of the membrane and secreted proteins are transported from the endoplasmic reticulum into the cytoplasm where processing subsequently occurs. Once bound to the class I MHC molecule, the peptides are recognized by antigen-specific receptors on CTL. Several methods have been developed to identify the peptides recognized by CTL, each method of which relies on the ability of a CTL to recognize and kill only those cells expressing the appropriate class I MHC molecule with the peptide bound to it. Mere expression of the class I MHC molecule is insufficient to trigger the CTL to kill the target cell if the antigenic peptide is not bound to the class I MHC molecule. Such peptides can be derived from a non-self source, such as a pathogen (for example, following the infection of a cell by a bacterium or a virus) or from a self-derived protein within a cell, such as a cancerous cell. The tumor antigens from which the peptides are derived can broadly be categorized as differentiation antigens, cancer/testis antigens, mutated gene products, widely expressed proteins, viral antigens and most recently, phosphopeptides derived from dysregulated signal transduction pathways. (Zarling et al. (2006) Proc Natl Acad Sci USA 103:12889-14894).

Immunization with melanoma-derived, class I or class II MHC-encoded molecule associated peptides, or with a precursor polypeptide or protein that contains the peptide, or with a gene that encodes a polypeptide or protein containing the peptide, are forms of immunotherapy that can be employed in the treatment of ovarian cancer. Identification of the immunogens is a necessary first step in the formulation of the appropriate immunotherapeutic agent or agents. Although a large number of tumor-associated peptide antigens recognized by tumor reactive CTL have been identified, there are few examples of antigens that are derived from proteins that are selectively expressed on a broad array of tumors, as well as associated with cellular proliferation and/or transformation.

Attractive candidates for this type of antigen are peptides derived from proteins that are differentially phosphorylated on serine (Ser), threonine (Thr), and tyrosine (Tyr; Zarling et al. (2000) J Exp Med 192:1755-1762). Due to the increased and dysregulated phosphorylation of cellular proteins in transformed cells as compared to normal cells, tumors are likely to present a unique subset of phosphorylated peptides on the cell surface that are available for recognition by cytotoxic T-lymphocytes (CTL). Presently, there is no way to predict which protein phosphorylation sites in a cell will be unique to tumors, survive the antigen processing pathway, and be presented to the immune system in the context of 8-14 residue phosphopeptides bound to class I MHC molecules.

Thirty-six phosphopeptides were disclosed as presented in association with HLA A*0201 on cancer cells. *See* Table 1 of Zarling et al. (2006) Proc Natl Acad Sci U S A 103:14889-14894. Parent proteins for four of these peptides (beta-catenin, insulin receptor substrate-2 (IRS-2), tensin-3 and Jun-C/D) are associated with cytoplasmic signaling pathways and cellular transformation.

Until the present disclosure, no studies have examined MHC class-I-bound phosphopeptide displayed on primary human tumor samples and there is only limited evidence of a human immune response against class-I restricted phosphopeptides.

There is a need in the art for class I therapeutic peptide antigen based immunotherapies in general and for ovarian cancer in particular.

### SUMMARY

This Summary lists several embodiments of the presently disclosed subject matter, and in many cases lists variations and permutations of these embodiments. This Summary is merely exemplary of the numerous and varied embodiments. Mention of one or more representative features of a given embodiment is likewise exemplary. Such an embodiment can typically exist with or without the feature(s) mentioned; likewise, those features can be applied to other embodiments of the presently disclosed subject matter, whether listed in this Summary or not. To avoid excessive repetition, this Summary does not list or suggest all possible combinations of such features.

In some embodiments, the presently disclosed subject matter relates to compositions comprising at least or about 1, 2, 3, 4, 5, 6, 7, 8, 9, 10 or more synthetic target peptides each of which are about or at least 8, 9, 10, 11, 12, 13, 14 or 15 amino acids long wherein the target peptides comprise for example, amino acid sequences as set forth in any of SEQ ID NOs: 1-193; and wherein the composition has the ability to stimulate a T cell mediated immune response to at least one of the target synthetic peptides.

In some embodiments, at least one serine residue in any of the peptides is replaced with a homo-serine. In some embodiments, the composition comprises a non-hydrolyzable phosphate. In some embodiments, the composition is immunologically suitable for at least 60 to 88% of ovarian cancer patients. In some embodiments, the composition comprises at least 5 different target peptides. In some embodiments, the composition comprises at least 10 different target peptides. In some embodiments, the composition comprises at least 15 different target peptides. In some embodiments, the composition comprises a peptide capable of binding to an MHC class I molecule of the HLA-A*0201 allele.

In some embodiments, the composition is capable of increasing the 5-year survival rate of ovarian cancer patients treated with the composition by at least 20 percent relative to average 5-year survival rates that could have been expected without treatment with the composition. In some embodiments, the composition is capable of increasing the survival rate of ovarian cancer patients treated with the composition by at least 20 percent relative to a survival rate that could have been expected without treatment with the composition. In some embodiments, the composition is capable of increasing the treatment response rate of ovarian cancer patients treated with the composition by at least 20 percent relative to a treatment rate that could have been expected without treatment with the composition. In some embodiments, the composition is capable of increasing the overall median survival of patients of ovarian cancer patients treated with the composition by at least two months relative to an overall median survival that could have been expected without treatment with the composition.

In some embodiments, the composition comprises at least one peptide derived from MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP and TPS.

In some embodiments, the composition comprises an adjuvant selected from the group consisting of montanide ISA-51 (Seppic Inc., Fairfield, New Jersey, United States of America), QS-21 (Aquila Biopharmaceuticals, Inc., Framingham, Nassachusetts, United States of America), tetanus helper peptides (such as but not limited to QYIKANSKFIGITEL (SEQ ID NO: 242) and/or AQYIKANSKFIGITEL (SEQ ID NO: 234), GM-CSF, cyclophosamide, bacillus Calmette-Guerin (BCG), *Corynbacterium parvum,* levamisole, azimezone, isoprinisone, dinitrochlorobenezene (DNCB), keyhole limpet hemocyanins (KLH), Freunds adjuvant (complete and incomplete), mineral gels, aluminum hydroxide (Alum), lysolecithin, pluronic polyols, polyanions, peptides, oil emulsions, dinitrophenol, diphtheria toxin (DT).

In some embodiments, the presently disclosed subject matter relates to an *in vitro* population of dendritic cells comprising the aforementioned compositions or a composition comprising at least one target peptide.

In some embodiments, the presently disclosed subject matter relates to an *in vitro* population of CD8⁺ T cells capable of being activated upon being brought into contact with a population of dendritic cells, wherein the dendritic cells comprise the aforementioned compositions.

In some embodiments, the presently disclosed subject matter relates to an antibody or antibody-like molecule that specifically binds to both a first complex of MHC class I molecule and a target peptide. In some embodiments, the antibody or antibody-like molecule is a member of the immunoglobulin superfamily. In some embodiments, the antibody or antibody-like molecule comprises a binding member selected from the group consisting an Fab, Fab', F(ab')₂, Fv, and a single-chain antibody. In some embodiments, the antibody or antibody-like molecule comprises a therapeutic agent selected from the group consisting of an alkylating agent, an antimetabolite, a mitotic inhibitor, a taxoid, a vinca alkaloid and an antibiotic. In some embodiments, the antibody or antibody-like molecule is a T cell receptor, optionally linked to a CD3 agonist.

In some embodiments, the presently disclosed subject matter relates to an *in vitro* population of T cells transfected with mRNA encoding the aforementioned target peptide-specific T cell receptors.

In some embodiments, the presently disclosed subject matter relates to methods of treating or preventing cancer comprising administering to a patient in need thereof a dose of the aforementioned compositions.

In some embodiments, the presently disclosed subject matter relates to methods of treating or preventing ovarian cancer comprising administering to a patient in need thereof a dose of the aforementioned compositions with a pharmaceutically acceptable carrier.

In some embodiments, the presently disclosed subject matter relates to methods of treating or preventing cancer comprising administering to a patient in need thereof a dose of the aforementioned CD8⁺ T in combination with a pharmaceutically acceptable carrier.

In some embodiments, the presently disclosed subject matter relates to methods of treating or preventing cancer comprising administering to a patient in need thereof the population of the aforementioned dendritic cells in combination with a pharmaceutically acceptable carrier.

In some embodiments, the presently disclosed subject matter relates to methods of treating or preventing cancer comprising administering to a patient in need thereof the aforementioned population T cells in combination with a pharmaceutically acceptable carrier.

In some embodiments, the presently disclosed subject matter relates to methods of making a cancer vaccine comprising combining the aforementioned compositions with the aforementioned adjuvant and a pharmaceutically acceptable carrier; and placing the composition, adjuvant and pharmaceutical carrier into a syringe.

In some embodiments, the presently disclosed subject matter relates to methods of methods of screening target peptides for inclusion in an immunotherapy composition comprising administering the target peptide to a human; determining whether the target peptide is capable of inducing a target peptide-specific memory T cell response in the human; selecting the target peptide for inclusion in an immunotherapy composition if the target peptide elicits a memory T cell response in the human.

In some embodiments, the presently disclosed subject matter relates to a method of determining the prognosis of a cancer patient comprising: administering a target peptide associated with the patient's cancer to the patient; determining whether the target peptide is capable of inducing a target peptide-specific memory T cell response in the patient; determining that the patient has a better prognosis if the patient mounts a memory T cell response to the target peptide than if the patient did not mount a memory T cell response to the target peptide.

In some embodiments, the presently disclosed subject matter relates to a kit comprising at least one target peptide composition comprising at least one target peptide and a cytokine and/or an adjuvant. In some embodiments, the kit comprises at least 2, 3, 4 or 5 or more compositions.

In some embodiments, the cytokine is selected from the group consisting of transforming growth factors (TGFs) such as TGF-alpha and TGF-beta; insulin-like growth factor-I and -II; erythropoietin (EPO); osteoinductive factors; interferons such as interferon-alpha -beta, and -gamma; colony stimulating factors (CSFs) such as macrophage-CSF (M-CSF); granulocyte-macrophage-CSF (GM-CSF); and granulocyte-CSF (G-CSF).

In some embodiments, the adjuvant selected from the group consisting of montanide ISA-51 (Seppic, Inc.), QS-21 (Aquila Pharmaceuticals, Inc.), tetanus helper peptides, GM-CSF, cyclophosamide, bacillus Calmette-Guerin (BCG), corynbacterium parvum, levamisole, azimezone, isoprinisone, dinitrochlorobenezene (DNCB), keyhole limpet hemocyanins (KLH), Freunds adjuvant (complete and incomplete), mineral gels, aluminum hydroxide (Alum), lysolecithin, pluronic polyols, polyanions, peptides, oil emulsions, dinitrophenol, diphtheria toxin (DT).

In some embodiments, the cytokine is selected from the group consisting of nerve growth factors such as NGF-beta; platelet-growth factor; transforming growth factors (TGFs) such as TGF-alpha and TGF-beta; insulin-like growth factor-I and -II; erythropoietin (EPO); osteoinductive factors; interferons such as interferon-alpha - beta, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-13, IL-14, IL-15, IL-16, IL-17, IL-18, LIF, G-CSF, GM-CSF, M-CSF, EPO, kit-ligand, or FLT-3, angiostatin, thrombospondin, endostatin, tumor necrosis factor, and LT.

In some embodiments, the kit comprises at least one additional peptide derived from MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, and TPS.

In some embodiments, the kit comprises at least one target peptide that comprises an amino acid as set forth in any of SEQ ID NOs: 1-193.

These and other aspects and embodiments which will be apparent to those of skill in the art upon reading the specification provide the art with immunological tools and agents useful for diagnosing, prognosing, monitoring, and/or treating human cancers.

### BRIEF DESCRIPTION OF THE SEQUENCE LISTING

A more complete understanding of the presently disclosed subject matter can be obtained by reference to the accompanying Sequence Listing, when considered in conjunction with the subsequent Detailed Description. The embodiments presented in the Sequence Listing are intended to be exemplary only and should not be construed as limiting the presently disclosed subject matter to the listed embodiments, in which SEQ ID NOs: 1-193 provide a listing of exemplary MHC class I target peptides associated with ovarian cancer. Additional details with respect to SEQ ID NOs: 1-193 are provided in Table 3 herein below.

### DETAILED DESCRIPTION

While the following terms are believed to be well understood by one of ordinary skill in the art, the following definitions are set forth to facilitate explanation of the presently disclosed subject matter.

All technical and scientific terms used herein, unless otherwise defined below, are intended to have the same meaning as commonly understood by one of ordinary skill in the art. Mention of techniques employed herein are intended to refer to the techniques as commonly understood in the art, including variations on those techniques or substitutions of equivalent techniques that would be apparent to one of skill in the art. While the following terms are believed to be well understood by one of ordinary skill in the art, the following definitions are set forth to facilitate explanation of the presently disclosed subject matter. Thus, unless defined otherwise, all technical and scientific terms and any acronyms used herein have the same meanings as commonly understood by one of ordinary skill in the art in the field of the presently disclosed subject matter. Although any compositions, methods, kits, and means for communicating information similar or equivalent to those described herein can be used to practice the presently disclosed subject matter, particular compositions, methods, kits, and means for communicating information are described herein. It is understood that the particular compositions, methods, kits, and means for communicating information described herein are exemplary only and the presently disclosed subject matter is not intended to be limited to just those embodiments.

Following long-standing patent law convention, the terms "a", "an", and "the" refer to "one or more" when used in this application, including the embodiments. Thus, in some embodiments the phrase "a peptide" refers to one or more peptides.

The term "about", as used herein to refer to a measurable value such as an amount of weight, time, dose (*e.g.,* therapeutic dose), etc., is meant to encompass in some embodiments variations of ±20%, in some embodiments ±10%, in some embodiments ±5%, in some embodiments ±1%, in some embodiments ±0.1%, in some embodiments ±0.5%, and in some embodiments ±0.01% from the specified amount, as such variations are appropriate to perform the disclosed methods.

As used herein, the term "and/or" when used in the context of a list of entities, refers to the entities being present singly or in any and every possible combination and subcombination. Thus, for example, the phrase "A, B, C, and/or D" includes A, B, C, and D individually, but also includes any and all combinations and subcombinations of A, B, C, and D. It is further understood that for each instance wherein multiple possible options are listed for a given element (*i.e.,* for all "Markush Groups" and similar listings of optional components for any element), in some embodiments the optional components can be present singly or in any combination or subcombination of the optional components. It is implicit in these forms of lists that each and every combination and subcombination is envisioned and that each such combination or subcombination has not been listed simply merely for convenience. Additionally, it is further understood that all recitations of "or" are to be interpreted as "and/or" unless the context clearly requires that listed components be considered only in the alternative (*e.g.,* if the components would be mutually exclusive in a given context and/or could not be employed in combination with each other).

As used herein, the phrase "amino acid sequence as set forth in any of SEQ ID NOs: [A]-[B]" refers to any amino acid sequence that is disclosed in any one or more of SEQ ID NOs: A-B. In some embodiments, the amino acid sequence is any amino acid sequence that is disclosed in any of the SEQ ID NOs. that are present in the Sequence Listing. In some embodiments, the phrase refers to the full length sequence of any amino acid sequence that is disclosed in any of the SEQ ID NOs. that are present in the Sequence Listing, such that an "amino acid sequence as set forth in any of SEQ ID NOs: [A]-[B]" refers to the full length sequence of any of the sequences disclosed in the Sequence Listing. By way of example and not limitation, in some embodiments an "amino acid sequence as set forth in any of SEQ ID NOs: 1-193" refers to the full length amino acid sequence disclosed in any of SEQ ID NOs: 1-193 and not to a subsequence of any of SEQ ID NOs: 1-193.

The presently disclosed subject matter relates in some embodiments to post-translationally-modified immunogenic therapeutic target peptides, *e.g.,* phosphopeptides and/or O-GlcNAc peptides, for use in immunotherapy and diagnostic methods of using the target peptides, as well as methods of selecting the same to make compositions for immunotherapy, *e.g*., in vaccines and/or in compositions useful in adaptive cell transfer.

### I. Target Peptides

In some embodiments, the target peptides of the presently disclosed subject matter are post-translationally-modified by being provided with a phosphate group (referred to herein as "phosphopeptides") and/or an O-linked beta-N-acetylglucosamine ("O-GlcNAc") moiety (referred to herein as "O-GlcNAc peptides").

The target peptides of the presently disclosed subject matter are in some embodiments not the entire proteins from which they are derived. They are in some embodiments from 8 to 50 contiguous amino acid residues of the native human protein. They can in some embodiments contain exactly, about, or at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 amino acids. The peptides of the presently disclosed subject matter can also in some embodiments have a length that falls in the ranges of 8-10, 9-12, 10-13, 11-14, 12-15, 15-20, 20-25, 25-30, 30-35, 35-40, and 45-50 amino acids. Exactly, about, or at least 1, 2, 3, 4, 5, 6, 7, 8, 9, 10, 11, 12, 13, 14, 15, 16, 17, or more of the amino acid residues within the recited sequence of a target peptide can phosphorylated and/or contain an O-GlcNAc moiety.

Target peptides can be modified and analogs (using for example, beta-amino acids, L-amino acids, N-methylated amino acids, amidated amino acids, non-natural amino acids, retro inverse peptides, peptoids, PNA, halogenated amino acids) can be synthesized that retain their ability to stimulate a particular immune response, but which also gain one or more beneficial features, such as those described below. Thus, particular target peptides can, for example, have use for treating and vaccinating against multiple cancer types.

In some embodiments, substitutions can be made in the target peptides at residues known to interact with the MHC molecule. Such substitutions can in some embodiments have the effect of increasing the binding affinity of the target peptides for the MHC molecule and can also increase the half-life of the target peptide-MHC complex, the consequence of which is that the analog is in some embodiments a more potent stimulator of an immune response than is the original peptide.

Additionally, the substitutions can in some embodiments have no effect on the immunogenicity of the target peptide *per se,* but rather can prolong its biological half-life or prevent it from undergoing spontaneous alterations which might otherwise negatively impact on the immunogenicity of the peptide.

The target peptides disclosed herein can in some embodiments have differing levels of immunogenicity, MHC binding and ability to elicit CTL responses against cells displaying a native target peptide, *e.g.,* on the surface of a tumor cell.

The amino acid sequences of the target peptides can in some embodiments be modified such that immunogenicity and/or binding is enhanced. In some embodiments, the modified target peptide binds an MHC class I molecule about or at least 10%, 20%, 30%, 40%, 50%, 60%, 70%, 80%, 90%, 95%, 100%, 110%, 125%, 150%, 175%, 200%, 225%, 250%, 275%, 300%, 350%, 375%, 400%, 450%, 500%, 600%, 700%, 800%, 1000%, or more tightly than its native (unmodified) counterpart.

However, given the exquisite sensitivity of the T-cell receptor, it cannot be foreseen whether such enhanced binding and/or immunogenicity will render a modified target peptide still capable of inducing an activated CTL that will cross react with the native target peptide being displayed on the surface of a tumor. Indeed, it is disclosed herein that the binding affinity of a target peptide does not predict its functional ability to elicit a T cell response.

Target peptides of the presently disclosed subject matter can in some embodiments be mixed together to form a cocktail. The target peptides can in some embodiments be in an admixture, or they can in some embodiments be linked together in a concatamer as a single molecule. Linkers between individual target peptides can in some embodiments be used; these can, for example, in some embodiments be formed by any 10 to 20 amino acid residues. The linkers can in some embodiments be random sequences, or they can in some embodiments be optimized for degradation by dendritic cells.

In certain specified positions, a native amino acid residue in a native human protein can in some embodiments be altered to enhance the binding to the MHC class I molecule. These can occur in "anchor" positions of the target peptides, often in positions 1, 2, 3, 9, or 10. Valine, alanine, lysine, leucine tyrosine, arginine, phenylalanine, proline, glutamic acid, threonine, serine, aspartic acid, tryptophan, and methionine can also be used in some embodiments as improved anchoring residues. Anchor residues for different HLA molecules are listed below. Anchor residues for HLA molecules are listed in Table 1.

**Table 1**

| Anchor Residues for Different HLA Molecules | |
|---|---|
| HLA A^{∗}0201 | Residue 2 = L, M |
| | Residue 9 or last residue = V |
| HLA A^{∗}0301 | Residue 2 = L, M |
| | Residue 9 or last residue = K |
| HLA A^{∗}0101 | Residue 2 = T, S |
| | Residue 3 = D, E |
| | Residue 9 or last residue = Y |
| HLA B^{∗}2705 | Residue 1 = R |
| | Residue 2 = R |
| | Residue 9 or last residue L, F, K, R, M |
| HLA B^{∗}0702 | Residue 2 = P |
| | Residue 9 or last residue = L, M, V, F |
| HLA B^{∗}4402 | Residue 2 = E |
| | Residue 9 or last residue = F, Y, W |

In some embodiments, the immunogenicity of a target peptide is measured using transgenic mice expressing human MHC class I genes. For example, "ADD Tg mice" express an interspecies hybrid class I MHC gene, AAD, which contains the alpha-1 and alpha-2 domains of the human HLA-A2.1 gene and the alpha-3 transmembrane and cytoplasmic domains of the mouse H-2Dd gene, under the direction of the human HLA-A2.1 promoter. Immunodetection of the HLA-A2.1 recombinant transgene established that expression was at equivalent levels to endogenous mouse class I molecules. The mouse alpha-3 domain expression enhances the immune response in this system. Compared to unmodified HLA-A2.1, the chimeric HLA-A2.1/H2-Dd MHC Class I molecule mediates efficient positive selection of mouse T cells to provide a more complete T cell repertoire capable of recognizing peptides presented by HLA-A2.1 Class I molecules. The peptide epitopes presented and recognized by mouse T cells in the context of the HLA-A2.1/H2-Dd class I molecule are the same as those presented in HLA-A2.1⁺ humans. This transgenic strain facilitates the modeling of human T cell immune responses to HLA-A2 presented antigens, and identification of those antigens. This transgenic strain is a preclinical model for design and testing of vaccines for infectious diseases or cancer therapy involving optimal stimulation of CD8⁺ cytolytic T cells.

In some embodiments, the immunogenicity of a modified target peptide is determined by the degree of Interferon gamma and/or TNF-alpha production of T-cells from ADD Tg mice immunized with the target peptide, *e.g.,* by immunization with target peptide pulsed bone marrow derived dendritic cells.

In some embodiments, the modified target peptides are about or at least 10%, 20%, 30%, 40%, 50%, 60%, 70%, 80%, 90%, 95%, 100%, 110%, 125%, 150%, 175%, 200%, 225%, 250%, 275%, 300%, 350%, 375%, 400%, 450%, 500%, 600%, 700%, 800%, 1000%, 1500%, 2000%, 2500%, 3000%, 4000%, 5000%, or more immunogenic, *e.g*., in terms of numbers of Interferon gamma and/or TNF-alpha positive (*i.e.,* "activated") T-cells relative to numbers elicited by native target peptides in ADD Tg mice immunized with target peptides pulsed bone marrow derived dendritic cells. In some embodiments, the modified target peptides are able to elicit CD8⁺ T cells which are cross-reactive with the modified and the native target peptide in general and when such modified and native target peptides are complexed with MHC class I molecules in particular. In some embodiments, the CD8⁺ T cells which are cross-reactive with the modified and the native target peptides are able to reduce tumor size by about or at least 10%, 20%, 30%, 40%, 50%, 60%, 70%, 80%, 90%, 95%, 97%, or 99% in a NOD/SCID/IL-2Ryc^{-/-} knock out mouse (which has been provided transgenic T cells specific form an immune competent donor) relative to IL-2 treatment without such cross-reactive CD8⁺ T cells.

The term "capable of inducing a target peptide-specific memory T cell response in a patient" as used herein relates to eliciting a response from memory T cells (also referred to as "antigen-experienced T cell") which are a subset of infection- and cancer-fighting T cells that have previously encountered and responded to their cognate antigen. Such T cells can recognize foreign invaders, such as bacteria or viruses, as well as cancer cells. Memory T cells have become "experienced" by having encountered antigen during a prior infection, encounter with cancer, or previous vaccination. At a second encounter with the cognate antigen, *e.g.,* by way of an initial inoculation with a target peptide of the invention, memory T cells can reproduce to mount a faster and stronger immune response than the first time the immune system responded to the invader (*e.g*., through the body's own consciously unperceived recognition of a target peptide being associated with diseased tissue). This behavior can be assayed in T lymphocyte proliferation assays, which can reveal exposure to specific antigens. Memory T cells comprise two subtypes: central memory T cells (T_{CM} cells) and effector memory T cells (T_{EM} cells). Memory cells can be either CD4⁺ or CD8⁺. Memory T cells typically express the cell surface protein CD45RO. Central memory T_{CM} cells generally express L-selectin and CCR7, they secrete IL-2, but not IFNγ or IL-4. Effector memory T_{EM} cells, however, generally do not express L-selectin or CCR7 but produce effector cytokines like IFNγ and IL-4.

A memory T cell response generally results in the proliferation of memory T cell and/or the upregulation or increased secretion of the factors such as CD45RO, L-selectin, CCR7, IL-2, IPNγ, CD45RA, CD27 and/or IL-4. In some embodiments, the target peptides of the presently disclosed subject matter are capable of inducing a T_{CM} cell response associated with L-selectin, CCR7, IL-2 (but not IFNγ or IL-4) expression and/secretion. *See e.g.,* Hamann et al. (1997) J Exp Med 186:1407-1418. In some embodiments, a T_{CM} cell response is associated with an at least or about 1%, 5%, 10%, 15%, 20%, 25%, 30%, 35%, 45%, 50%, 55%, 60%, 65%, 70%, 75%, 80%, 90%, 95%, 97%, 98%, 99%, 100%, 125%, 150%, 175%, 200%, 250%, 300%, 400%, 500%, 600%, 700%, 800%, 900%, 1000%, 1500%, 2000%, or more increase in T cell CD45RO/RA, L-selectin, CCR7, or IL-2 expression and/secretion.

In some embodiments, the target peptides of the presently disclosed subject matter are capable of inducing a CD8⁺ T_{CM} cell response in a patient the first time that patient is provided the composition including the selected target peptides. As such, the target peptides of the presently disclosed subject matter can in some embodiments be referred to as "neo-antigens". Although target peptides might be considered "self' for being derived from self-tissue, they generally are only found on the surface of cells with a dysregulated metabolism, *e.g*., aberrant phosphorylation, they are likely never presented to immature T cells in the thymus. As such, these "self' antigens act are neo-antigens because they are nevertheless capable of eliciting an immune response.

In some embodiments, about or at least 1%, 5%, 10%, 15%, 20%, 25%, 30%, 35%, 45%, 50%, 55%, 60%, 65%, 70%, 75%, 80%, 90%, 95%, 97%, 98%, or 99% of T cells activated by particular target peptide in a particular patient sample are T_{CM} cells. In some embodiments, a patient sample is taken exactly, about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more days after an initial exposure to a particular target peptide and then assayed for target peptide specific activated T cells and the proportion of T_{CM} cells thereof. In some embodiments, the compositions of the presently disclosed subject matter are able to elicit a CD8⁺ T_{CM} cell response in at least or about 1%, 5%, 10%, 15%, 20%, 25%, 30%, 35%, 45%, 50%, 55%, 60%, 65%, 70%, 75%, 80%, 90%, 95%, 97%, 98%, 99%, or 100% of patients and/or healthy volunteers. In some embodiments, the compositions of the presently disclosed subject matter are able to elicit a CD8⁺ T_{CM} cell response in a patient about or at least 1%, 5%, 10%, 15%, 20%, 25%, 30%, 35%, 45%, 50%, 55%, 60%, 65%, 70%, 75%, 80%, 90%, 95%, 97%, 98%, 99%, or 100% of patients and/or healthy volunteers specific to all or at least or about 1, 2, 3, 4, 5, 6, 7, 8, 9, or 10 target peptides in the composition. In some embodiments, the aforementioned T cell activation tests are done by ELISpot assay.

### II. O-GlcNAc Peptides

The term "O-GlcNAc peptides" includes MHC class I and MHC class II specific O-GlcNAc peptides.

Modification of proteins with O-linked β-N-acetylglucosamine (O-GlcNAc) was previously technically difficult to detect. However, it rivals phosphorylation in both abundance and distribution of the protein targets for this modification. Like phosphorylation, O-GlcNAcylation is a reversible modification of nuclear and cytoplasmic proteins and consists of the attachment of a single β-N-acetylglucosamine moiety to hydroxyl groups of serine or threonine residues. Modification by O-GlcNAcylation is often competitive with phosphorylation at the same sites or at proximal sites on proteins. Furthermore, crosstalk between O-GlcNAcylation and phosphorylation affects the posttranslational state of hundreds of proteins in response to nutrients and stress and plays an important role in chronic diseases of metabolism, such as diabetes and neurodegeneration.

O-GlcNAc transferase (OGT) catalyzes the addition of the sugar moiety from the donor substrate uridine 5'-diphosphate (UDP)-GlcNAc to proteins. During M phase, OGT localizes to discrete structures, such as centrosomes (metaphase) and the spindle (anaphase), and then moves to the midbody during cytokinesis. OGT, along with O-GlcNAcase (OGA), the enzyme that removes the sugar, dynamically interacts with AURKB and PP1 at the midbody. Together, these proteins form a complex regulating M-phase O-GlcNAcylation, which in turn influences the phosphorylation state, of vimentin. However, the identity of other OGT mitotic substrates is currently not known.

Peptides modified with O-GlcNAc can be difficult to detect by standard mass spectrometric methods. The modification is usually present at sub-stoichiometric amounts, modified and unmodified peptides co-elute during high-performance liquid chromatography (HPLC), and ionization of the modified peptide is suppressed in the presence of unmodified peptides. Consequently, sample enrichment is often required to successfully detect and characterize O-GlcNAcylated peptides. Enrichment can be achieved through chemoenzymatic approaches that biotinylate O-GlcNAc peptides and capture them by avidin chromatography. Alternatively, a chemoenzymatic approach using a photocleavable biotin-alkyne reagent (PCbiotin- alkyne) tag can be used (*see* Fig. S1A of Wang et al. (2010) Sci Signal 3(104):ra2 (hereinafter "Wang", incorporated herein by reference). Photocleavage not only allows efficient and quantitative recovery from the affinity column, but also tags the peptide with a charged moiety that facilitates O-GlcNAc site mapping by electron-transfer dissociation (ETD) mass spectrometry. This tagging approach also makes it possible to use conventional collision-activated dissociation mass spectrometry (CAD MS) to screen samples for the presence of O-GlcNAc-modified peptides by monitoring for two-signature fragment ions characteristic of the tag (*see* Fig. S1B of Wang).

OGlcNAcylation rivals phosphorylation in both abundance and distribution of the modified proteins and alterations in O-GlcNAcylation disrupt both the chromosomal passenger complex, containing AURKB, INCENP, PP1, Borealin, and Surviven, and the circuits regulating CDK1 activity.

O-GlcNAc is nearly as abundant as phosphate on proteins associated with the spindle and midbody. Many of the O-GlcNAcylation sites identified are identical or proximal to known phosphorylation sites. O-GlcNAcylation and phosphorylation work together to control complicated mitotic processes, such as spindle formation. For example, OGT overexpression altered the abundance of transcripts and proteins encoded by several mitotic genes, changed the localization of NuMA1, and disrupted the chromosomal passenger complex and the CDK1 activation circuit.

An interplay exists between O-GlcNAcylation and phosphorylation for several protein classes, most noticeably transcriptional regulators and cytoskeletal proteins. Many of the O-GlcNAcylation and phosphorylation sites are located in the regulatory head domains of intermediate filament proteins. Phosphorylation of these sites causes filament disassociation during M phase. For example, vimentin is phosphorylated at multiple sites during M phase and there is an O-GlcNAcylation site that is also a mitotic phosphorylation site (Ser55; Slawson et al. (2005) J Biol Chem 280:32944-32956; Slawson et al. (2008) Mol Biol Cell 19:4130-4140; Wang et al. (2007) Mol Cell Proteomics 6:1365-1379; Molina et al. (2007) Proc Natl Acad Sci U S A 104:2199-2204). There are three additional O-GlcNAcylation sites on vimentin at Ser7, Thr33, and Ser34 *(see* Tables S5 and S6 of Wang), all of which are in the regulatory head domain of the protein. Two of these, Ser7 and Ser34, are also phosphorylation sites (Dephoure et al. (2008) Proc Natl Acad Sci U S A 105:10762-10767; Molina et al. (2007) Proc Natl Acad Sci U S A 104:2199-2204). Signaling pathways involving cytoskeletal proteins are regulated by reciprocal occupancy on specific sites by phosphate and O-GlcNAc. In these classes of molecules, areas of multiple phosphorylation are also likely to be targeted for OGlcNAcylation.

OGT overexpression profoundly affects multiple mitotic signaling circuits. Although overexpression of OGT does not interfere with the formation of the midbody complex or localization of AURKB, AURKB activity is altered toward the cytoskeletal protein, vimentin. The reduction in the abundance of AURKB or INCENP dampens kinase activity to a point that retards mitotic progression especially during anaphase and telephase. Furthermore, OGT overexpression reduced phosphorylation of INCENP and borealin, but to what extent this alters the function of the midbody complex is unclear.

Multiple components of the cyclin B-CDK1 activation circuit were disrupted by the overexpression of OGT. The loss of PLK1 inhibitory phosphorylation on MYT1 and the increase in the abundance of MYT1 are likely contributors to the loss in cyclin B-CDK1 activity observed in OGT-overexpressing cells *(see* Fig. 7 of Wang). However, the reduction in cyclin B-CDK1 activity is likely only partially due to the increase in MYT1 activity, because the mRNA for CDC25C, the key CDK1 dual-specific phosphatase, is substantially reduced. The "on" switch for CDK1 activation, the reduction of MYT1 and the increase in CDC25C activity, is pushed toward "off' by OGT overexpression. Both MYT1 and CDC25C are substrates for PLK1. The protein and transcript abundance of PLK1 is substantially reduced in response to OGT overexpression, but there is little change in the extent of activating phosphorylation of PLK1.

Because O-GlcNAcylation is directly coupled to nutrient uptake and metabolism, the sugar residue is an ideal metabolic sensor for regulating mitotic progression. Whereas, phosphorylation might act as a master switch initiating the mitotic process, O-GlcNAcylation might act as an adjuster of signals to make these processes more responsive to environmental cues. How O-GlcNAcylation exerts control on specific mitotic proteins and how OGlcNAcylation will integrate into well-known signaling pathways represent another layer of cellular regulation.

### III. Phosphopeptides

The term "phosphopeptides" includes MHC class I and MHC class II specific phosphopeptides. Exemplary MHC class I phosphopeptides of the presently disclosed subject matter are set forth in SEQ ID NOs: 1-193, for example.

In some embodiments, the phosphopeptides of the presently disclosed subject matter comprise the sequences of at least one of the MHC class I binding peptides listed in SEQ ID NOs: 1-193. Moreover, in some embodiments about or at least 1, 2, 3, 4, 5, 6, 7, 8, 9, 10, 11, 12, 13, 14, 15, or more of the serine, homo-serine, threonine, or tyrosine residues within the recited sequence is phosphorylated. The phosphorylation can in some embodiments be with a natural phosphorylation (-CH₂-O-PO₃H) or with an enzyme non-degradable, modified phosphorylation, such as (-CH₂-CF₂-PO₃H or -CH₂- CH₂-PO₃H). Some phosphopeptides can contain more than one of the peptides listed in SEQ ID NOs: 1-193, for example, if they are overlapping, adjacent, or nearby within the native protein from which they are derived.

The chemical structure of a phosphopeptide mimetic appropriate for use in the presently disclosed subject matter can in some embodiments closely approximate the natural phosphorylated residue which is mimicked, and also can in some embodiments be chemically stable (*e.g*., resistant to dephosphorylation by phosphatase enzymes). This can be achieved with a synthetic molecule in which the phosphorous atom is linked to the amino acid residue, not through oxygen, but through carbon. In some embodiments, a CF₂ group links the amino acid to the phosphorous atom. Mimetics of several amino acids which are phosphorylated in nature can be generated by this approach. Mimetics of phosphoserine, phosphothreonine, and phosphotyrosine can be generated by placing a CF₂ linkage from the appropriate carbon to the phosphate moiety. The mimetic molecule L-2-amino-4 (diethylphosphono)-4,4-difluorobutanoic acid (F2Pab) can in some embodiments substitute for phosphoserine (Otaka et al., Tetrahedron Letters 36: 927-930 (1995)). L-2-amino-4-phosphono-4,4difluoro-3-methylbutanoic acid (F2Pmb) can in some embodiments substitute for phosphothreonine. L-2-amino-4-phosphono (difluoromethyl) phenylalanine (F2Pmp) can in some embodiments substitute for phosphotyrosine (Akamatsu et al. (1997) Bioorg Med Chem 5:157-163; Smyth et al. (1992) Tetrahedron Lett 33:4137-4140). Alternatively, the oxygen bridge of the natural amino acid can in some embodiments be replaced with a methylene group. In some embodiments, serine and threonine residues are substituted with homo-serine and homo-threonine residues, respectively. A phosphomimetic can in some embodiments also include vanadate, pyrophosphate or fluorophosphates.

### IV. Immunosuitablity

In some embodiments, the target peptides of the presently disclosed subject matter are combined into compositions which can be used in vaccine compositions for eliciting anti-tumor immune responses or in adoptive T-cell therapy of ovarian cancer patients. Table 3 provides target peptides presented on the surface of cancer cells.

Although individuals in the human population display hundreds of different HLA alleles, some are more prevalent than others. For example, 88% of melanoma patients carry at least one of the six HLA alleles: HLA-A*0201 (51%), HLA-A*0101(29%), HLA-A*0301 (21%), HLA-A*4402 (27%), HLA-A*0702 (30%), and HLA-A*2705 (7%).

The presently disclosed subject matter provides in some embodiments target peptides which are immunologically suitable for each of the foregoing HLA alleles and, in particular, HLA-A*0201. "Immunologically suitable" means that a target peptide will bind at least one allele of an MHC class I molecule in a given patient. Compositions of the presently disclosed subject matter are in some embodiments immunologically suitable for a patient when at least one target peptide of the composition will bind at least one allele of an MHC class I molecule in a given patient. Compositions of multiple target peptides presented by each of the most prevalent alleles used in a cocktail, ensures coverage of the human population and to minimize the possibility that the tumor will be able to escape immune surveillance by down-regulating expression of any one class I target peptide.

The compositions of the presently disclosed subject matter can in some embodiments have at least one target peptide specific for HLA-A*0201. The compositions can in some embodiments have at least one phosphopeptide specific from at least the HLA-A*0201 allele. In some embodiments, the compositions can further comprise additional phosphopeptides from other MHC class I alleles.

As such, the compositions of the presently disclosed subject matter containing various combinations of target peptides will in some embodiments be immunologically suitable for between or about 3-88%, 80-89%, 70-79%, 60-69%, 57-59%, 55-57%, 53-55% or 51-53% or 5-90%, 10-80%, 15-75%, 20-70%, 25-65%, 30-60%, 35-55%, or 40-50% of the population of a particular cancer, *e.g*., ovarian cancer. In some embodiments, the compositions of the presently disclosed subject matter are able to act as vaccine compositions for eliciting anti-tumor immune responses or in adoptive T-cell therapy of ovarian cancer patients, wherein the compositions are immunologically suitable for about or at least 88, 87, 86, 85, 84, 83, 82, 81, 80, 79, 78, 77, 76,75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 20, 19, 18, 17, 16, 15, 14, 13, 12, 11, 10, 9, 8, 7, 6, 5, 4 or 3 percent of cancer, *e.g.*, ovarian cancer, patients.

### V. Compositions

"Target peptide compositions" as used herein refers to at least one target peptide formulated for example, as a vaccine; or as a preparation for pulsing cells in a manner such that the pulsed cells, *e.g.,* dendritic cells, will display the at least one target peptide in the composition on their surface, *e.g.,* to T-cells in the context of adoptive T-cell therapy.

The compositions of the presently disclosed subject matter can include in some embodiments about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 50-55, 55-65, 65-80, 80-120, 90-150, 100-175, or 175-250 different target peptides.

The compositions of the presently disclosed subject matter generally include MHC class I specific target peptide(s) but in some embodiments can also include one or more target peptides specific for MHC class II or other peptides associated with tumors, *e.g.*, tumor-associated antigen ("TAA").

Compositions comprising the presently disclosed target peptide are typically substantially free of other human proteins or peptides. They can be made synthetically or by purification from a biological source. They can be made recombinantly. In some embodiments, they are at least 90%, 92%, 93%, 94%, at least 95%, or at least 99% pure. For administration to a human body, in some embodiments they do not contain other components that might be harmful to a human recipient. The compositions are typically devoid of cells, both human and recombinant producing cells. However, as noted below, in some cases, it can be desirable to load dendritic cells with a target peptide and use those loaded dendritic cells as either an immunotherapy agent themselves, or as a reagent to stimulate a patient's T cells *ex vivo.* The stimulated T cells can be used as an immunotherapy agent. In some embodiments, it can be desirable to form a complex between a target peptide and an HLA molecule of the appropriate type. Such complexes can in some embodiments be formed *in vitro* or *in vivo.* Such complexes are typically tetrameric with respect to an HLA-target peptide complex. Under certain circumstances it can be desirable to add additional proteins or peptides, for example, to make a cocktail having the ability to stimulate an immune response in a number of different HLA type hosts. Alternatively, additional proteins or peptide can provide an interacting function within a single host, such as an adjuvant function or a stabilizing function. As a non-limiting example, other tumor antigens can be used in admixture with the target peptides, such that multiple different immune responses are induced in a single patient.

Administration of target peptides to a mammalian recipient can in some embodiments be accomplished using long target peptides, *e.g.,* longer than 15 residues, or using target peptide loaded dendritic cells. *See* Melief (2009) J Med Sci 2:43-45. The immediate goal is to induce activation of CD8⁺ T cells. Additional components which can be administered to the same patient, either at the same time or close in time (*e.g.*, within 21 days of each other) include TLR-ligand oligonucleotide CpG and related target peptides that have overlapping sequences of at least 6 amino acid residues. To ensure efficacy, mammalian recipients should express the appropriate human HLA molecules to bind to the target peptides. Transgenic mammals can be used as recipients, for example, if they express appropriate human HLA molecules. If a mammal's own immune system recognizes a similar target peptide then it can be used as model system directly, without introducing a transgene. Useful models and recipients can in some embodiments be at increased risk of developing metastatic cancer, such as metastatic ovarian cancer. Other useful models and recipients can be predisposed, *e.g*., genetically or environmentally, to develop ovarian cancer or other cancer.

### V.A. Selection of Target Peptides

Disclosed herein is the finding that immune responses can be generated against phosphorylated peptides tested in healthy and diseased individuals. The T-cells associated with these immune responses, when expanded *in vitro,* are able to recognize and kill malignant tissue (both established cells lines and primary tumor samples). Cold-target inhibition studies reveal that these target peptide-specific T-cell lines kill primary tumor tissue in a target peptide-specific manner.

When selecting target peptides of the presently disclosed subject matter for inclusion in immunotherapy, *e.g.*, in adaptive cell therapy or in the context of a vaccine, one can preferably pick target peptides that in some embodiments: 1) are associated with a particular cancer/tumor cell type; 2) are associated with a gene/protein involved in cell proliferation; 3) are specific for an HLA allele carried the group of patients to be treated; and/or 4) are capable of inducing a target peptide-specific memory T cell response in the patients to be treated upon a first exposure to a composition including the selected target peptides.

### V.B. Target peptide Vaccines

The antigen target peptides can also in some embodiments be used to vaccinate an individual. The antigen target peptides can be injected alone or in some embodiments can be administered in combination with an adjuvant and a pharmaceutically acceptable carrier. Vaccines are envisioned to prevent or treat certain diseases in general and cancers in particular.

The target peptides compositions of the presently disclosed subject matter can in some embodiments be used as a vaccine for cancer, and more specifically for melanoma, leukemia, ovarian, breast, colorectal, or lung squamous cancer, sarcoma, renal cell carcinoma, pancreatic carcinomas, squamous tumors of the head and neck, brain cancer, liver cancer, prostate cancer, and cervical cancer. The compositions can in some embodiments include target peptides. The vaccine compositions can in some embodiments include only the target peptides, or peptides disclosed herein, or they can include other cancer antigens that have been identified.

The vaccine compositions can in some embodiments be used prophylactically for the purposes of preventing, reducing the risk of, and/or delaying initiation of a cancer in an individual that does not currently have cancer. Alternatively, they can be used to treat an individual that already has cancer, so that recurrence or metastasis is delayed and/or prevented. Prevention relates to a process of prophylaxis in which the individual is immunized prior to the induction or onset of cancer. For example, individuals with a history of poor life style choices and at risk for developing ovarian cancer can in some embodiments be immunized prior to the onset of the disease.

Alternatively or in addition, individuals that already have cancer can be immunized with the antigens of the presently disclosed subject matter so as to stimulate an immune response that would be reactive against the cancer. A clinically relevant immune response would be one in which the cancer partially or completely regresses and/or is eliminated from the patient, and it would also include those responses in which the progression of the cancer is blocked without being eliminated. Similarly, prevention need not be total, but can in some embodiments result in a reduced risk, delayed onset, and/or delayed progression or metastasis.

The target peptide vaccines of the presently disclosed subject matter can in some embodiments be given to patients before, after, or during any of the aforementioned stages of ovarian cancer. In some embodiments, they are given to patients with stage malignant ovarian cancer.

In some embodiments, the 5-year survival rate of patients treated with the vaccines of the presently disclosed subject matter is increased by a statistically significant amount, *e.g.,* by about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more percent, relative to the average 5-year survival rates described above.

In some embodiments, the target peptide vaccine composition of the presently disclosed subject matter will increase survival rates in patients with metastatic ovarian cancer by a statistically significant amount of time, *e.g.,* by about or at least, 0.25, 0.5, 0.75, 1.0, 1.25, 1.5, 1.75, 2.0, 2.25, 2.5, 2.75, 3.0, 3.25, 3.5, 4.0, 4.25, 4.5, 4.75, 5.0, 5.25, 5.5, 5.75, 6.0, 6.25, 6.5, 6.75, 7.0, 7.25, 7.5, 7.75, 8.0, 8.25, 8.5, 8.75, 9.0, 9.25, 9.50, 9.75, 10.0, 10.25, 10.5, 10.75, 11.0, 11.25, 11.5, 11.75, or 12 months or more compared to what could have been expected without vaccine treatment at the time of filing of this disclosure.

In some embodiments, the survival rate, *e.g.,* the 1, 2, 3, 4, or 5-year survival rate, of patients treated with the vaccines of the presently disclosed subject matter is increased by a statistically significant amount, *e.g.,* by about, or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 percent, relative to the average 5-year survival rates described above.

The target peptide vaccines of the presently disclosed subject matter are in some embodiments envisioned to illicit a T cell associated immune response, *e.g.*, generating activated CD8⁺ T cells specific for native target peptide/MHC class I expressing cells, specific for at least or about 1, 2, 3, 4, 5, 6, 7, 8, 9, 10, or more of the target peptides in the vaccine in a patient for about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 07, 98, 99, or 100 days after providing the vaccine to the patient.

In some embodiments, the treatment response rates of patients treated with the target peptide vaccines of the presently disclosed subject matter are increased by a statistically significant amount, *e.g.,* by about, or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 07, 98, 99, 100, 150, 200, 250, 300, 350, 400, 450, 500, or more percent, relative to treatment without the vaccine.

In some embodiments, overall median survival of patients treated with the target peptide vaccines of the presently disclosed subject matter is increased by a statistically significant amount, *e.g.,* by about, or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 350, 400, 450, 500, or more percent, relative to treatment without the vaccine. In some embodiments, the overall median survival of ovarian cancer patients treated the target peptide vaccines is envisioned to be about or at least 10.0, 10.25, 10.5, 10.75, 11.0, 11.25, 11.5, 11.75, 12, 12.25, 12.5, 12.75, 13, 13.25, 13.5, 13.75, 14, 14.25, 14.5, 14.75, 15, 16, 17, 18, 19, 20, 21, 22, 23, 24, 25, 26, 27, 28, 29, 30, 31, 32, 33, 34, 35, 36, or more months.

In some embodiments, tumor size of patients treated with the target peptide vaccines of the presently disclosed subject matter is decreased by a statistically significant amount, *e.g.,* by about, or by at least, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 350, 400, 450, 500, or more percent, relative to treatment without the vaccine.

In some embodiments, the compositions of the presently disclosed subject matter provide an clinical tumor regression by a statistically significant amount, *e.g*., in about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 percent of patients treated with a composition of the presently disclosed subject matter.

In some embodiments, the compositions of the presently disclosed subject matter provide a CTL response specific for the cancer being treated (such as but not limited to ovarian cancer) by a statistically significant amount, *e.g.,* in about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 percent of patients treated with a composition of the presently disclosed subject matter.

In some embodiments, the compositions of the presently disclosed subject matter provide an increase in progression free survival in the cancer being treated, *e.g*., ovarian cancer, of about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 225, 250, 275, 300, 325, 350, 375, 400, 425, 450, 475, 500, or more percent compared to the progression free survival or patients not treated with the composition.

In some embodiments, progression free survival, CTL response rates, clinical tumor regression rates, tumor size, survival rates (including but not limited to overall survival rates), and/or response rates are determined, assessed, calculated, and/or estimated weekly, monthly, bi-monthly, quarterly, semi-annually, annually, and/or bi-annually over a period of about or at least 1, 2, 3, 4, 5, 6, 7, 8, 9, 10, 11, 12, 13, 14, 15 or more years or about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 225, 250, 275, 300, 325, 350, 375, 400, 425, 450, 475, 500, or more weeks.

### V.C. Compositions for Priming T cells

Adoptive cell transfer is the passive transfer of cells, in some embodiments immune-derived cells, into a recipient host with the goal of transferring the immunologic functionality and characteristics into the host. Clinically, this approach has been exploited to transfer either immune-promoting or tolergenic cells (often lymphocytes) to patients to enhance immunity against cancer. The adoptive transfer of autologous tumor infiltrating lymphocytes (TIL) or genetically re-directed peripheral blood mononuclear cells has been used to successfully treat patients with advanced solid tumors, including melanoma and ovarian carcinoma, as well as patients with CD19-expressing hematologic malignancies. In some embodiments, adoptive cell transfer (ACT) therapies achieve T-cell stimulation *ex vivo* by activating and expanding autologous tumor-reactive T-cell populations to large numbers of cells that are then transferred back to the patient. *See e.g.,* Gattinoni et al. (2006) Nature Rev Immunol 6:383-393.

The target peptides of the presently disclosed subject matter can in some embodiments take the form of antigen peptides formulated in a composition added to autologous dendritic cells and used to stimulate a T helper cell or CTL response *in vitro.* The *in vitro* generated T helper cells or CTL can then be infused into a patient with cancer (Yee et al. (2002) Proc Natl Acad Sci U S A 99:16168-16173), and specifically a patient with a form of cancer that expresses one or more of antigen target peptides.

Alternatively or in addition, the target peptides of the presently disclosed subject matter can be added to dendritic cells *in vitro,* with the loaded dendritic cells being subsequently transferred into an individual with cancer in order to stimulate an immune response. Alternatively or in addition, the loaded dendritic cells can be used to stimulate CD8⁺ T cells *ex vivo* with subsequent reintroduction of the stimulated T cells to the patient. Although a particular target peptide can be identified on a particular cancer cell type, it can be found on other cancer cell types.

The presently disclosed subject matter envisions treating cancer by providing a patient with cells pulsed with a composition of target peptides. The use of dendritic cells ("DCs") pulsed with target peptide antigens allows for manipulation of the immunogen in two ways: varying the number of cells injected and varying the density of antigen presented on each cell. Exemplary methods for DC-based based treatments can be found for example in Mackensen et al. (2000) Int J Cancer 86:385-392.

### V.D. Additional Peptides Present in Target Peptide Compositions

The target peptide compositions (or target peptide composition kits) of the presently disclosed subject matter can in some embodiments also include at least one additional peptide derived from tumor-associated antigens. Examples of tumor-associated antigens include MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein/cyclophilin C-associated protein), TAAL6, TAG72, TLP, TPS, prostatic acid phosphatase, and the like. Particular examples of additional peptides derived from tumor-associated antigens that can be employed alone or in combination with the compositions of the presently disclosed subject matter those set forth in Table 2 below.

**Table 2**

| Exemplary Peptides Derived from Tumor-associated Antigens | | |
|---|---|---|
| Polypeptide Name^{a} | Amino Acid Sequence^{b} | Exemplary GENBANK® Acc. No(s).^{c} |
| CEA₆₁₋₆₉ | HLFGYSWYK (SEQ ID NO: 194) | NP_001264092.1 |
| | | XP_005278431.1 |
| CEA₆₀₄₋₆₁₂ | YLSGADLNL (SEQ ID NO: 195) | XP_005278431.1 |
| FBP/FOLR1₁₉₁₋₁₉₉ | EIWTHSYKV (SEQ ID NO: 196) | NP_000793.1 |
| gp100₁₇₋₂₅ | ALLAVGATK (SEQ ID NO: 197) | NP_001186982.1 |
| gp100₄₄₋₅₉ | WNRQLYPEWTEAQRLD (SEQ ID NO: 198) | NP_008859.1 |
| gp100₈₇₋₉₅ | ALNFPGSQK (SEQ ID NO: 199) | NP_008859.1 |
| gp100₈₉₋₉₅ | SQNFPGSQK (SEQ ID NO: 200) | NP_008859.1 |
| gp100₁₅₄₋₁₆₂ | KTWGQYWQV (SEQ ID NO: 201) | NP_008859.1 |
| gp100₂₀₉₋₂₁₇ | ITDQVPFSV (SEQ ID NO: 202) | NP_008859.1 |
| gp100₂₀₉₋₂₁₇ | IMDQVPFSV (SEQ ID NO: 203) | NP_008859.1 |
| gp100₂₈₀₋₂₈₈ | YLEPGPVTA (SEQ ID NO: 204) | NP_008859.1 |
| gp100₄₇₆₋₄₈₅ | VLYRYGSFSV (SEQ ID NO: 205) | NP_008859.1 |
| gp100₆₁₄₋₆₂₂ | LIYRRRLMK (SEQ ID NO: 206) | NP_008859.1 |
| Her2/neu₃₆₉₋₃₇₇ | KIFGSLAFL (SEQ ID NO: 207) | NP_004439.2 |
| Her2/neu₇₅₄₋₇₆₂ | VLRENTSPK (SEQ ID NO: 208) | NP_004439.2 |
| MAGE-A1₁₁₄₋₁₂₇ | LLKYRAREPVTKAE (SEQ ID NO: 209) | NP_004979.3 |
| MAGE-A2,3,6₁₂₁₋₁₃₄ | | NP_005352.1 |
| | | NP_005353.1 |
| | | NP_005354.1 |
| MAGE-A1₉₆₋₁₀₄ | SLFRAVITK (SEQ ID NO: 210) | NP_004979.3 |
| MAGE-A1₁₆₁₋₁₆₉ | EADPTGHSY (SEQ ID NO: 211) | NP_004979.3 |
| MAGE-A3₁₆₈₋₁₇₆ | EVDPIGHLY (SEQ ID NO: 212) | NP_005353.1 |
| MAGE-A3₂₈₁₋₂₉₅ | TSYVKVLHHMVKISG (SEQ ID NO: 213) | NP_005353.1 |
| MAGE-A10₂₅₄₋₂₆₂ | GLYDGMEHL (SEQ ID NO: 214) | NP_001011543.2 |
| MART-1/MelanA₂₇₋₃₅ | AAGIGILTV (SEQ ID NO: 215) | NP_005502.1 |
| MART-1/MelanA₅₁₋₇₃ | RNGYRALMDKSLHVGTQCALTRR (SEQ ID NO: 216) | NP_005502.1 |
| MART-1/MelanA₉₇₋₁₁₆ | VPNAPPAYEKLsAEQSPPPY (SEQ ID NO: 217) | NP_005502.1 |
| MART-1/MelanA₉₈₋₁₀₉ | PNAPPAYEKLsA (SEQ ID NO: 218) | NP_005502.1 |
| MART-1/MelanA₉₉₋₁₁₀ | PNAPPAYEKLsA (SEQ ID NO: 219) | NP_005502.1 |
| MART-1/MelanA₁₀₀₋₁₀₈ | APPAYEKLs (SEQ ID NO: 220) | NP_005502.1 |
| MART-1/MelanA₁₀₀₋₁₁₁ | APPAYEKLsAEQ (SEQ ID NO: 221) | NP_005502.1 |
| MART-1/MelanA₁₀₀₋₁₁₄ | APPAYEKLsAEQSPP (SEQ ID NO: 222) | NP_005502.1 |
| MART-1[MelanA₁₀₀₋₁₁₅ | APPAYEKLsAEQSPPP (SEQ ID NO: 223) | NP_005502.1 |
| MART-1/MelanA₁₀₀₋₁₁₆ | APPAYEKLsAEQSPPPY (SEQ ID NO: 224) | NP_005502.1 |
| MART-1/MelanA₁₀₁₋₁₀₉ | PPAYEKLsA (SEQ ID NO: 225) | NP_005502.1 |
| MART-1/MelanA₁₀₁₋₁₁₂ | PPAYEKLsAEQS (SEQ ID NO: 226) | NP_005502.1 |
| MART-1/MelanA₁₀₂₋₁₁₀ | PAYEKLsAE (SEQ ID NO: 227) | NP_005502.1 |
| MART-1/MelanA₁₀₂₋₁₁₃ | PAYEKLsAEQSP (SEQ ID NO: 228) | NP_005502.1 |
| MART-1/MelanA₁₀₃₋₁₁₄ | AYEKLsAEQSPP (SEQ ID NO: 229) | NP_005502.1 |
| MART-1/MelanA₁₀₄₋₁₁₅ | YEKLsAEQSPPP (SEQ ID NO: 230) | NP_005502.1 |
| NY-ESO-1 | AAQERRVPR (SEQ ID NO: 231) | AAD05203.1 |
| | | CAA10193.1 |
| NY-ESO-1 | LLGPGRPYR (SEQ ID NO: 232) | NP_001913.2 |
| NY-ESO-1₅₃₋₆₂ | ASGPGGGAPR (SEQ ID NO: 233) | NP_001318.1 |
| p2₈₃₀₋₈₄₄ | AQYIKANSKFIGITEL (SEQ ID NO: 234) | NP_783831.1 |
| TAG-1,2 | RLSNRLLLR (SEQ ID NO: 235) | |
| Tyr₅₆₋₇₀ | AQNILLSNAPLGPQFP (SEQ ID NO: 236) | NP_000363.1 |
| Tyr₁₄₆₋₁₅₆ | SSDYVIPIGTY (SEQ ID NO: 237) | NP_000363.1 |
| Tyr₂₄₀₋₂₅₁ | SDAEKSDICTDEY (SEQ ID NO: 238) | NP_000363.1 |
| Tyr₂₄₃₋₂₅₁ | KCDICTDEY (SEQ ID NO: 239) | NP_000363.1 |
| Tyr₃₆₉₋₃₇₇ | YMDGTMSQV (SEQ ID NO: 240) | NP_000363.1 |
| Tyr₃₈₈₋₄₀₆ | FLLHHAFVDSIFEQWLQRHRP (SEQ ID NO: 241) | NP_000363.1 |

| | | |
|---|---|---|
| ^{a} Numbers listed in subscript are the amino acids positions of the listed peptide sequence in the corresponding polypeptide including, but not limited to the amino acid sequences provided in the GENBANK® biosequence database. ^{b} lower case amino acids in this column are optionally phosphorylated. ^{c} GENBANK® biosequence database Accession Numbers listed here are intended to be exemplary only and should not be interpreted to limit the disclosed peptide sequences to only these polypeptides. | | |

Such tumor specific peptides (including the MHC class I phosphopeptides disclosed in SEQ ID NOs: 1-193 and in Table 3 can be added to the target peptide compositions in a manner, number, and/or in an amount as if they were an additional target peptide added to the target peptide compositions as described herein.

### V.E. Combination Therapies

In some embodiments, the target peptide compositions (or target peptide composition kits) of the presently disclosed subject matter are administered as a vaccine or in the form of pulsed cells as first, second, third, or fourth line treatment for the cancer. In some embodiments, the compositions of the presently disclosed subject matter are administered to a patient in combination with one or more therapeutic agents, *e.g.*, anti-CA125 (or oregovomab Mab B43.13), anti-idiotype Ab (ACA-125), anti-HER-2 (trastuzumab, pertuzumab), anti-MUC-1 idiotypic Ab (HMFG1), HER-2/neu peptide, NY-ESO-1, anti-Programed Death-1 ("PD1") (or PD1-antagonists such as BMS-936558), anti-CTLA-4 (or CTLA-4 antagonists), vermurafenib, ipilimumab, dacarbazine, IL-2, IFN-α, IFN-γ, temozolomide, receptor tyrosine kinase inhibitors (*e.g*., imatinib, gefitinib, erlotinib, sunitinib, tyrphostins, telatinib), sipileucel-T, tumor cells transfected with GM-CSF, a platinum-based agent, a taxane, an alkylating agent, an antimetabolite and/or a vinca alkaloid or combinations thereof. In an embodiment, the cancer is sensitive to or refractory, relapsed or resistant to one or more chemotherapeutic agents, *e.g.,* a platinum-based agent, a taxane, an alkylating agent, an anthracycline (*e.g.,* doxorubicin (*e.g.,* liposomal doxorubicin)), an antimetabolite and/or a vinca alkaloid. In some embodiments, the cancer is, *e.g.,* ovarian cancer, and the ovarian cancer is refractory, relapsed or resistant to a platinum-based agent (*e.g.,* carboplatin, cisplatin, oxaliplatin), a taxane (*e.g*., paclitaxel, docetaxel, larotaxel, cabazitaxel) and/or an anthracycline (*e.g.,* doxorubicin (*e.g.,* liposomal doxorubicin)). In some embodiments, the cancer is, *e.g*., ovarian cancer, and the cancer is refractory, relapsed or resistant to an antimetabolite (*e.g.,* an antifolate (*e.g.,* pemetrexed, floxuridine, raltitrexed) and a pyrimidine analogue (*e.g*., capecitabine, cytrarabine, gemcitabine, 5FU)) and/or a platinum-based agent (*e.g*., carboplatin, cisplatin, oxaliplatin). In some embodiments, the cancer is, *e.g.,* lung cancer, and the cancer is refractory, relapsed or resistant to a taxane (*e.g*., paclitaxel, docetaxel, larotaxel, cabazitaxel), a platinum-based agent (*e.g.,* carboplatin, cisplatin, oxaliplatin), a vinca alkaloid (*e.g.,* vinblastine, vincristine, vindesine, vinorelbine), a vascular endothelial growth factor (VEGF) pathway inhibitor, an epidermal growth factor (EGF) pathway inhibitor) and/or an antimetabolite (*e.g.,* an antifolate (*e.g.,* pemetrexed, floxuridine, raltitrexed) and a pyrimidine analogue (*e.g*., capecitabine, cytrarabine, gemcitabine, 5FU)). In some embodiments, the cancer is, *e.g.,* breast cancer, and the cancer is refractory, relapsed or resistant to a taxane (e.g., paclitaxel, docetaxel, larotaxel, cabazitaxel), a vascular endothelial growth factor (VEGF) pathway inhibitor, an anthracycline (*e.g*., daunorubicin, doxorubicin (*e.g*., liposomal doxorubicin), epirubicin, valrubicin, idarubicin), a platinum-based agent (*e.g.,* carboplatin, cisplatin, oxaliplatin), and/or an antimetabolite (*e.g.,* an antifolate (*e.g.,* pemetrexed, floxuridine, raltitrexed) and a pyrimidine analogue (*e.g.*, capecitabine, cytrarabine, gemcitabine, 5FU)). In some embodiments, the cancer is, *e.g.,* gastric cancer, and the cancer is refractory, relapsed or resistant to an antimetabolite (*e.g.,* an antifolate (*e.g.,* pemetrexed, floxuridine, raltitrexed) and a pyrimidine analogue (*e.g*., capecitabine, cytrarabine, gemcitabine, 5FU)) and/or a platinum-based agent (*e.g.,* carboplatin, cisplatin, oxaliplatin).

In some embodiments, the target peptide compositions (or target peptide composition kits) of the presently disclosed subject matter are associated with agents that inhibit T cell apoptosis or anergy thus potentiating a T cell response ("T cell potentiator"). Such agents include B7RP1 agonists, B7-H3 antagonists, B7-H4 antagonists, HVEM antagonists, HVEM antagonists, GAL9 antagonists or alternatively CD27 agonists, OX40 agonists, CD137 agonists, BTLA agonists, ICOS agonists CD28 agonists, or soluble versions of PDL1, PDL2, CD80, CD96, B7RP1, CD137L, OX40 or CD70. *See* Pardoll, National Reviews of Cancer, Focus on Tumour Immunology & Immunotherapy, 254, April 2012, Volume 12.

In some embodiments, the T cell potentiator is a PD1 antagonist. Programmed death 1 (PD-1) is a key immune checkpoint receptor expressed by activated T cells, and it mediates immunosuppression. PD-1 functions primarily in peripheral tissues, where T cells can encounter the immunosuppressive PD-1 ligands PD-L1 (B7-H1) and PD-L2 (B7-DC), which are expressed by tumor cells, stromal cells, or both. In some embodiments, the anti-PD-1 monoclonal antibody BMS-936558 (also known as MDX-1106 and ONO-4538) is used. In some embodiments, the T cell potentiator, *e.g.,* PD1 antagonist, is administered as an intravenous infusion at least or about every 1, 1.5, 2, 2.5, 3, 3.5, or 4 weeks of each 4, 5, 6, 7, 8, 9, or 10-week treatment cycle of about for at least 1, 2, 3, 4, 5, 6, 7, 8, 9, 10, 11, 12, 13, 14, 15, 16, 17, 18, 19, 20, or more cycles. Exemplary, non-limiting doses of the PD1 antagonists are envisioned to be exactly, about, or at least 0.1, 0.2, 0.3, 0.4, 0.5, 0.6, 0.7, 0.8, 0.9, 1, 2, 3, 4, 5, 6, 7, 8, 9, 10, 11, 12, 13,14, 15, 16, 17, 18, 19, 20, or more mg/kg. *See* Brahmer et al., N Engl J Med 2012;366:2455-65.

The exemplary therapeutic agents disclosed herein above are envisioned to be administered at a concentration of, *e.g.,* about 1 to 100 mg/m², about 10 to 80 mg/m², about 40 to 60 mg/m², *e.g.,* about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more mg/mm². Alternatively, the exemplary therapeutic agents disclosed herein above are envisioned to be administered at a concentration of, *e.g.,* about or at least 0.001 to 100 mg/kg or 0.1 to 1 mg/kg. In some embodiments, the exemplary therapeutic agents disclosed herein above are envisioned to be administered at a concentration of, *e.g.,* about or at least from 0.01 to 10 mg/kg.

The target peptide compositions (or target peptide composition kits) of the presently disclosed subject matter can in some embodiments also be provided with administration of cytokines such as lymphokines, monokines, growth factors and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; prostaglandin, fibroblast growth factor; prolactin; placental lactogen, OB protein; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and -II; erythropoietin (EPO); osteoinductive factors; interferons such as interferon-alpha -beta, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-13, IL-14, IL-15, IL-16, IL-17, IL-18, LIF, G-CSF, GM-CSF, M-CSF, EPO, kit-ligand or FLT-3, angiostatin, thrombospondin, endostatin, tumor necrosis factor and LT. As used herein, the term cytokine includes proteins from natural sources or from recombinant cell culture and biologically active equivalents of the native sequence cytokines.

The target peptide compositions of the presently disclosed subject matter can in some embodiments be provided with administration of cytokines around the time, (*e.g.,* about or at least 1, 2, 3, or 4 weeks or days before or after) of the initial dose of a target peptide composition.

Exemplary, non-limiting doses of a cytokine would be about or at least 1-100, 10-80, 20-70, 30-60, 40-50, or 1, 2, 3, 4, 5, 6, 7, 8, 9, or 10 Mu/m²/day over about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or 70 days. The cytokine can in some embodiments be delivered at least or about once every 1, 2, 3, 4, 5, 6, 7, 8, 9, 10, 11, 12, 13, 14, 15, 16, 17, 18, 19, 20, 21, 22, 23, or 24 hours. Cytokine treatment can in some embodiments be provided in at least or about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 cycles of at least or about 1, 2, 3, 4, 5, 6, 7, 8, 9, or 10 weeks, wherein each cycle has at least or about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 cytokine doses. Cytokine treatment can be on the same schedule as administration of the target peptide compositions or on a different (but in some embodiments overlapping) schedule.

In some embodiments, the cytokine is IL-2 and is dosed in an amount of about or at least 100,000 to 1,000,000; 200,000-900,000; 300,000-800,000; 450,000-750,000; 600,000-800,000; or 700,000-800,000; or 720,000 units (IU)/kg administered, *e.g*., as a bolus, every 1, 2, 3, 4, 5, 6, 7, 8, 9, 10, 11, 12, 13, 14, 15, 16, 17, 18, 19, or 20 hours for 1, 2, 3, 4, 5, 6, 7, 8, 9, 10, 11, 12, 13, or 14 days, in a cycle, for example.

### VI Types of Proliferative Disease

The compositions of the presently disclosed subject matter are envisioned to useful in the treatment of benign and malignant proliferative diseases. Excessive proliferation of cells and turnover of cellular matrix can contribute significantly to the pathogenesis of several diseases, including but not limited to cancer, atherosclerosis, rheumatoid arthritis, psoriasis, idiopathic pulmonary fibrosis, scleroderma and cirrhosis of the liver, ductal hyperplasia, lobular hyperplasia, papillomas, and others.

In some embodiments, the proliferative disease is cancer, which in some embodiments is selected from the group consisting of breast cancer, colorectal cancer, squamous carcinoma of the lung, sarcoma, renal cell carcinoma, pancreatic carcinomas, squamous tumors of the head and neck, leukemia, brain cancer, liver cancer, prostate cancer, ovarian cancer, and cervical cancer. In some embodiments, the compositions of the presently disclosed subject matter are used to treat colorectal cancer, acute myelogenous leukemia (AML), acute lyphocytic leukemia (ALL), chronic lymphocytic lymphoma (CLL), chronic myelogenous leukemia (CML), breast cancer, renal cancer, pancreatic cancer, and/or ovarian cancer.

The target peptide compositions of the presently disclosed subject matter are in some embodiments used to treat ovarian cancer. When metastatic, the ovarian cancer is in the lung, bone, liver, and/or brain.

In some embodiments, the cancer is a cancer of the bladder (including accelerated and metastatic bladder cancer), breast (*e.g*., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney (*e.g.,* renal cell carcinoma), liver, lung (including small cell lung cancer and non-small cell lung cancer (including adenocarcinoma, squamous cell carcinoma, bronchoalveolar carcinoma and large cell carcinoma)), genitourinary tract, *e.g.,* ovary (including fallopian, endometrial and peritoneal cancers), cervix, prostate and testes, lymphatic system, rectum, larynx, pancreas (including exocrine pancreatic carcinoma), stomach (*e.g*., gastroesophageal, upper gastric or lower gastric cancer), gastrointestinal cancer (*e.g.,* anal cancer), gall bladder, thyroid, lymphoma (*e.g*., Burkitt's, Hodgkin's, or non-Hodgkin's lymphoma), leukemia (*e.g.,* acute myeloid leukemia), Ewing's sarcoma, nasoesophageal cancer, nasopharyngeal cancer, neural and glial cell cancers (*e.g.,* glioblastoma multiforme), and head and neck. Exemplary cancers include but are not limited to melanoma, breast cancer (*e.g*., metastatic or locally advanced breast cancer), prostate cancer (*e.g.,* hormone refractory prostate cancer), renal cell carcinoma, lung cancer (*e.g.,* small cell lung cancer and non-small cell lung cancer (including adenocarcinoma, squamous cell carcinoma, bronchoalveolar carcinoma and large cell carcinoma)), pancreatic cancer, gastric cancer (*e.g.*, gastroesophageal, upper gastric or lower gastric cancer), colorectal cancer, squamous cell cancer of the head and neck, ovarian cancer (*e.g.,* advanced ovarian cancer, platinum-based agent resistant or relapsed ovarian cancer), lymphoma (*e.g*., Burkitt's, Hodgkin's, or non-Hodgkin's lymphoma), leukemia (*e.g.,* acute myeloid leukemia) and gastrointestinal cancer.

### VII. Administration of Vaccine Compositions

### VII.A. Routes of Administration

The target peptide compositions of the presently disclosed subject matter can in some embodiments be administered parenterally, systemically, and/or topically. By way of example and not limitation, composition injection can be performed by intravenous (i.v). injection, sub-cutaneous (s.c). injection, intradermal (i.d). injection, intraperitoneal (i.p). injection, and/or intramuscular (i.m). injection. One or more such routes can be employed. Parenteral administration can be, for example, by bolus injection or by gradual perfusion over time. Alternatively or concurrently, administration can be by the oral route.

In some embodiments, intradermal (i.d). injection is employed. The target peptide compositions of the presently disclosed subject matter are suitable for administration of the peptides by any acceptable route such as oral (enteral), nasal, ophthal, or transdermal. In some embodiments, the administration is subcutaneous and can be administered by an infusion pump.

### VII.B. Formulation

Pharmaceutical carriers, diluents, and excipients are generally added to the target peptide compositions or (target peptide compositions kits) that are compatible with the active ingredients and acceptable for pharmaceutical use. Examples of such carriers include, but are not limited to, water, saline solutions, dextrose, and/or glycerol. Combinations of carriers can also be used. The vaccine compositions can further incorporate additional substances to stabilize pH and/or to function as adjuvants, wetting agents, and/or emulsifying agents, which can serve to improve the effectiveness of the vaccine.

The target peptide compositions can include one or more adjuvants such but not limited to montanide ISA-51 (Seppic, Inc.); QS-21 (Aquila Pharmaceuticals, Inc.); Arlacel A; oeleic acid; tetanus helper peptides (*e.g.*, QYIKANSKFIGITEL (SEQ ID NO: 242) or AQYIKANSKFIGITEL (SEQ ID NO: 234); GM-CSF; cyclophosamide; bacillus Calmette-Guerin (BCG); corynbacterium parvum; levamisole, azimezone; isoprinisone; dinitrochlorobenezene (DNCB); keyhole limpet hemocyanins (KLH) including Freunds adjuvant (complete and incomplete); mineral gels; aluminum hydroxide (Alum); lysolecithin; pluronic polyols; polyanions; peptides; oil emulsions; nucleic acids (*e.g.,* dsRNA) dinitrophenol; diphtheria toxin (DT); toll-like receptor (TLR, *e.g.,* TLR3, TLR4, TLR7, TLR8 or TLR9) agonists (*e.g,* endotoxins such as lipopolysaccharide (LPS); monophosphoryl lipid A (MPL); polyinosinic-polycytidylic acid (poly-ICLC/HILTONOL®; Oncovir, Inc., Washington, DC, United States of America); IMO-2055, glucopyranosyl lipid A (GLA), QS-21 - a saponin extracted from the bark of the *Quillaja saponaria* tree, also known as the soap bark tree or Soapbark; resiquimod (TLR7/8 agonist), CDX-1401 - a fusion protein consisting of a fully human monoclonal antibody with specificity for the dendritic cell receptor DEC-205 linked to the NY-ESO-1 tumor antigen; Juvaris' Cationic Lipid-DNA Complex; Vaxfectin; and combinations thereof.

Polyinosinic-Polycytidylic acid (Poly IC) is a double-stranded RNA (dsRNA) that acts as a TLR3 agonist. To increase half-life, it has been stabilized with polylysine and carboxymethylcellulose as poly-ICLC. It has been used to induce interferon in cancer patients, with intravenous doses up to 300 µg/kg. Like poly-IC, poly-ICLC is a TLR3 agonist. TLR3 is expressed in the early endosome of myeloid DC; thus poly ICLC preferentially activates myeloid dendritic cells, thus favoring a Th1 cytotoxic T-cell response. Poly ICLC activates natural killer (NK) cells, induces cytolytic potential, and induces IFN-gamma from myeloid DC.

In some embodiments, the adjuvant is provided at about or at least 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 micrograms per dose or per kg in each dose. In some embodiments, the adjuvant is provided at least or about 0.1, 0.2, 0.3, 0.40, 0.50, 0.60, 0.70, 0.80, 0.90, 0.100, 1.10, 1.20, 1.30, 1.40, 1.50, 1.60, 1.70, 1.80, 1.90, 2.00, 2.10, 2.20, 2.30, 2.40, 2.50, 2.60, 2.70, 2.80, 2.90, 3.00, 3.10, 3.20, 3.30, 3.40, 3.50, 3.60, 3.70, 3.80, 3.90, 4.00, 4.10, 4.20, 4.30, 4.40, 4.50, 4.60, 4.70, 4.80, 4.90, 5.00, 5.10, 5.20, 5.30, 5.40, 5.50, 5.60, 5.70, 5.80, 5.90, 6.00, 6.10, 6.20, 6.30, 6.40, 6.50, 6.60, 6.70, 6.80, 6.90, 7.00, 7.10, 7.20, 7.30, 7.40, 7.50, 7.60, 7.70, 7.80, 7.90, 8.00, 8.10, 8.20, 8.30, 8.40, 8.50, 8.60, 8.70, 8.80, 8.90, 9.00, 9.10, 9.20, 9.30, 9.40, 9.50, 9.60, 9.70, 9.80, or 9.90 grams per dose or per kg in each dose. In some embodiments, the adjuvant is given at about or at least 10, 15, 20, 25, 50, 75, 100, 125, 150, 175, 150, 175, 200, 225, 250, 275, 300, 325, 350, 375, 400, 425, 450, 500, 525, 550, 575, 600, 625, 675, 700, 725, 750, 775, 800, 900, 1000, 1100, 1200, 1300, 1400, 1500, 1600, 1700, 1800, 1900, or 2000 endotoxin units ("EU") per dose.

The target peptide compositions of the presently disclosed subject matter can in some embodiments be provided with an administration of cyclophosamide around the time, (*e.g.,* about or at least 1, 2, 3, or 4 weeks or days before or after) the initial dose of a target peptide composition. An exemplary dose of cyclophosamide would in some embodiments be about or at least 100, 200, 300, 400, 500, 600, 700, 800, 900, or 1000 mg/m²/day over about or at least 1, 2, 3, 4, 5, 6, 7, 8, 9, or 10 days.

The compositions of the presently disclosed subject matter can in some embodiments comprise the presently disclosed target peptides in the free form and/or in the form of a pharmaceutically acceptable salt.

As used herein, "a pharmaceutically acceptable salt" refers to a derivative of the disclosed target peptides wherein the target peptide is modified by making acid or base salts of the target peptide. For example, acid salts are prepared from the free base (typically wherein the neutral form of the drug has a neutral --NH₂ group) involving reaction with a suitable acid. Suitable acids for preparing acid salts include both organic acids such as but not limited to acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids such as but not limited to hydrochloric acid, hydrobromic acid, sulfuric acid, nitric acid, phosphoric acid, and the like. Conversely, basic salts of acid moieties which can be present on a target peptide are prepared using a pharmaceutically acceptable base such as sodium hydroxide, potassium hydroxide, ammonium hydroxide, calcium hydroxide, trimmethylamine or the like. By way of example and not limitation, the compositions can in some embodiments comprise the target peptides as salts of acetic acid (acetates), ammonium, or hydrochloric acid (chlorides).

In some embodiments, a composition can include one or more sugars, sugar alcohols, amino acids such a glycine, arginine, glutaminic acid, and others as framework former. The sugars can be mono-, di- or trisaccharide. These sugars can be used alone, as well as in combination with sugar alcohols. Examples of sugars include glucose, mannose, galactose, fructose or sorbose as monosaccharides, sucrose, lactose, maltose or trehalose as disaccharides and raffinose as a trisaccharide. A sugar alcohol can be, for example, mannitose. In some embodiments, the composition comprises sucrose, lactose, maltose, trehalose, mannit and/or sorbit. In some embodiments, the composition comprises mannitol.

Furthermore, in some embodiments the presently disclosed compositions can include physiological well-tolerated excipients (*see e.g.,* the Handbook of Pharmaceutical Excipients, 5th ed. (2006) Rowe et al. (eds)., Pharmaceutical Press, London, United Kingdom), such as antioxidants like ascorbic acid or glutathione, preserving agents such as phenol, m-cresole, methyl- or propylparabene, chlorobutanol, thiomersal or benzalkoniumchloride, stabilizer, framework former such as sucrose, lactose, maltose, trehalose, mannitose, mannitol and/or sorbitol, mannitol and/or lactose and solubilizer such as polyethyleneglycols (PEG), *i.e.* PEG 3000, 3350, 4000, or 6000, or cyclodextrines, *i.e.* hydroxypropyle-β-cyclodextrine, sulfobutylethyl-β-cyclodextrine or γ-cyclodextrine, or dextranes or poloxaomers, *i.e.* poloxaomer 407, poloxamer 188, or TWEEN™20, TWEEN™ 80. In some embodiments, one or more well tolerated excipients can be included, selected from the group consisting of antioxidants, framework formers, and stabilizers.

In some embodiments, the pH for intravenous and intramuscular administration is selected from pH 2 to pH 12, while the pH for subcutaneous administration is selected from pH 2.7 to pH 9.0 as the rate of *in vivo* dilution is reduced resulting in more potential for irradiation at the injection site. (Strickley (2004) Pharm Res 21:201-230).

### VII.C. Dosage

It is understood that a suitable dosage of a target peptide composition vaccine immunogen will depend upon the age, sex, health, and weight of the recipient, the kind of concurrent treatment, if any, the frequency of treatment, and the nature of the effect desired. However, a desired dosage can be tailored to the individual subject, as determined by the researcher or clinician. The total dose employed for any given treatment can typically be determined with respect to a standard reference dose based on the experience of the researcher or clinician, such dose being administered either in a single treatment or in a series of doses, the success of which can depend on the production of a desired immunological result (*i.e.,* successful production of a T helper cell and/or CTL-mediated response to the target peptide immunogen composition, which response gives rise to the prevention and/or treatment desired). Thus, in some embodiments the overall administration schedule can be considered in determining the success of a course of treatment and not whether a single dose, given in isolation, would or would not produce the desired immunologically therapeutic result or effect. As such, a therapeutically effective amount (*i.e.,* that producing the desired T helper cell and/or CTL-mediated response) can in some embodiments depend on the antigenic composition of the vaccine used, the nature of the disease condition, the severity of the disease condition, the extent of any need to prevent such a condition where it has not already been detected, the manner of administration dictated by the situation requiring such administration, the weight and state of health of the individual receiving such administration, and/or the sound judgment of the clinician or researcher. Needless to say, the efficacy of administering additional doses and of increasing or decreasing the interval can be re-evaluated on a continuing basis, in view of the recipient's immunocompetence (for example, the level of T helper cell and/or CTL activity with respect to tumor-associated or tumor-specific antigens).

The concentration of the T helper or CTL stimulatory target peptides of the invention in pharmaceutical formulations are subject to wide variation, including anywhere from less than 0.01% by weight to as much as 50% or more. Factors such as volume and viscosity of the resulting composition can also be considered. The solvents, or diluents, used for such compositions can include one or more of water, phosphate buffered saline (PBS), saline itself, and/or other possible carriers and/or excipients. The immunogens of the presently disclosed subject matter can in some embodiments also be contained in artificially created structures such as liposomes, which structures can in some embodiments contain additional molecules, such as proteins or polysaccharides, inserted in the outer membranes of the structures and having the effect of targeting the liposomes to particular areas of the body, or to particular cells within a given organ or tissue. Such targeting molecules can in some embodiments be some type of immunoglobulin. Antibodies can work particularly well for targeting the liposomes to tumor cells.

Single i.d., i.m., s.c., i.p., and i.v. doses of *e.g.,* about 1 to 50 µg, 1 to 100 µg, 1 to 500 µg, 1 to 1000 µg, or about 1 to 50 mg, 1 to 100 mg, 1 to 500 mg, or 1 to 1000 mg of a target peptide composition of the presently disclosed subject matter can in some embodiments be given and in some embodiments can depend from the respective compositions of target peptides with respect to total amount for all target peptides in the composition or alternatively for each individual target peptide in the composition. A single dose of a target peptide vaccine composition of the presently disclosed subject matter can in some embodiments have a target peptide amount (*e.g.*, total amount for all target peptides in the composition or alternatively for each individual target peptide in the composition) of about or at least 1, 5, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, or 950 µg. Alternatively, a single dose of a target peptide composition of the presently disclosed subject matter can in some embodiments have a total target peptide amount (*e.g.,* total amount for all target peptides in the composition or alternatively for each individual target peptide in the composition) of about or at least 1, 5, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, or 950 mg. In some embodiments, the target peptides of a composition of the presently disclosed subject matter are present in equal amounts of about 100 micrograms per dose in combination with an adjuvant peptide present in an amount of about 200 micrograms per dose.

In a single dose of the target peptide composition of the presently disclosed subject matter, the amount of each target peptide in the composition is in some embodiments equal or is in some embodiments substantially equal. Alternatively, the ratio of the target peptides present in the least amount relative to the target peptide present in the greatest amount is in some embodiments about or at least 1:1.25, 1:1.5, 1:1.75, 1:2.0, 1:2.25, 1:2.5, 1:2.75, 1:3, 1:4, 1:5, 1:6, 1:7, 1:8, 1:9, 1:10, 1:20, 1:30; 1:40, 1:50, 1:100, 1:200, 1:500, 1:1000, 1:5000; 1:10,000; or 1:100,000. Alternatively, the ratio of the target peptides present in the least amount relative to the target peptide present in the greatest amount is in some embodiments about or at least 1 or 2 to 25; 1 or 2 to 20; 1 or 2 to 15; 1 or 2 to 10; 1 to 3; 1 to 4; 1 to 5; 1 to 6; 1 to 7; 1 to 10; 2 to 3; 2 to 4; 2 to 5; 2 to 6; 2 to 7; 2 to 10; 3 to 4; 3 to 5; 3 to 6; 3 to 7; 3 to 10; 5 to 10; 10 to 15; 15 to 20; 20 to 25; 1 to 40; 1 to 30; 1 to 20; 1 to 15; 10 to 40; 10 to 30; 10 to 20; 10 to 15; 20 to 40; 20 to 30; or 20 to 25; 1 to 100; 25 to 100; 50 to 100; 75 to 100; 25 to 75, 25 to 50, or 50 to 75; 25 to 40; 25 to 50; 30 to 50; 30 to 40; or 30 to 75.

Single dosages can in some embodiments be given to a patient about or at least 1, 2, 3, 4, or 5 times per day. Single dosages can in some embodiments be given to a patient about or at least 1, 2, 3, 4, 5, 6, 7, 8, 9, 10, 11, 12, 13, 14, 15, 16, 18, 19, 20, 21, 22, 23, 24, 36, 48, 60, or 72 hours subsequent to a previous dose.

Single dosages can in some embodiments be given to a patient about or at least 1, 2, 3, 4, 5, 6, or 7 times per week or every other, third, fourth, or fifth day. Single doses can in some embodiments also be given every week, every other week, or only during 1, 2, or 3 weeks per month. A course of treatment can in some embodiments last about or at least 1, 2, 3, 4, 5, 6, 7, 8, 9, 10, 11, or 12 months.

In some embodiments, single dosages of the compositions of the presently disclosed subject matter are provided to a patient in at least two phases, *e.g.*, during an initial phase and then a subsequent phase. An initial phase can in some embodiments be about or at least 1, 2, 3, 4, 5, or 6 weeks in length. The subsequent phase can in some embodiments last at least or about 1, 2, 3, 4, 5, 6, 7, or 8 times as long as the initial phase. The initial phase can in some embodiments be separated from the subsequent phase by about or at least 1, 2, 3, 4, 5, 6, 7, 8, 9, 10, 11, or 12 weeks or months.

The target peptide composition dosage during the subsequent phase can in some embodiments be at least 1, 2, 3, 4, 5, 6, 7, 8, 9, 10, 15, 20, 30, 40, 50, 60, 70, 80, 90, 100, 200, 300, 400, 500, 600, 700, 800, 900, or 1000 times greater than during the initial phase. The target peptide composition dosage during the subsequent phase can in some embodiments be at least 1, 2, 3, 4, 5, 6, 7, 8, 9, 10, 15, 20, 30, 40, 50, 60, 70, 80, 90, 100, 200, 300, 400, 500, 600, 700, 800, 900, or 1000 times lower than during the initial phase.

In some embodiments, the initial phase is about three weeks and the second phase is about 9 weeks. In some embodiments, the target peptide compositions would be administered to the patient on or about days 1, 8, 15, 36, 57, and 78.

### VII.D. Kits and Storage

In some embodiments, the presently disclosed subject matter provides a kit. In some embodiments the kit comprises (a) a container that contains at least one target peptide composition as described above in solution or in lyophilized form; (b) optionally, a second container containing a diluent or reconstituting solution for the lyophilized formulation; and (c) also optionally, instructions for (i) use of the solution; and/or (ii) reconstitution and/or use of the lyophilized formulation. The kit can in some embodiments further comprise one or more of (iii) a buffer, (iv) a diluent, (v) a filter, (vi) a needle, and/or (v) a syringe. In some embodiments, the container is selected from the group consisting of a bottle, a vial, a syringe, a test tube, and a multi-use container. In some embodiments, the target peptide composition is lyophilized.

The kits can in some embodiments contain exactly, about, or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, or more target peptide-containing compositions. Each composition in the kit can in some embodiments be administered at the same time or at different times to a subject.

In some embodiments, the kits can comprise a lyophilized formulation of the presently disclosed compositions and/or vaccines in a suitable container and instructions for its reconstitution and/or use. Suitable containers include, for example, bottles, vials (*e.g.* dual chamber vials), syringes (such as dual chamber syringes), and test tubes. The container can in some embodiments be formed from a variety of materials such as glass or plastic. In some embodiments, the kit and/or container include instructions on or associated with the container that indicate directions for reconstitution and/or use. For example, the label can in some embodiments indicate that the lyophilized formulation is to be reconstituted to target peptide concentrations as described above. The label can in some embodiments further indicate that the formulation is useful or intended for subcutaneous administration. Lyophilized and liquid formulations are in some embodiments stored at -20° C to -80° C.

The container holding the target peptide composition(s) can in some embodiments be a multi-use vial, which allows for repeat administrations (*e.g.*, from 2-6 administrations) of the reconstituted formulation. The kit can in some embodiments further comprise a second container comprising a suitable diluent such as, but not limited to a sodium bicarbonate solution.

In some embodiments, upon mixing of the diluent and the lyophilized formulation, the final peptide concentration in the reconstituted formulation is at least or about 0.15, 0.20, 0.25, 0.5, 0.75, 1.0, 1.25, 1.5, 1.75, 2.0, 2.25, 2.5, 2.75, 3.0, 3.25, 3.50, 3.75, 4.0, 4.25, 4.5, 4.75, 5.0, 6.0, 7.0, 8.0, 9.0, or 10 mg/mL/target peptide. In some embodiments, upon mixing of the diluent and the lyophilized formulation, the final peptide concentration in the reconstituted formulation is at least or about 0.15, 0.20, 0.25, 0.5, 0.75, 1.0, 1.25, 1.5, 1.75, 2.0, 2.25, 2.5, 2.75, 3.0, 3.25, 3.50, 3.75, 4.0, 4.25, 4.5, 4.75, 5.0, 6.0, 7.0, 8.0, 9.0 or 10 µg/mL/target peptide.

The kit can in some embodiments further comprise other materials desirable from a commercial and user standpoint, including but not limited to other buffers, diluents, filters, needles, syringes, and/or package inserts with instructions for use.

The kits can in some embodiments have a single container that comprises the formulation of the target peptide compositions with or without other components (*e.g.,* other compounds or compositions of these other compounds) or can in some embodiments have a distinct container for each component.

Additionally, the kits can in some embodiments comprise a formulation of the presently disclosed target peptide compositions and/or vaccines packaged for use in combination with the co-administration of a second compound such as but not limited to adjuvants (*e.g*. imiquimod), a chemotherapeutic agent, a natural product, a hormone or antagonist, an anti-angiogenesis agent or inhibitor, an apoptosis-inducing agent, or a chelator or a composition thereof. The components of the kit can in some embodiments be pre-complexed or each component can in some embodiments be in a separate distinct container prior to administration to a patient. The components of the kit can in some embodiments be provided in one or more liquid solutions. In some embodiments, the liquid solution is an aqueous solution. In some embodiments, the liquid solution is a sterile aqueous solution. The components of the kit can in some embodiments also be provided as solids, which in some embodiments are converted into liquids by addition of suitable solvents, which can in some embodiments be provided in another distinct container.

The container of a therapeutic kit can in some embodiments be a vial, a test tube, a flask, a bottle, a syringe, or any other article suitable to enclose a solid or liquid. In some embodiments, when there is more than one component, the kit can contain a second vial and/or other container, which allows for separate dosing. The kit can in some embodiments also contain another container for a pharmaceutically acceptable liquid. In some embodiments, a therapeutic kit contains an apparatus (*e.g.,* one or more needles, syringes, eye droppers, pipette, etc.) that facilitates administration of the agents of the disclosure that are components of the present kit.

### VIII.E. Markers for Efficacy

When administered to a patient, the vaccine compositions of the presently disclosed subject matter are envisioned to have certain physiological effects, including but not limited to the induction of a T cell mediated immune response.

### VIII.E.1 Immunohistochemistry, Immunofluorescence, Western Blots, and Flow Cytometry

Validation and testing of antibodies for characterization of cellular and molecular features of lymphoid neogenesis has been performed. Commercially available antibodies for use in immunohistochemistry (IHC), immunofluorescence (IF), flow cytometry (FC), and western blot (WB) can in some embodiments be employed. In some embodiments, such techniques can be employed to analyze patient samples, *e.g*., formalin-fixed, paraffin-embedded tissue samples, for CD1a, S100, CD83, DC-LAMP, CD3, CD4, CD8, CD20, CD45, CD79a, PNAd, TNFalpha, LIGHT, CCL19, CCL21, CXCL12, TLR4, TLR7, FoxP3, PD-1 and Ki67 expression. In some embodiments, flow cytometry is used to determine CD3, CD4, CD8, CD13, CD14, CD16, CD19, CD45RA, CD45RO, CD56, CD62L, CD27, CD28, CCR7, FoxP3 (intracellular), and MHC-peptide tetramers for I MHC associated (phospho)-peptides. In some embodiments, positive control tissue selected from among normal human peripheral blood lymphocytes (PBL), PBL activated with CD3/CD28 beads (activated PBL), human lymph node tissue from non-ovarian cancer patients (LN), and inflamed human tissue from a surgical specimen of Crohn's disease (Crohn's) can be employed.

### VII.E.2. ELISpot Assay

In some embodiments, vaccination site infiltrating lymphocytes and lymphocytes from the sentinel immunized nod (SIN) and vaccine site can be evaluated by ELISpot. ELISpot permits the direct counting of T-cells reacting to antigen by production of INFγ. Peripheral blood lymphocytes can be evaluated by ELISpot assay for the number of peptide-reactive T-cells. Vaccine site infiltrating lymphocytes and SIN lymphocytes can be compared to those in peripheral blood. It is envisioned that positive results of the ELISpot assay correlate with increased patient progression free survival. Progression free survival is in some embodiments defined as the time from start of treatment until death from any cause or date of last follow up.

### VII.E.3. Tetramer Assay

Peripheral blood lymphocytes and lymphocytes from the SIN and vaccine site can be evaluated by flow cytometry after incubation with MHC-peptide tetramers for the number of peptide-reactive T-cells.

### VII.E.4. Proliferation Assay/Cytokine Analysis

Peripheral blood mononuclear cells (PBMC), vaccine-site inflammatory cells, and lymphocytes from the SIN from patients can in some embodiments be evaluated for CD4 T cell reactivity to, *e.g.,* tetanus helper peptide mixture, using a ³H-thymidine uptake assay. Additionally, Th1 (IL-2, IFN-gamma, TNFa), Th2 (IL-4, IL-5, IL-10), Th17 (IL-17, and IL23), and T-reg (TGF-beta) cytokines in media from 48 hours in that proliferation assay can be employed to determine if the microenvironment supports generation of Th1, Th2, Th17, and/or T-reg responses. In some embodiments, two peptides are used as negative controls: a tetanus peptide and the PADRE peptide (AK(X)VAAWTLKAA; SEQ ID NO: 243).

### VII.E.5. Evaluation of Tumors

In some embodiments tumor tissue collected prior to treatment or at the time of progression can be evaluated by routine histology and immunohistochemistry. Alternatively or in addition, *in vitro* evaluations of tumor tissue and tumor infiltrating lymphocytes can be completed.

### VII.E.6. Studies of Homing Receptor Expression

Patient samples can in some embodiments be studied for T cell homing receptors induced by vaccination the compositions of the invention. These include, but are not limited to, integrins (including alphaE-beta7, alpha1-beta1, alpha4-beta1), chemokine receptors (including CXCR3), and selectin ligands (including CLA, PSL) on lymphocytes, and their ligands in the vaccine sites and SIN. These can be assayed by immunohistochemistry, flow cytometry or other techniques.

### VII.E.7. Studies of Gene and Protein Expression

Differences in gene expression and/or for differences in panels of proteins can in some embodiments be assayed by high-throughput screening assays (e.g. nucleic acid chips, protein arrays, etc.) in the vaccine sites and sentinel immunized nodes.

### VIII. Antibodies Including Antibody-Like Molecules

Antibodies and antibody-like molecules (*e.g.* T cell receptors) specific for target peptides or target peptide/MHC complexes are, for example, useful, *inter alia,* for analyzing tissue to determine the pathological nature of tumor margins and/or can be employed in some embodiments as therapeutics. Alternatively, such molecules can in some embodiments be employed as therapeutics targeting cells, *e.g.,* tumor cells, which display target peptides on their surface. In some embodiments, the antibodies and antibody-like molecules bind the target peptides or target peptide-MHC complex specifically and do not substantially cross react with non-phosphorylated native peptides.

As used herein, "antibody" and "antibody peptide(s)" refer to intact antibodies, antibody-like molecules, and binding fragments thereof that compete with intact antibodies for specific binding. Binding fragments are in some embodiments produced by recombinant DNA techniques or in some embodiments by enzymatic or chemical cleavage of intact antibodies. Binding fragments include Fab, Fab', F(ab')₂, Fv, and single-chain antibodies. An antibody other than a "bispecific" or "bifunctional" antibody is understood to have each of its binding sites identical. An antibody in some embodiments substantially inhibits adhesion of a receptor to a counterreceptor when an excess of antibody reduces the quantity of receptor bound to counterreceptor by at least about 20%, 40%, 60%, 80%, 85%, 90%, 91%, 92%, 93%, 94%, 95%, 96%, 97%, 98%, 99%, or greater than 99% as measured, for example, in an *in vitro* competitive binding assay.

The term "MHC" as used herein refers to the Major Histocompability Complex, which is defined as a set of gene loci specifying major histocompatibility antigens. The term "HLA" as used herein refers to Human Leukocyte Antigens, which are defined as the histocompatibility antigens found in humans. As used herein, "HLA" is the human form of "MHC".

The terms "MHC light chain" and "MHC heavy chain" as used herein refer to portions of MHC molecules. Structurally, class I molecules are heterodimers comprised of two non-covalently bound polypeptide chains, a larger "heavy" chain (α) and a smaller "light" chain (β-2-microglobulin or β2m). The polymorphic, polygenic heavy chain (45 kDa), encoded within the MHC on chromosome six, is subdivided into three extracellular domains (designated 1, 2, and 3), one intracellular domain, and one transmembrane domain. The two outermost extracellular domains, 1 and 2, together form the groove that binds antigenic peptide. Thus, interaction with the TCR occurs at this region of the protein. The 3 domain of the molecule contains the recognition site for the CD8 protein on the CTL; this interaction serves to stabilize the contact between the T cell and the APC. The invariant light chain (12 kDa), encoded outside the MHC on chromosome 15, consists of a single, extracellular polypeptide. The terms "MHC light chain", "β2-microglobulin", and "β2m" are used interchangeably herein.

The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. An antibody or antibody like molecule is said to "specifically" bind an antigen when the dissociation constant is in some embodiments less than 1 µM, in some embodiments less than 100 nM, and in some embodiments less than 10 nM.

The term "antibody" is used in the broadest sense, and specifically covers monoclonal antibodies (including full length monoclonal antibodies), polyclonal antibodies, multispecific antibodies (*e.g*., bispecific antibodies), and antibody fragments (*e.g.*, Fab, F(ab')₂ and Fv), as well as "antibody-like molecules" so long as they exhibit the desired biological activity. Antibodies (Abs) and immunoglobulins (Igs) are glycoproteins having the same structural characteristics. The term is also meant to encompass "antibody like molecules" and other members of the immunoglobulin superfamily, *e.g.,* T-cell receptors, MHC molecules, containing *e.g.,* an antigen-binding regions and/or variable regions, *e.g*., complementary determining regions (CDRs) which specifically bind the target peptides disclosed herein.

In some embodiments, antibodies and antibody-like molecules bind to the target peptides of the presently disclosed subject matter but do not substantially and/or specifically cross react with the same peptide in a modified form. *See e.g.,* U.S. Patent Application Publication No. 2009/0226474, which is incorporated by reference.

The presently disclosed subject matter also includes antibodies that recognize target peptides associated with a tumorigenic or disease state, wherein the peptides are displayed in the context of HLA molecules. These antibodies typically mimic the specificity of a T cell receptor (TCR) but can in some embodiments have higher binding affinity such that the molecules can be employed as therapeutic, diagnostic, and/or research reagents. Methods of producing a T-cell receptor mimic of the presently disclosed subject matter include identifying a target peptide of interest, wherein the target peptide of interest comprises an amino acid sequence as set forth in any of SEQ ID NOs: 1-193. Then, an immunogen comprising at least one target peptide/MHC complex is formed. An effective amount of the immunogen is then administered to a host for eliciting an immune response, and serum collected from the host is assayed to determine if desired antibodies that recognize a three-dimensional presentation of the target peptide in the binding groove of the MHC molecule are being produced. The desired antibodies can differentiate the target peptide/MHC complex from the MHC molecule alone, the target peptide alone, and a complex of MHC and irrelevant target peptide. Finally, in some embodiments the desired antibodies are isolated.

The term "antibody" also encompasses soluble T cell receptors (TCR) cytoplasmic domains which are stable at low concentrations and which can recognize MHC-peptide complexes. *See e.g.,* U.S. Patent Application Publication No. 2002/0119149, which is incorporated by reference. Such soluble TCRs might for example be conjugated to immunostimulatory peptides and/or proteins or moieties, such as CD3 agonists (anti-CD3 antibody), for example. The CD3 antigen is present on mature human T cells, thymocytes, and a subset of natural killer cells. It is associated with the TCR and is responsible for the signal transduction of the TCR.

Antibodies specific for the human CD3 antigen are well-known. One such antibody is the murine monoclonal antibody OKT3 which was the first monoclonal antibody approved by the FDA. OKT3 is reported to be a potent T cell mitogen (Van Wauve (1980) J Immunol 124:2708-2718; *see also* U.S. Patent No. 4,361,539) and a potent T cell killer (Wong (1990) Transplantation 50:683-389). Other antibodies specific for the CD3 antigen have also been reported. (*see* PCT International Patent Application Publication No. WO 2004/0106380; U.S. Patent Application Publication No. 2004/0202657; U.S. Patent No. 6,750,325; U.S. Patent No. 6,706,265; GB 2249310A; Clark et al. (1989) Eur J Immunol 19:381-388; U.S. Patent No. No. 5,968,509; and U.S. Patent Application Publication No. 2009/0117102). ImmTACs (Immunocore Limited, Milton Park, Abington, Oxon, United Kingdom) are innovative bifunctional proteins that combine high-affinity monoclonal T cell receptor (mTCR) targeting technology with a clinically-validated, highly potent therapeutic mechanism of action (Anti-CD3 scFv).

Native antibodies and immunoglobulins are usually heterotetrameric glycoproteins of about 150,000 daltons, composed of two identical light (L) chains and two identical heavy (H) chains. Each light chain is linked to a heavy chain by one covalent disulfide bond. The number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end. The constant domain of the light chain is aligned with the first constant domain of the heavy chain, and the light chain variable domain is aligned with the variable domain of the heavy chain. Particular amino acid residues are believed to form an interface between the light and heavy chain variable domains (Clothia et al. (1985) J Mol Biol 186:651-66; Novotny & Haber (1985) Proc Natl Acad Sci U S A 82:4592-4596).

An "isolated" antibody is one which has been separated, identified, and/or recovered from a component of the environment in which it was produced. Contaminant components of its production environment are materials which would interfere with diagnostic or therapeutic uses for the antibody, and can include enzymes, hormones, and other proteinaceous or nonproteinaceous solutes. In some embodiments, the antibody is purified as measurable by at least one of the following three different methods: 1) to in some embodiments greater than 50% by weight of antibody as determined by the Lowry method, such as but not limited to in some embodiments greater than 75% by weight, in some embodiments greater than 85% by weight, in some embodiments greater than 95% by weight, in some embodiments greater than 99% by weight; 2) to a degree sufficient to obtain at least 10 residues of N-terminal or internal amino acid sequence by use of a spinning cup sequentator, such as at least 15 residues of sequence; or 3) to homogeneity by SDS-PAGE under reducing or non-reducing conditions using Coomasie blue or, in some embodiments, silver stain. Isolated antibodies include the antibody *in situ* within recombinant cells since at least one component of the antibody's natural environment is not present. In some embodiments, however, isolated antibodies are prepared by a method that includes at least one purification step.

The terms "antibody mutant", "antibody variant", and "antibody derivative" refer to an amino acid sequence variant of an antibody wherein one or more of the amino acid residues of a reference antibody has been modified (*e.g*., substituted, deleted, chemically modified, etc.). Such mutants necessarily have less than 100% sequence identity or similarity with the amino acid sequence of either the heavy or light chain variable domain of the reference antibody. The resultant sequence identity or similarity between the modified antibody and the reference antibody is thus in some embodiments at least 80%, in some embodiments at least 85%, in some embodiments at least 90%, in some embodiments at least 95%, in some embodiments at least 97%, and in some embodiments at least 99%.

The term "variable" in the context of variable domain of antibodies, refers to the fact that certain portions of the variable domains differ extensively in sequence among antibodies and are used in the binding and specificity of each particular antibody for its particular antigen(s). However, the variability is not evenly distributed through the variable domains of antibodies. It is concentrated in three segments called complementarity determining regions (CDRs) also known as hypervariable regions both in the light chain and the heavy chain variable domains. There are at least two techniques for determining CDRs: (1) an approach based on cross-species sequence variability (*i.e.,* Kabat et al. (1987) Sequences of Proteins of Immunological Interest, National Institute of Health, Bethesda, Maryland, United States of America); and (2) an approach based on crystallographic studies of antigen-antibody complexes (Chothia et al. (1989) Nature 342:877-883). The more highly conserved portions of variable domains are called the framework (FR) regions. The variable domains of native heavy and light chains each comprise four FR regions, largely adopting a β-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen binding site of antibodies (*see* Kabat *et al.,* 1987, *op. cit.*)*.* The constant domains are not involved directly in binding an antibody to an antigen, but exhibit various effector function, such as participation of the antibody in antibody-dependent cellular toxicity.

The term "antibody fragment" refers to a portion of a full-length antibody, generally the antigen binding or variable region. Examples of antibody fragments include Fab, Fab', F(ab')₂ and Fv fragments. Papain digestion of antibodies produces two identical antigen binding fragments, called the Fab fragment, each with a single antigen binding site, and a residual "Fc" fragment, so-called for its ability to crystallize readily. Pepsin treatment yields an F(ab')₂ fragment that has two antigen binding fragments which are capable of cross-linking antigen, and a residual other fragment (which is termed pFc'). As used herein, "functional fragment" with respect to antibodies, refers to Fv, F(ab) and F(ab')₂ fragments.

An "Fv" fragment is the minimum antibody fragment which contains a complete antigen recognition and binding site. This region consists of a dimer of one heavy and one light chain variable domain in a tight, non-covalent association (V_{H}-V_{L} dimer). It is in this configuration that the three CDRs of each variable domain interact to define an antigen binding site on the surface of the V_{H}-V_{L} dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

The Fab fragment, also designated as F(ab), also contains the constant domain of the light chain and the first constant domain (CH1) of the heavy chain. Fab' fragments differ from Fab fragments by the addition of a few residues at the carboxyl terminus of the heavy chain CH1 domain including one or more cysteines from the antibody hinge region. Fab'-SH is the designation herein for Fab' in which the cysteine residue(s) of the constant domains have a free thiol group. F(ab') fragments are produced by cleavage of the disulfide bond at the hinge cysteines of the F(ab')₂ pepsin digestion product. Additional chemical couplings of antibody fragments are known to those of ordinary skill in the art.

The light chains of antibodies (immunoglobulin) from any vertebrate species can be assigned to one of two clearly distinct types, called kappa and lambda, based on the amino sequences of their constant domain.

Depending on the amino acid sequences of the constant domain of their heavy chains, immunoglobulins can be assigned to different classes. There are at least five (5) major classes of immunoglobulins: IgA, IgD, IgE, IgG and IgM, and several of these can be further divided into subclasses (isotypes), *e.g.,* IgG₁, IgG₂, IgG₃, and IgG₄; IgA₁ and IgA₂. The heavy chains constant domains that correspond to the different classes of immunoglobulins are called alpha (α), delta (Δ), epsilon (ε), gamma (γ), and mu (µ), respectively. The subunit structures and three-dimensional configurations of different classes of immunoglobulins are well-known.

The term "monoclonal antibody" as used herein refers to an antibody obtained from a population of substantially homogeneous antibodies, *i.e.,* the individual antibodies comprising the population are identical except for possible naturally occurring mutations that can be present in minor amounts. Monoclonal antibodies are highly specific, being directed against a single antigenic site. Furthermore, in contrast to conventional (polyclonal) antibody preparations, which typically include different antibodies directed against different determinants (epitopes), each monoclonal antibody is directed against a single determinant on the antigen. In addition to their specificity, monoclonal antibodies can be advantageous in that they can be synthesized in hybridoma culture, uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the presently disclosed subject matter can in some embodiments be made by the hybridoma method first described by Kohler & Milstein (1975) Nature 256:495, or can in some embodiments be made by recombinant methods, *e.g.,* as described in U.S. Patent No. 4,816,567. The monoclonal antibodies for use with the presently disclosed subject matter can in some embodiments also be isolated from phage antibody libraries using the techniques described in Clackson et al. (1991) Nature 352:624-628 or in Marks et al. (1991) J Mol Biol 222:581-597.

Utilization of the monoclonal antibodies of the presently disclosed subject matter can in some embodiments require administration of such or similar monoclonal antibody to a subject, such as a human. However, when the monoclonal antibodies are produced in a non-human animal, such as a rodent, administration of such antibodies to a human patient will normally elicit an immune response, wherein the immune response is directed towards the antibodies themselves. Such reactions limit the duration and effectiveness of such a therapy. In order to overcome such problem, the monoclonal antibodies of the presently disclosed subject matter can be "humanized": that is, the antibodies can be engineered such that antigenic portions thereof are removed and like portions of a human antibody are substituted therefor, while the antibodies' affinity for specific peptide/MHC complexes is retained. This engineering can in some embodiments only involve a few amino acids, or can in some embodiments include entire framework regions of the antibody, leaving only the complementarity determining regions of the antibody intact. Several methods for humanizing antibodies are known in the art and are disclosed, for example, in U.S. Patent Nos. 6,180,370 to Queen et al.*;* 6,054,927 to Brickell; 5,869,619 to Studnicka; 5,861,155 to Lin; 5,712,120 to Rodriquez et al.*;* and 4,816,567 to Cabilly et al.*,* the entire content of each of which is hereby expressly incorporated herein by reference in its entirety.

Humanized forms of antibodies are chimeric immunoglobulins, immunoglobulin chains, or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. In some embodiments, humanization can be performed following the method of Winter and co-workers (*see e.g.,* Jones et al. (1986) Nature 321:522-525; Riechmann et al. (1988) Nature 332:323-327; Verhoeyen et al. (1988) Science 239:1534-1536) by substituting rodent CDRs or CDR sequences for the corresponding sequences of a human antibody. *See also* U.S. Patent No. 5,225,539. In some embodiments, Fᵥ framework residues of a human immunoglobulin are replaced by corresponding non-human residues.

Humanized antibodies can also comprise residues which are found neither in the recipient antibody nor in the imported CDR or framework sequences. In general, a humanized antibody comprises substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally can in some embodiments also comprise at least a portion of an immunoglobulin constant region (Fc), typically that of a human immunoglobulin. *See e.g.,* Jones et al. (1986) Nature 321:522-525; Riechmann et al. (1988) Nature 332:323-327; Presta (1992) Proc Natl Acad Sci U S A 89:4285-4289.

Many articles relating to the generation or use of humanized antibodies teach useful examples of protocols that can be utilized with the presently disclosed subject matter, such as but not limited to Sandborn et al. (2001) Gastroenterology 120:1330-1338; Mihara et al. (2001) Clin Immunol 98:319; Yenari et al. (2001) Neurol Res 23:72; Morales et al. (2000) Nucl Med Biol 27:199; Richards et al. (1999) Cancer Res 59:2096; Yenari et al. (1998) Exp Neurol 153:223; and Shinkura et al. (1998) Anticancer Res 18:1217, all of which are expressly incorporated in their entireties by reference. For example, a treatment protocol that can be utilized in such a method includes a single dose, generally administered intravenously, of 10-20 mg of humanized mAb per kg (Sandborn, et al. (2001) Gastroenterology 120:1330-1338). In some embodiments, alternative dosing patterns can be appropriate, such as but not limited to the use of three infusions, administered once every two weeks, of 800 to 1600 mg or even higher amounts of humanized mAb (Richards *et al.,* 1999, *op. cit*.). However, it is to be understood that the presently disclosed subject matter is not limited to the treatment protocols described above, and other treatment protocols that are known to a person of ordinary skill in the art can be utilized in the methods of the presently disclosed subject matter.

The presently disclosed and embodimented subject matter further includes in some embodiments fully human monoclonal antibodies against specific target peptide/MHC complexes. Fully human antibodies essentially relate to antibody molecules in which the entire sequence of both the light chain and the heavy chain, including the CDRs, arise from human genes. Such antibodies are referred to herein as "human antibodies" or "fully human antibodies". Human monoclonal antibodies can be prepared by the trioma technique; the human B-cell hybridoma technique *(see* Kozbor et al. (1983) Hybridoma, 2:7), and the EBV hybridoma technique to produce human monoclonal antibodies (*see* Cole et al. (1985) Proc Natl Acad Sci U S A 82:859). Human monoclonal antibodies can in some embodiments be utilized in the practice of the presently disclosed subject matter and can in some embodiments be produced by using human hybridomas (*see* Cote et al. (1983) Proc Natl Acad Sci U S A 80:2026) or by transforming human B-cells with Epstein Barr Virus *in vitro* (*see* Cole *et al.,* 1985, *op. cit*.).

In addition, human antibodies can also be produced using additional techniques, including but not limited to phage display libraries (Hoogenboom et al. (1991) Nucleic Acids Res 19:4133; Marks et al. (1991) J Mol Biol 222:581). Similarly, human antibodies can be made by introducing human immunoglobulin loci into transgenic animals, *e.g*., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; and 5,661,016; and in Marks et al. (1992) J Biol Chem 267:16007; Lonberg et al. (1994) Nature 368:856; Fishwild et al. (1996) Nature Biotechnol 14:845; Neuberger (1996) Nature Biotechnol 14:826; and Lonberg & Huszar (1995) Intl Rev Immunol 13:65.

Human antibodies can in some embodiments additionally be produced using transgenic nonhuman animals which are modified so as to produce fully human antibodies rather than the animal's endogenous antibodies in response to challenge by an antigen. *See* PCT International Patent Application Publication No. WO 1994/02602). Typically, the endogenous genes encoding the heavy and light immunoglobulin chains in the non-human host are incapacitated, and active loci encoding human heavy and light chain immunoglobulins are inserted into the host's genome. The human genes are incorporated, for example, using yeast artificial chromosomes containing the requisite human DNA segments. An animal that provides all the desired modifications is then obtained as progeny by crossbreeding intermediate transgenic animals containing fewer than the full complement of the modifications.

A non-limiting example of such a nonhuman animal is a mouse, and is termed the XENOMOUSE™ as disclosed in PCT International Patent Application Publication Nos. WO 1996/33735 and WO 1996/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a non-human host, exemplified as a mouse, lacking expression of an endogenous immunoglobulin heavy chain is disclosed in U.S. Patent No. 5,939,598 to Kucherlapati et al. (incorporated herein by reference). It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

An exemplary method for producing an antibody of interest, such as a human antibody, is disclosed in U.S. Patent No. 5,916,771 to Hori et al. (incorporated herein by reference). It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

The antigen target peptides are known to be expressed on a variety of cancer cell types. Thus, antibodies and antibody-like molecules can be used where appropriate, in treating, diagnosing, vaccinating, preventing, retarding, and/or attenuating melanoma, ovarian cancer, breast cancer, colorectal cancer, squamous carcinoma of the lung, sarcoma, renal cell carcinoma, pancreatic carcinomas, squamous tumors of the head and neck, leukemia, brain cancer, liver cancer, prostate cancer, ovarian cancer, and cervical cancer.

Antibodies generated with specificity for the antigen target peptides can be used to detect the corresponding target peptides in biological samples. The biological sample could come from an individual who is suspected of having cancer and thus detection would serve to diagnose the cancer. Alternatively, the biological sample can in some embodiments come from an individual known to have cancer, and detection of the antigen target peptides would serve as an indicator of disease prognosis, cancer characterization, or treatment efficacy. Appropriate immunoassays are well-known in the art and include, but are not limited to, immunohistochemistry, flow cytometry, radioimmunoassay, western blotting, and ELISA. Biological samples suitable for such testing include, but are not limited to, cells, tissue biopsy specimens, whole blood, plasma, serum, sputum, cerebrospinal fluid, pleural fluid, and urine. Antigens recognized by T cells, whether helper T lymphocytes or CTL, are not recognized as intact proteins, but rather as small peptides that associate with class I or class II MHC proteins on the surface of cells. During the course of a naturally occurring immune response antigens that are recognized in association with class II MHC molecules on antigen presenting cells are acquired from outside the cell, internalized, and processed into small peptides that associate with the class II MHC molecules. Conversely, the antigens that give rise to proteins that are recognized in association with class I MHC molecules are generally proteins made within the cells, and these antigens are processed and associate with class I MHC molecules. It is now well-known that the peptides that associate with a given class I or class II MHC molecule are characterized as having a common binding motif, and the binding motifs for a large number of different class I and II MHC molecules have been determined. It is also well-known that synthetic peptides can be made which correspond to the sequence of a given antigen and which contain the binding motif for a given class I or II MHC molecule. These peptides can then be added to appropriate antigen presenting cells, and the antigen presenting cells can be used to stimulate a T helper cell or CTL response either *in vitro* or *in vivo.* The binding motifs, methods for synthesizing the peptides, and methods for stimulating a T helper cell or CTL response are all well-known and readily available.

Kits can in some embodiments be composed for help in diagnosis, monitoring, and/or prognosis. The kits are to facilitate the detecting and/or measuring of cancer-specific target peptides or proteins. Such kits can in some embodiments contain in a single or divided container, a molecule comprising an antigen-binding region. Such molecules can in some embodiments be antibodies and/or antibody-like molecules. Additional components that can be included in the kit include, for example, solid supports, detection reagents, secondary antibodies, instructions for practicing, vessels for running assays, gels, control samples, and the like. The antibody and/or antibody-like molecules can in some embodiments be directly or indirectly labeled, as an option.

Alternatively or in addition, the antibody or antibody-like molecules specific for target peptides and/or target peptide/MHC complexes can in some embodiments be conjugated to therapeutic agents. Exemplary therapeutic agents include:
Alkylating Agents: Alkylating agents are drugs that directly interact with genomic DNA to prevent cells from proliferating. This category of chemotherapeutic drugs represents agents that affect all phases of the cell cycle, that is, they are not phase-specific. An alkylating agent can in some embodiments include, but is not limited to, a nitrogen mustard, an ethylenimene, a methylmelamine, an alkyl sulfonate, a nitrosourea or a triazines. They include but are not limited to busulfan, chlorambucil, cisplatin, cyclophosphamide (cytoxan), dacarbazine, ifosfamide, mechlorethamine (mustargen), and melphalan.

Antimetabolites: Antimetabolites disrupt DNA and RNA synthesis. Unlike alkylating agents, they specifically influence the cell cycle during S phase. Antimetabolites can be differentiated into various categories, such as folic acid analogs, pyrimidine analogs and purine analogs and related inhibitory compounds. Antimetabolites include but are not limited to 5-fluorouracil (5-FU), cytarabine (Ara-C), fludarabine, gemcitabine, and methotrexate.

Natural Products: Natural products generally refer to compounds originally isolated from a natural source, and identified as having a pharmacological activity. Such compounds, as well as analogs and derivatives thereof, can in some embodiments be isolated from a natural source, chemically synthesized or recombinantly produced by any technique known to those of skill in the art. Natural products include such categories as mitotic inhibitors, antitumor antibiotics, enzymes and biological response modifiers.

Mitotic inhibitors include plant alkaloids and other natural agents that can inhibit either protein synthesis required for cell division or mitosis. They operate during a specific phase during the cell cycle. Mitotic inhibitors include, for example, docetaxel, etoposide (VP16), teniposide, paclitaxel, taxol, vinblastine, vincristine, and vinorelbine.

Taxoids are a class of related compounds isolated from the bark of the ash tree, *Taxus brevifolia.* Taxoids include, but are not limited to, compounds such as docetaxel and paclitaxel. Paclitaxel binds to tubulin (at a site distinct from that used by the vinca alkaloids) and promotes the assembly of microtubules.

Vinca alkaloids are a type of plant alkaloid identified to have pharmaceutical activity. They include such compounds as vinblastine (VLB) and vincristine.

Antibiotics: Certain antibiotics have both antimicrobial and cytotoxic activity. These drugs can also interfere with DNA by chemically inhibiting enzymes and mitosis or altering cellular membranes. These agents are typically not phase-specific so they work in all phases of the cell cycle. Examples of cytotoxic antibiotics include but are not limited to bleomycin, dactinomycin, daunorubicin, doxorubicin (Adriamycin), plicamycin (mithramycin), and idarubicin.

Miscellaneous Agents: Miscellaneous cytotoxic agents that do not fall into the previous categories include but are not limited to platinum coordination complexes, anthracenediones, substituted ureas, methyl hydrazine derivatives, amsacrine, L-asparaginase, and tretinoin. Platinum coordination complexes include such compounds as carboplatin and cisplatin (cis-DDP). An exemplary anthracenedione is mitoxantrone. An exemplary substituted urea is hydroxyurea. An exemplary methyl hydrazine derivative is procarbazine (N-methylhydrazine, MIH). These examples are not limiting and it is contemplated that any known cytotoxic, cytostatic, and/or cytocidal agent can be conjugated or otherwise attached to targeting peptides and administered to a targeted organ, tissue, and/or cell type within the scope of the presently disclosed subject matter.

Chemotherapeutic (cytotoxic) agents include but are not limited to 5-fluorouracil, bleomycin, busulfan, camptothecin, carboplatin, chlorambucil, cisplatin (CDDP), cyclophosphamide, dactinomycin, daunorubicin, doxorubicin, estrogen receptor binding agents, etoposide (VP16), farnesyl-protein transferase inhibitors, gemcitabine, ifosfamide, mechlorethamine, melphalan, mitomycin, navelbine, nitrosurea, plicomycin, procarbazine, raioxifene, tamoxifen, taxol, temazolomide (an aqueous form of DTIC), transplatinum, vinblastine and methotrexate, vincristine, or any analog or derivative variant of the foregoing. Most chemotherapeutic agents fall into the categories of alkylating agents, antimetabolites, antitumor antibiotics, corticosteroid hormones, mitotic inhibitors, and nitrosoureas, hormone agents, miscellaneous agents, and any analog or derivative variant thereof.

The peptides identified and tested thus far in peptide-based vaccine approaches have generally fallen into one of three categories: 1) mutated on individual tumors, and thus not displayed on a broad cross section of tumors from different patients; 2) derived from unmutated tissue-specific proteins, and thus compromised by mechanisms of self-tolerance; and 3) expressed in subsets of cancer cells and normal testes.

Antigens linked to transformation or oncogenic processes are of primary interest for immunotherapeutic development based on the hypothesis that tumor escape through mutation of these proteins can be more difficult without compromising tumor growth or metastatic potential.

The target peptides of the presently disclosed subject matter are unique in that the identified target peptides are modified by intracellular modification. This modification is of particular relevance because it is associated with a variety of cellular control processes, some of which are dysregulated in cancer cells. For example, the source proteins for class I MHC-associated phosphopeptides are often known phosphoproteins, supporting the idea that the phosphopeptides are processed from folded proteins participating in signaling pathways.

Although not wishing to be bound by any particular theory, it is envisioned that the target peptides of the presently disclosed subject matter are unexpectedly superior to known tumor-associated antigen-derived peptides for use in immunotherapy because: 1) they only displayed on the surface of cells in which intracellular phosphorylation is dysregulated, *i.e.,* cancer cells, and not normal thymus cells, and thus they are not are not compromised by self-tolerance (as opposed to TAA which are associated with overexpression or otherwise expressed on non-mutated cells); and/or 2) they identify a cell displaying them on their surface as having dysregulated phosphorylation. Thus, post-translationally-modified phosphopeptides that are differentially displayed on cancer cells and derived from source proteins objectively linked to cellular transformation and metastasis allow for more extensive anti-tumor responses to be elicited following vaccination. Target peptides are, therefore, better immunogens in peptide-based vaccines, as target peptides are derived from proteins involved with cellular growth control, survival, or metastasis and alterations in these proteins as a mechanism of immune escape can interfere with the malignant phenotype of tumors.

As such, the presently disclosed subject matter also relates in some embodiments to methods for identifying target peptides for use in immunotherapy which are displayed on transformed cells but are not substantially expressed on normal tissue in general or in the thymus in particular. In some embodiments, target peptides bind the MHC class I molecule more tightly than their non-phosphorylated native counterparts. Moreover, such target peptides can in some embodiments have additional binding strength by having amino acid substitutions at certain anchor positions. In some embodiments, such modified target peptides can remain cross-reactive with TCRs specific for native target peptide MHC complexes. Additionally, it is envisioned that the target peptides associated with proteins involved in intracellular signaling cascades or cycle regulation are of particular interest for use in immunotherapy. In some cases, the TCR binding can specifically react with the phosphate groups on the target peptide being displayed on an MHC class I molecule.

In some embodiments, the method of screening target peptides for use in immunotherapy, *e.g*., in adaptive cell therapy or in a vaccine, involves determining whether the candidate target peptides are capable of inducing a memory T cell response. The contemplated screening methods can include providing target peptides, *e.g.,* those disclosed herein or those to be identified in the future, to a healthy volunteer and determining the extent to which a target peptide-specific T cell response is observed. In some embodiments, the extent to which the T cell response is a memory T cell response is also determined. In some embodiments, it is determined the extent to which a T_{CM} response is elicited, *e.g.,* relative to other T cell types. In some embodiments, those target peptides which are capable of inducing a memory T cell response in health and/or diseased patients are selected for inclusion in the therapeutic compositions of the presently disclosed subject matter.

In some embodiments, the presently disclosed subject matter provides methods for inducing a target peptide-specific memory T cell response (e.g., T_{CM}) response in a patient by providing the patient with a composition comprising the target peptides disclosed herein. In some embodiments, the compositions are those disclosed herein and are provided in a dosing regimen disclosed herein.

In some embodiments, the presently disclosed subject matter relates to methods for determining a cancer disease prognosis. These methods involve providing a patient with target peptide compositions and determining the extent to which the patient is able to mount a target peptide specific T cell response. In some embodiments, the target peptide composition contains target peptides selected in the same substantially the same manner that one would select target peptides for inclusion in a therapeutic composition. If a patient is able to mount a significant target peptide-specific T cell response, then the patient is likely to have a better prognosis than a patient with the similar disease and therapeutic regimen that is not able to mount a target peptide-specific T cell response. In some embodiments, the methods involve determining whether the target peptide specific T cell response is a T_{CM} response. In some embodiments, the presence of a target peptide-specific T cell response as a result of the presently disclosed diagnostic methods correlates with an at least or about 5, 10, 15, 20, 25, 30, 35, 40, 45, 50, 55, 60, 65, 70, 75, 80, 85, 90, 95, 100, 125, 150, 175, 200, 250, 300, 400, 500, or more percent increase in progression free survival over standard of care.

### REFERENCES

All references listed in the instant disclosure, including but not limited to all patents, patent applications and publications thereof, scientific journal articles, and database entries (including but not limited to Uniprot, EMBL, and GENBANK® biosequence database entries and including all annotations available therein) are incorporated herein by reference in their entireties to the extent that they supplement, explain, provide a background for, and/or teach methodology, techniques, and/or compositions employed herein. The discussion of the references is intended merely to summarize the assertions made by their authors. No admission is made that any reference (or a portion of any reference) is relevant prior art. Applicants reserve the right to challenge the accuracy and pertinence of any cited reference.
Altman et al. (1996) Phenotypic analysis of antigen-specific T lymphocytes [published erratum appears in Science 1998 Jun 19;280(5371):1821]. Science 274:94-96.
Arentz-Hansen et al. (2000) The intestinal T cell response to alpha-gliadin in adult celiac disease is focused on a single deamidated glutamine targeted by tissue transglutaminase. J Exp Med 191:603-612.
Arozarena et al. (2011) In melanoma, beta-catenin is a suppressor of invasion. Oncogene 30:4531-4543.
Bachmann et al. (2005) Importance of P-cadherin, beta-catenin, and Wnt5a/frizzled for progression of melanocytic tumors and prognosis in cutaneous melanoma. Clin Cancer Res 11:8606-8614.
Baron et al. (2005) Graft-versus-tumor effects after allogeneic hematopoietic cell transplantation with nonmyeloablative conditioning. J Clin Oncol 23:1993-2003.
Bertoletti et al. (1994) Natural variants of cytotoxic epitopes are T-cell receptor antagonists for antiviral cytotoxic T cells. Nature 369:407-410.
Berzofsky et al. (1988) Antigen processing for presentation to T lymphocytes: function, mechanisms, and implications for the T-cell repertoire. Immunol Rev 106:5-31.
Bullock et al. (2000) The density of peptides displayed by dendritic cells affects immune responses to human tyrosinase and gp100 in HLA-A2 transgenic mice. J Immunol 164:2354-2361.
Chi (2011) Cancer research: Promise of protection. Nature 471:537-538.
Chien et al. (2009) Activated Wnt/beta-catenin signaling in melanoma is associated with decreased proliferation in patient tumors and a murine melanoma model. Proc Natl Acad Sci U S A 106:1193-1198.
Cobbold et al. (2005) Adoptive transfer of cytomegalovirus-specific CTL to stem cell transplant patients after selection by HLA-peptide tetramers. J Exp Med 202:379-386.
Crawford et al. (1999) The metalloproteinase matrilysin is a target of beta-catenin transactivation in intestinal tumors. Oncogene 18:2883-2891.
Demunter et al. (2002) Loss of membranous expression of beta-catenin is associated with tumor progression in cutaneous melanoma and rarely caused by exon 3 mutations. Modern Pathol 15:454-461.
Dephoure et al. (2008) A quantitative atlas of mitotic phosphorylation. Proc Natl Acad Sci USA 105:10762-10767.
Depontieu et al. (2009) Identification of tumor-associated, MHC class II-restricted phosphopeptides as targets for immunotherapy. Proc Natl Acad SciU S A 106:12073-12078.
Dudley et al. (2008) Adoptive cell therapy for patients with metastatic melanoma: evaluation of intensive myeloablative chemoradiation preparative regimens. J Clin Oncol 26:5233-5239.
DuPage et al. (2012) Expression of tumour-specific antigens underlies cancer immunoediting. Nature 482:405-409.
Finn (2003) Premalignant lesions as targets for cancer vaccines. J Exp Med 198:1623-1626.
Fiol et al. (1988) Phosphoserine as a recognition determinant for glycogen synthase kinase-3: phosphorylation of a synthetic peptide based on the G-component of protein phosphatase-1. Arch Biochem Biophys 267:797-802.
Fiol et al. (1990) Ordered multisite protein phosphorylation. Analysis of glycogen synthase kinase 3 action using model peptide substrates. J Biol Chem 265:6061-6065.
Fischbein et al. (2000) CD40 signaling replaces CD4+ lymphocytes and its blocking prevents chronic rejection of heart transplants. J Immunol 165:7316-7322.
Gale et al. (1994) Identical-twin bone marrow transplants for leukemia. Ann Intern Med 120:646-652.
Gerdes et al. (1999) Analysis of beta-catenin gene mutations in pancreatic tumors. Digestion 60:544-548.
Girbal-Neuhauser et al. (1999) The epitopes targeted by the rheumatoid arthritis-associated antifilaggrin autoantibodies are posttranslationally generated on various sites of (pro)filaggrin by deimination of arginine residues. J Immunol 162:585-594.
Goldman & DeFrancesco (2009) The cancer vaccine roller coaster, Nat Biotech 27:129-139 (Corrected online: 7 June 2010 | doi:10.1038/nbt0209-129).
Hall et al. (2010) Comprehensive analysis of phosphorylation sites in Tensin1 reveals regulation by p38MAPK. Mol Cell Proteomics 9:2853-2863.
Haluska et al. (2006) Genetic alterations in signaling pathways in melanoma. Clin Cancer Res 12:2301s-2307s.
He et al. (1998) Identification of c-MYC as a target of the APC pathway. Science 281:1509-1512.
Hirohashi et al. (2009) The functioning antigens: beyond just as the immunological targets. Cancer Sci 100:798-806.
Ho et al. (2006) In vitro methods for generating CD8+ T-cell clones for immunotherapy from the naive repertoire. J Immunol Methods 310:40-52.
Hoek et al. (2006) Metastatic potential of melanomas defined by specific gene expression profiles with no BRAF signature. Pigment Cell Res 19:290-302.
Hogan et al. (1998) The peptide recognized by HLA-A68.2-restricted, squamous cell carcinoma of the lung-specific cytotoxic T lymphocytes is derived from a mutated elongation factor 2 gene. Cancer Res 58:5144-5150.
Homfray et al. (1998) Defects in mismatch repair occur after APC mutations in the pathogenesis of sporadic colorectal tumours. Human Mutation 11:114-120.
Horowitz et al. (1990) Graft-versus-leukemia reactions after bone marrow transplantation. Blood 75:555-562.
Hulsken et al. (1994) E-cadherin and APC compete for the interaction with beta-catenin and the cytoskeleton. J Cell Biol 127:2061-2069.
Ilyas et al. (1997) Beta-catenin mutations in cell lines established from human colorectal cancers. Proc Natl Acad Sci U S A 94:10330-10334.
Isakoff et al. (2005) Breast cancer-associated PIK3CA mutations are oncogenic in mammary epithelial cells. Cancer Res 65:10992-11000.
Jimbow et al. (1975) Mitotic activity in non-neoplastic melanocytes in vivo as determined by histochemical, autoradiographic, and electron microscope studies. J Cell Biol 66:663-670.
Jones et al. (2008) Core signaling pathways in human pancreatic cancers revealed by global genomic analyses. Science 321:1801-1806.
Kageshita et al. (2001) Loss of beta-catenin expression associated with disease progression in malignant melanoma. Br J Dermatol 145:210-216.
Kantoff et al. (2010) Sipuleucel-T immunotherapy for castration-resistant prostate cancer. N Engl J Med 363:411-422.
Kielhorn et al. (2003) Tissue microarray-based analysis shows phospho-beta-catenin expression in malignant melanoma is associated with poor outcome. Intl J Cancer 103:652-656.
Kim et al. (2000) beta-catenin expression and mutational analysis in renal cell carcinomas. Pathol Intl 50:725-730.
Kimelman & Xu (2006) beta-catenin destruction complex: insights and questions from a structural perspective. Oncogene 25:7482-7491.
Klenerman et al. (1994) Cytotoxic T-cell activity antagonized by naturally occurring HIV-1 Gag variants. Nature 369:403-407.
Kolb et al. (1990) Donor leukocyte transfusions for treatment of recurrent chronic myelogenous leukemia in marrow transplant patients. Blood 76:2462-2465.
Kolb (2008) Graft-versus-leukemia effects of transplantation and donor lymphocytes. Blood 112:4371-4383.
Krengel et al. (2004) Cadherin expression pattern in melanocytic tumors more likely depends on the melanocyte environment than on tumor cell progression. J Cutaneous Pathol 31:1-7.
Kroger et al. (2005) Stem cell transplantation from identical twins in patients with myelodysplastic syndromes. Bone Marrow Transplant 35:37-43.
Ley et al. (2008) DNA sequencing of a cytogenetically normal acute myeloid leukaemia genome. Nature 456:66-72.
Liu et al. (2002) Control of beta-catenin phosphorylation/degradation by a dual-kinase mechanism. Cell 108:837-847.
Lucas & Coulie (2008) About human tumor antigens to be used in immunotherapy. Sem Immunol 20:301-307.
Maelandsmo et al. (2003) Reduced beta-catenin expression in the cytoplasm of advanced-stage superficial spreading malignant melanoma. Clin Cancer Res 9:3383-3388.
Mamula et al. (1999) Isoaspartyl post-translational modification triggers autoimmune responses to self-proteins. J Biol Chem 274:22321-22327.
Marafioti et al. (2004) Leukocyte-specific phosphoprotein-1 and PU.1: two useful markers for distinguishing T-cell-rich B-cell lymphoma from lymphocyte-predominant Hodgkin's disease. Haematologica 89:957-964.
Matsushita et al. (2012) Cancer exome analysis reveals a T-cell-dependent mechanism of cancer immunoediting. Nature 482:400-404.
Meyer et al. (2009) Identification of natural MHC class II presented phosphopeptides and tumor-derived MHC class I phospholigands. J Proteome Res 8:3666-3674.
Miyake et al. (2001) Absence of mutations in the beta-catenin and adenomatous polyposis coli genes in papillary and follicular thyroid carcinomas. Pathol Intl 51:680-685.
Mohammed et al. (2008) Phosphorylation-dependent interaction between antigenic peptides and MHC class I: a molecular basis for the presentation of transformed self. Nat Immunol 9:1236-1243.
Molina et al. (2007) Global proteomic profiling of phosphopeptides using electron transfer dissociation tandem mass spectrometry. Proc Natl Acad Sci U S A 104:2199-2204.
Morin et al. (1997) Activation of beta-catenin-Tcf signaling in colon cancer by mutations in beta-catenin or APC. Science 275:1787-1790.
Newberg et al. (1992) Species specificity in the interaction of CD8 with the α3 domain of MHC class I molecules. J Immunol 149:136-142.
Niedermann et al. (1995) Contribution of proteasome-mediated proteolysis to the hierarchy of epitopes presented by major histocompatibility complex class I molecules. Immunity 2:289-299.
Nunes et al. (2011) A novel tumor antigen derived from enhanced degradation of bax protein in human cancers. Cancer Res 71:5435-5444.
Offringa (2009) Antigen choice in adoptive T-cell therapy of cancer. Curr Opin Immunol 21:190-199.
Ogasawara et al. (2006) Mutations and nuclear accumulation of beta-catenin correlate with intestinal phenotypic expression in human gastric cancer. Histopathology 49:612-621.
Ohgaki et al. (2004) APC mutations are infrequent but present in human lung cancer. Cancer Lett 207:197-203.
Oliva et al. (2006) High frequency of beta-catenin mutations in borderline endometrioid tumours of the ovary. J Pathol 208:708-713.
Olmeda et al. (2003) Beta-catenin regulation during the cell cycle: implications in G2/M and apoptosis. Mol Biol Cell 14:2844-2860.
Omholt et al. (2001) Cytoplasmic and nuclear accumulation of beta-catenin is rarely caused by CTNNB1 exon 3 mutations in cutaneous malignant melanoma. Intl J Cancer 92:839-842.
Parsons et al. (2011) The Genetic Landscape of the Childhood Cancer Medulloblastoma. Science 331:435-439.
Pavletic et al. (2007) Genetically identical twin transplantation for chronic lymphocytic leukemia. Leukemia 21:2452-2455.
PCT International Patent Application Publication No. WO 2011/0149909.
Pecina-Slaus et al. (2007) E-cadherin and beta-catenin expression patterns in malignant melanoma assessed by image analysis. J Cutaneous Pathol 34:239-246.
Petersen et al. (2009) Phosphorylated self-peptides alter human leukocyte antigen class I-restricted antigen presentation and generate tumor-specific epitopes. Proc Natl Acad Sci U S A 106:2776-2781.
Pollock & Hayward (2002) Mutations in exon 3 of the beta-catenin gene are rare in melanoma cell lines. Melanoma Res 12:183-186.
Preudhomme et al. (2010) Imatinib plus peginterferon alfa-2a in chronic myeloid leukemia. N Engl J Med 363:2511-2521.
Rappsilber et al. (2007) Protocol for micro-purification, enrichment, pre-fractionation and storage of peptides for proteomics using StageTips. Nature Protocols 2:1896-1906.
Restifo et al. (1993) Identification of human cancers deficient in antigen processing. J Exp Med 177:265-272.
Rimm et al. (1999) Frequent nuclear/cytoplasmic localization of beta-catenin without exon 3 mutations in malignant melanoma. Am J Pathol 154:325-329.
Robila et al. (2008) MHC class II presentation of gp100 epitopes in melanoma cells requires the function of conventional endosomes and is influenced by melanosomes. J Immunol 181:7843-7852.
Rosenberg & Dudley (2009) Adoptive cell therapy for the treatment of patients with metastatic melanoma. Curr Opin Immunol 21:233-240.
Rosenberg et al. (1986) A new approach to the adoptive immunotherapy of cancer with tumor-infiltrating lymphocytes. Science 233:1318-1321.
Rosenberg et al. (2004) Cancer immunotherapy: moving beyond current vaccines. Nat Med 10:909-915.
Ruppert et al. (1993) Prominent role of secondary anchor residues in peptide binding to A2.1 molecules. Cell 74:929-937.
Sadot et al. (2002) Regulation of S33/S37 phosphorylated beta-catenin in normal and transformed cells. J Cell Sci 115:2771-2780.
Sanders et al. (1999) Alterations in cadherin and catenin expression during the biological progression of melanocytic tumours. Mol Pathol 52:151-157.
Schreiber et al. (2011) Cancer immunoediting: integrating immunity's roles in cancer suppression and promotion. Science 331:1565-1570.
Seidensticker & Behrens (2000) Biochemical interactions in the wnt pathway. Biochim Biophys Acta 1495:168-182.
Sette et al. (1994) The relationship between class I binding affinity and immunogenicity of potential cytotoxic T cell epitopes. J Immunol 153:5586-5592.
Slawson et al. (2005) Perturbations in O-linked β-N-acetylglucosamine proteim modification cause severe defects in mitotic progression and cytokinesis. J Biol Chem 280:32944-32956.
Slawson et al. (2008) A mitotic GlcN Acylation/phosphorylation signaling complex alters the posttranslational state of the cytoskeletal proteim vimentin. Mol Biol. Cell 19:4130-4140.
Slingluff et al. (2000) Melanomas with concordant loss of multiple melanocytic differentiation proteins: immune escape that may be overcome by targeting unique or undefined antigens. Cancer Immunol Immunother 48:661-672.
Sun et al. (2005) Infrequent mutation of APC, AXIN1, and GSK3B in human pituitary adenomas with abnormal accumulation of CTNNB1. J Neurooncol 73:131-134.
Takahashi et al. (2002) Identification of membrane-type matrix metalloproteinase-1 as a target of the beta-catenin/Tcf4 complex in human colorectal cancers. Oncogene 21:5861-5867.
Takemaru et al. (2008) An oncogenic hub: beta-catenin as a molecular target for cancer therapeutics. Handb Exp Pharmacol 186:261-84.
Talpaz et al. (1986) Hematologic remission and cytogenetic improvement induced by recombinant human interferon alpha A in chronic myelogenous leukemia. N Engl J Med 314:1065-1069.
Tetsu & McCormick (1999) Beta-catenin regulates expression of cyclin D1 in colon carcinoma cells. Nature 398:422-426.
U.S. Patent Application Publication No. 2005/0277161.
U.S. Provisional Application Serial No. 61/695,776.
Utz et al. (1997) Proteins phosphorylated during stress-induced apoptosis are common targets for autoantibody production in patients with systemic lupus erythematosus. J Exp Med 185:843-854.
van Doom et al. (2005) Epigenetic profiling of cutaneous T cell lymphoma: promoter hypermethylation of multiple tumor suppressor genes including BCL7a, PTPRG, and p73. J Clin Oncol 23:3886-3896.
Wang et al. (2007) Dynamic interplay between O-linked N-acetylglucosaminylation and glyocen synthase kinase-3-dependent phosphorylation. Mol Cell Proteomics 6:1365-1379.
Wang et al. (2010) Extensive Crosstalk Between O-GlcNAcylation and Phosphorylation Regulates Cytokinesis, Sci Signal 3(104):ra2, including Supplemental Materials.
Waun Ki Hong et al. Holland-Frei Cancer Medicine 10 A.D. McGraw-Hill Medical. Ref Type: Edited Book
Worm et al. (2004) Genetic and epigenetic alterations of the APC gene in malignant melanoma. Oncogene 23:5215-5226.
Wuttge et al. (1999) T cell recognition of lipid peroxidation products breaks tolerance to self proteins. Immunol 98:273-279.
Yewdell (2002) To DRiP or not to DRiP: generating peptide ligands for MHC class I molecules from biosynthesized proteins. Mol Immunol 39:139-146.
Yost et al. (1996) The axis-inducing activity, stability, and subcellular distribution of beta-catenin is regulated in Xenopus embryos by glycogen synthase kinase 3. Genes Dev 10:1443-1454.
Zarling et al. (2000) Phosphorylated peptides are naturally processed and presented by MHC class I molecules in vivo. J Exp Med 192:1755-1762.
Zarling et al. (2006) Identification of class I MHC associated phosphopeptides as targets for cancer immunotherapy. Proc Natl Acad Sci U S A 103:14889-14894.

It will be understood that various details of the presently disclosed subject matter can be changed without departing from the scope of the presently disclosed subject matter. Furthermore, the foregoing description is for the purpose of illustration only, and not for the purpose of limitation.

**Table 3**

| HLA A^{∗}0201 Phosphopeptides on Transformed Ovarian Cells (FHIOSE and/or SKOV3) | | | | | | |
|---|---|---|---|---|---|---|
| SEQ ID NO. | Peptide Sequence | F/S | Start | Stop | UniProt/ GENBANK® Acc. No. | Source Protein |
| 1 | AILsPAFKV | F | 381 | 389 | P34932 | Heat shock 70 kDa protein 4 |
| 2 | AIMRsPQMV | F | 187 | 195 | P35222 | Catenin beta-1 |
| 3 | ALDsGASLLHL | S | 482 | 492 | P57078 | Receptor-interacting serine/threonine-protein kinase 4 |
| 4 | ALGNtPPFL | S | 111 | 119 | Q7Z739 | YTH domain family protein 3 |
| 5 | ALLsLLKRV | S | 25 | 33 | Q9UPU9 | Protein Smaug homolog 1 |
| 6 | AMAAsPHAV | S | 64 | 72 | Q13151 | Heterogeneous nuclear ribonucleoprotein A0 |
| 7 | AMLGSKsPDPYRL | F/S | 904 | 916 | P18583 | Protein SON |
| 8 | ATWsGSEFEV | S | 356 | 368 | Q9BQQ3 | Golgi reassembly-stacking protein 1 |
| 9 | AVVsPPALHNA | S | 855 | 865 | O60885 | Bromodomain-containing protein 4 |
| 10 | DLRtVEKEL | F | 240 | 248 | P35237 | Serpin B6 |
| 11 | DLWKItKVMD | S | 430 | 439 | O96005 | Cleft lip and palate transmembrane protein 1 |
| 12 | ELFSsPPAV | F | 953 | 961 | 094916 | Nuclear factor of activated T-cells 5 |
| 13 | ELRISGsVQL | F | 322 | 331 | Q96DT0 | Galectin-12 |
| 14 | FIGsPTTPAGL | S | 2125 | 2135 | 014686 | Histone-lysine N-methyltransferase MLL2 |
| 15 | FLDNsFEKV | F | 576 | 584 | O43303 | Centriolar coiled-coil protein of 110 kDa |
| 16 | FLDRPPtPLFI | S | 280 | 290 | Q86UC2 | Radial spoke head protein 3 homolog |
| 17 | FLDsLRDLI | F | 161 | 169 | P63010 | AP-2 complex subunit beta |
| 18 | FLFDKPVsPLLL | S | 192 | 203 | P06732 | Creatine kinase M-type |
| 19 | FLGVRPKsA | S | 1283 | 1291 | Q9BZ95 | Histone-lysine N-methyltransferase NSD3 |
| 20 | FLITGGGKGsGFSL | S | 246 | 259 | 043166 | Signal-induced proliferation-associated 1 -like protein 1 |
| 21 | FLLsQNFDDE | S | 354 | 363 | P54725 | UV excision repair protein RAD23 homolog A |
| 22 | GALsPSLLHSL | F | 1527 | 1537 | P10070 | Zinc finger protein GLI2 |
| 23 | GLAPtPPSM | S | 1197 | 1205 | Q99700 | Ataxin-2 |
| 24 | GLDsLDQVEI | S | 109 | 118 | 014561 | Acyl carrier protein, mitochondrial |
| 25 | GLGELLRsL | F | 110 | 118 | P50454 | Serpin H1 |
| 26 | GLIsPELRHL | F | 86 | 95 | Q147X3 | N-alpha-acetyltransferase 30 |
| 27 | GLIsPNVQL | F | 742 | 750 | A0AVK6 | Transcription factor E2F8 |
| 28 | GLIsPVWGA | F/S | 50 | 58 | Q76N32 | Centrosomal protein of 68 kDa |
| 29 | GLItPGGFSSV | S | 744 | 754 | Q13435 | Splicing factor 3B subunit 2 |
| 30 | GLLDsPTSI | F | 218 | 226 | Q07352 | Zinc finger protein 36, C3H1 type-like 1 |
| 31 | GLLGsPARL | F | 232 | 240 | Q6UXB0 | Protein FAM131A |
| 32 | GLLGsPVRA | F/S | 38 | 46 | P30305 | M-phase inducer phosphatase 2 |
| 33 | GLLsPRFVDV | S | 525 | 534 | Q8WYP5 | Protein ELYS |
| 34 | GLLsPRHSL | F | 913 | 921 | Q9Y2K2 | Serine/threonine-protein kinase SIK3 |
| 35 | GMLsPGKSIEV | S | 4474 | 4484 | Q8IVF2 | Protein AHNAK2 |
| 36 | GsQLAVMMYL | S | 17 | 26 | O60512 | Beta-1,4-galactosyltransferase 3 |
| 37 | GVAsPTITV | F | 626 | 634 | P46379 | Large proline-rich protein BAG6 |
| 38 | GVVsPTFEL | F | 447 | 455 | B4DIR9 | TGF-beta-activated kinase 1 and MAP3K7-binding protein 2 |
| 39 | HLHsPQHKL | S | 547 | 555 | Q6T4R5 | Nance-Horan syndrome protein |
| 40 | ILQtPQFQM | F/S | 208 | 216 | Q14980 | Nuclear mitotic apparatus protein 1 |
| 41 | ILQVsIPSL | S | 404 | 412 | Q86W92 | Liprin-beta-1 |
| 42 | IVLsDSEVIQL | S | 75 | 85 | Q8N3Z6 | Zinc finger CCHC domain-containing protein 7 |
| 43 | KAFsPVRSV | F/S | 2 | 10 | Q02363 | DNA-binding protein inhibitor ID-2 |
| 44 | KIAsEIAQL | F | 541 | 549 | Q8WXE0 | Caskin-2 |
| 45 | KIEsLENLYL | F | 385 | 394 | Q659A1 | NMDA receptor-regulated protein 2 |
| 46 | KIGsIIFQV | F/S | 1223 | 1231 | Q460N5 | Poly [ADP-ribose] polymerase 14 |
| 47 | KLAsLEREASV | S | 368 | 378 | Q8WYA0 | Intraflagellar transport protein 81 homolog |
| 48 | KLAsPEKLAGL | F/S | 987 | 997 | Q6T4R5 | Nance-Horan syndrome protein |
| 49 | KLAsPELERL | F/S | 70 | 79 | P05412 | Transcription factor AP-1 |
| 50 | KLFPDtPLAL | F/S | 587 | 596 | Q12906 | Interleukin enhancer-binding factor 3 |
| 51 | KLFsPSKEAEL | F | 845 | 855 | Q96RY5 | Protein cramped-like |
| 52 | KLIDIVsSQKV | S | 461 | 471 | O14757 | Serine/threonine-protein kinase Chk1 |
| 53 | KLKsQEIFL | F | 416 | 424 | Q9BZD4 | Kinetochore protein Nuf2 |
| 54 | KLLsPSDEKL | F | 544 | 553 | Q14694 | Ubiquitin carboxyl-terminal hydrolase 10 |
| 55 | KLLsPSNEKL | F | 544 | 553 | Q14694 | Ubiquitin carboxyl-terminal hydrolase 10 |
| 56 | KLMAPDIsL | F | 52 | 60 | Q12982 | BCL2/adenovirus E1B 19 kDa protein-interacting protein 2 |
| 57 | KLMsPKADV | F/S | 44 | 52 | Q86T90 | Uncharacterized protein KIAA1328 |
| 58 | KLMsPKADVKL | F/S | 44 | 54 | Q86T90 | Uncharacterized protein KIAA1328 |
| 59 | KLQEFLQtL | F | 16 | 24 | Q9NVI1 | Fanconi anemia group I protein |
| 60 | KQDsLVINL | F | 647 | 655 | Q9Y5B9 | FACT complex subunit SPT16 |
| 61 | KRLsTSPVRL | S | 757 | 766 | Q9Y2J2 | Band 4.1-like protein 3 |
| 62 | KTMsGTFLL | F | 592 | 600 | P52630 | Signal transducer and activator of transcription 2 |
| 63 | KTWKGsIGL | F/S | 822 | 831 | Q8IY63 | Angiomotin-like protein 1 |
| 64 | KVLsKEFHL | S | 150 | 158 | Q01105 | Protein SET |
| 65 | KVLsTEEMEL | F | 31 | 40 | Q6P582 | Mitotic-spindle organizing protein 2A |
| 66 | KVLStEEMEL | F | 31 | 40 | Q6P582 | Mitotic-spindle organizing protein 2A |
| 67 | LLAsPGHISV | S | 740 | 749 | A0FGR8 | Extended synaptotagmin-2 |
| 68 | LQLsPLKGLSL | F/S | 17 | 27 | P31350 | Ribonucleoside-diphosphate reductase subunit M2 |
| 69 | LQNItENQL | S | 86 | 94 | Q8N5J4 | Transcription factor Spi-C |
| 70 | NLGsRNHVHQL | S | 1398 | 1408 | Q9HAR2 | Latrophilin-3 |
| 71 | NLLsPDGKMISV | S | 395 | 405 | P35680 | Hepatocyte nuclear factor 1-beta |
| 72 | RASsLSITV | F | 839 | 847 | Q6ZS17 | Protein FAM65A- isoform 2 |
| 73 | REDsTPGKVFL | S | 61 | 71 | P13056 | Nuclear receptor subfamily 2 group C member 1 |
| 74 | RIDsKDSASEL | S | 602 | 612 | Q96S38 | Ribosomal protein S6 kinase delta-1 |
| 75 | RINsFEEHV | S | 475 | 483 | Q16875 | 6-phosphofructo-2-kinase/fructose-2,6-bisphosphatase 3 |
| 76 | RIQsKLYRA | F | 483 | 491 | O75643 | U5 small nuclear ribonucleoprotein 200 kDa helicase |
| 77 | RITsLIVHV | F | 315 | 323 | Q3ZCT1 | Zinc finger protein 260 |
| 78 | RLAsASRAL | F | | | | No database hit |
| 79 | RLAsLNAEAL | F | 118 | 127 | Q8TBE0 | Bromo adjacent homology domain-containing 1 protein |
| 80 | RLAsRPLLL | F | 3 | 11 | Q9P2B2 | Prostaglandin F2 receptor negative regulator |
| 81 | RLDsYLRAP | S | 137 | 145 | O95833 | Chloride intracellular channel protein 3 |
| 82 | RLDsYVR | F | 129 | 135 | Q9Y5R8 | Trafficking protein particle complex subunit 1 |
| 83 | RLDsYVRSL | F/S | 129 | 137 | Q9Y5R8 | Trafficking protein particle complex subunit 1 |
| 84 | RLDtGPQSL | S | 424 | 432 | P35269 | General transcription factor IIF subunit 1 |
| 85 | RLEsANRRL | S | 397 | 405 | Q9Y2J4 | Angiomotin-like protein 2 |
| 86 | RLFsKELRC* | F/S | 30 | 38 | Q15543 | Transcription initiation factor TFIID subunit 13 |
| 87 | RLFSLsNPSL | F | 365 | 374 | Q6UUV7 | CREB-regulated transcription coactivator 3 |
| 88 | RLFsQGQDV | S | 1796 | 1804 | P55196 | Afadin |
| 89 | RLGsFHELLL | F/S | 312 | 321 | Q5H9R7 | Serine/threonine-protein phosphatase 6 regulatory subunit 3 |
| 90 | RLKsDERPVHI | S | 1116 | 1126 | Q9UPN9 | E3 ubiquitin-protein ligase TRIM33 |
| 91 | RLLsDGQQHL | F | 2080 | 2089 | Q02224 | Centromere-associated protein E |
| 92 | RLLsDLEEL | F | 245 | 253 | Q8IWP9 | Coiled-coil domain-containing protein 28A |
| 93 | RLLsDQTRL | F | 232 | 240 | Q8TDM6 | Disks large homolog 5 |
| 94 | RLLsFQRYL | F | 110 | 118 | Q13946 | High affinity cAMP-specific 3',5'-cyclic phosphodiesterase 7A |
| 95 | RLLsPLSSA | F | 581 | 589 | E9PAU2 | Ribonucleoprotein PTB-binding 1 |
| 96 | RLLsPLSSARL | F | 581 | 589 | E9PAU2 | Ribonucleoprotein PTB-binding 1 |
| 97 | RLLsPRPSL | F | 936 | 944 | Q9Y618 | Nuclear receptor corepressor 2 |
| 98 | RLLsPRPSLL | F | 936 | 945 | Q9Y618 | Nuclear receptor corepressor 2 |
| 99 | RLLsVHDFDF | F | 188 | 197 | Q9BV36 | Melanophilin |
| 100 | RLNtSDFQKL | S | 243 | 252 | Q96B36 | Proline-rich AKT1 substrate 1 |
| 101 | RLPNRIPsL | F | 640 | 648 | Q9P227 | Rho GTPase-activating protein 23 |
| 102 | RLQsLIKNI | F/S | 632 | 640 | Q14527 | Helicase-like transcription factor |
| 103 | RLQsTSERL | F | 217 | 225 | Q96TA2 | ATP-dependent zinc metalloprotease YME1L1 |
| 104 | RLRsYEDMI | F/S | 317 | 325 | O60716 | Catenin delta-1 |
| 105 | RLSsPLHFV | F/S | 400 | 408 | Q8NC44 | Protein FAM134A |
| 106 | RMFPtPPSL | F | 863 | 871 | Q71F56 | Mediator of RNA polymerase II transcription subunit 13-like |
| 107 | RMFsPMEEKELL | F | 691 | 702 | Q9UHB7 | AF4/FMR2 family member 4 |
| 108 | RMIsTGSEL | F | 207 | 215 | Q86T82 | Ubiquitin carboxyl-terminal hydrolase 37 |
| 109 | RMLsLRDQRL | F | 15 | 24 | Q9Y324 | rRNA-processing protein FCF1 homolog |
| 110 | RMYsFDDVL | F | 802 | 810 | Q8WWI1 | LIM domain only protein 7 |
| 111 | RMYsPIIYQA | S | 200 | 209 | Q49A88 | Coiled-coil domain-containing protein 14 |
| 112 | RQDsTPGKVFL | F/S | 61 | 71 | P13056 | Nuclear receptor subfamily 2 group C member 1 |
| 113 | RQIsFKAEV | F | 181 | 189 | Q9Y385 | Ubiquitin-conjugating enzyme E2 J1 |
| 114 | RQIsQDVKL | F | 165 | 173 | Q01433 | AMP deaminase 2 |
| 115 | RQLsALHRA | F/S | 31 | 39 | P61313 | 60S ribosomal protein L15 |
| 116 | RQLsLEGSGLGV | S | 749 | 760 | Q9UMZ2 | Synergin gamma |
| 117 | RQLsSGVSEI | S | 79 | 88 | P04792 | Heat shock protein beta-1 |
| 118 | RQSsSRFNL | F | 86 | 94 | Q14738 | Serine/threonine-protein phosphatase 2A 56 kDa regulatory subunit |
| 119 | RRLsERETR | S | 148 | 156 | O60285 | NUAK family SNF1-like kinase 1 |
| 120 | RSAsPDDDLGSSN | S | 14 | 26 | O00193 | Small acidic protein |
| 121 | RSFsPTMKV | F/S | 211 | 219 | A3KN83 | Protein strawberry notch homolog 1 |
| 122 | RSLsQELVGV | S | 333 | 342 | Q5VUA4 | Zinc finger protein 318 |
| 123 | RTAsLIIKV | F | 2707 | 2715 | Q7Z7G8 | Vacuolar protein sorting-associated protein 13B |
| 124 | RTFsLDTIL | F | 88 | 96 | Q9C073 | Protein FAM117A |
| 125 | RTFsPTYGL | F/S | 426 | 434 | O15061 | Synemin |
| 126 | RTHsLLLLL | F/S | 5 | 13 | P34096 | Ribonuclease 4 |
| 127 | RTLsHISEA | F | 450 | 458 | Q6ZS17 | Protein FAM65A |
| 128 | RTSsFTEQL | F | 38 | 46 | Q13439 | Golgin subfamily A member 4 |
| 129 | RVAsPTSGV | F | 1097 | 1105 | Q9Y4H2 | Insulin receptor substrate 2 |
| 130 | RVDsPSHGL | F | 685 | 693 | Q9UER7 | Death domain-associated protein 6 |
| 131 | RVGsLVLNL | F | | | | No database hit |
| 132 | RVIsGVLQL | F | 341 | 349 | P35579 | Myosin-9 |
| 133 | RVLHsPPAV | F | 1212 | 1220 | A8MQ54 | Protein SOGA2 |
| 134 | RVPsLLVLL | F | 4 | 12 | P19021 | Peptidyl-glycine alpha-amidating monooxygenase |
| 135 | RVTsAEIKL | F | 648 | 656 | Q8N4X5 | Actin filament-associated protein 1-like 2 |
| 136 | RVWsPPRVHKV | S | 613 | 623 | O15209 | Zinc finger and BTB domain-containing protein 22 |
| 137 | SARGsPTRPNPPVR | F | 518 | 531 | Q14195 | Dihydropyrimidinase-related protein 3 |
| 138 | SILsFVSGL | S | 1715 | 1724 | O95996 | Adenomatous polyposis coli protein 2 |
| 139 | SIMsFHIDL | F/S | 204 | 213 | Q9H3Q1 | Cdc42 effector protein 4 |
| 140 | SIMsPEIQL | F/S | 153 | 162 | Q96RK0 | Protein capicua homolog |
| 141 | SISStPPAV | s | 260 | 268 | Q9H8Y8 | Golgi reassembly-stacking protein 2 |
| 142 | SKtVATFIL | F | 178 | 186 | Q92600 | Cell differentiation protein RCD1 homolog |
| 143 | SLAsLTEKI | F | 369 | 377 | Q5M775 | Cytospin-B |
| 144 | SLDSEDYsL | F | 253 | 261 | Q00987 | E3 ubiquitin-protein ligase Mdm2 |
| 145 | SLDsLGDVFL | F/S | 1789 | 1798 | Q14980 | Nuclear mitotic apparatus protein 1 |
| 146 | SLFGGsVKL | F | 103 | 111 | Q8WUM4 | Programmed cell death 6-interacting protein |
| 147 | SLFKRLYsL | F | 1058 | 1066 | P78527 | DNA-dependent protein kinase catalytic subunit |
| 148 | SLFsSEESNLGA | F | 403 | 414 | P04004 | Vitronectin |
| 149 | SLFsGDEENA | S | 22 | 31 | Q53EL6 | Programmed cell death protein 4 |
| 150 | SLFsGSYSSL | S | 147 | 156 | Q13490 | Baculoviral IAP repeat-containing protein 2 |
| 151 | SLLAsPGHISV | S | 739 | 749 | A0FGR8 | Extended synaptotagmin-2 |
| 152 | SLLHTSRsL | F | 1240 | 1248 | Q6P0Q8 | Microtubule-associated serine/threonine-protein kinase 2 |
| 153 | SLLsLHVDL | F | 179 | 187 | O14613 | Cdc42 effector protein 2 |
| 154 | SLMsGTLESL | F/S | 274 | 283 | Q4KMP7 | TBC1 domain family member 10B |
| 155 | SLQPRSHsV | S | 448 | 456 | Q9Y2H5 | Pleckstrin homology domain-containing family A member 6 |
| 156 | SLQsLETSV | S | 1233 | 1241 | P23634 | Plasma membrane calcium-transporting ATPase 4 |
| 157 | SLSsLLVKL | S | 1636 | 1644 | O15078 | Centrosomal protein of 290 kDa |
| 158 | SLVDGyFRL | F | 407 | 415 | P23458 | Tyrosine-protein kinase JAK1 |
| 159 | SMLsQEIQTL | S | 192 | 201 | Q9UHY8 | Fasciculation and elongation protein zeta-2 |
| 160 | SMSsLSREV | S | 2117 | 2125 | O15027 | Protein transport protein Sec16A |
| 161 | SMTRsPPRV | F/S | 248 | 256 | Q9BRL6 | Serine/arginine-rich splicing factor 8 |
| 162 | SPRssQLV | F | 538 | 545 | P32519 | ETS-related transcription factor Elf-1 |
| 163 | sPTRPNPPVRNLH | F | 522 | 534 | Q14195 | Dihydropyrimidinase-related protein 3 |
| 164 | SQIsPKSWGV | S | 563 | 571 | Q6IMN6 | Caprin-2 |
| 165 | STMsLNIITV | S | 243 | 252 | P54792 | Segment polarity protein dishevelled homolog DVL-1-like |
| 166 | sTMSLNIITV | S | 243 | 252 | P54792 | Segment polarity protein dishevelled homolog DVL-1-like |
| 167 | SVFsPSFGL | F/S | 1473 | 1481 | Q02880 | DNA topoisomerase 2-beta |
| 168 | SVGsDYYIQL | S | 546 | 555 | Q8IWU2 | Serine/threonine-protein kinase LMTK2 |
| 169 | SVLsPSFQL | F | 72 | 80 | Q12968 | Nuclear factor of activated T-cells, cytoplasmic 3 |
| 170 | SVMDsPKKL | F | 143 | 151 | Q8TBB0 | THAP domain-containing protein 6 |
| 171 | SVYsGDFGNLEV | S | 617 | 628 | Q9HCH5 | Synaptotagmin-like protein 2 |
| 172 | TLSsPPPGL | S | 2324 | 2332 | 095613 | Pericentrin |
| 173 | TMMsPSQFL | F | 520 | 528 | Q9ULH7 | MKL/myocardin-like protein 2 |
| 174 | TVMsNSSVIHL | S | 389 | 399 | Q7L7X3 | Serine/threonine-protein kinase TAO1 |
| 175 | VIDsQELSKV | S | 260 | 269 | P10451 | Osteopontin |
| 176 | VLFsSPPQM | F | 67 | 75 | P33991 | DNA replication licensing factor MCM4 |
| 177 | VLFSsPPQM | F | 67 | 75 | P33991 | DNA replication licensing factor MCM4 |
| 178 | VLSSLtPAKV | S | 559 | 568 | Q13330 | Metastasis-associated protein MTA1 |
| 179 | VMFRtPLASV | S | 319 | 328 | Q9UKT4 | F-box only protein 5 |
| 180 | VMIGsPKKV | F/S | 1437 | 1445 | Q68CZ2 | Tensin-3 |
| 181 | YAYDGKDyI | S | 140 | 148 | P18464 | HLA class I histocompatibility antigen, B-51 alpha chain |
| 182 | YLAsLEKKL | F | 77 | 85 | Q9BV29 | Uncharacterized protein C15orf57 |
| 183 | YLDsGIHSG | S | 30 | 38 | P35222 | Catenin beta-1 |
| 184 | YLDsGIHSGA | S | 30 | 39 | P35222 | Catenin beta-1 |
| 185 | yLGLDVPV | S | 1248 | 1255 | P04626 | Receptor tyrosine-protein kinase erbB-2 |
| 186 | YLGsISTLVTL | S | 498 | 508 | Q76N32 | Centrosomal protein of 68 kDa |
| 187 | YLIHsPMSL | S | 114 | 122 | P42330 | Aldo-keto reductase family 1 member C3 |
| 188 | YLLsPLNTL | F | 442 | 450 | Q8TF76 | Serine/threonine-protein kinase haspin |
| 189 | yLQSRYYRA | F | 359 | 367 | Q9H422 | Homeodomain-interacting protein kinase 3 |
| 190 | YLQsRYYRA | F/S | 359 | 367 | Q9H422 | Homeodomain-interacting protein kinase 3 |
| 191 | YLSDsDTEAKL | S | 1708 | 1718 | Q92614 | Unconventional myosin-XVIIIa |
| 192 | YQLsPTKLPSI | S | 429 | 439 | O60934 | Nibrin |
| 193 | YTAGtPYKV | S | 103 | 111 | Q92567 | Protein FAM168A |

| | | | | | | |
|---|---|---|---|---|---|---|
| Column 2: Phosphopeptide sequences; pSer, pThr and pTyr are specified by s, t, and y, respectively. * = Cysteinylated Column 3: S = SKOV3 Cells: F = FHIOSE Cells Column 4 & 5: Entries define the location of the phosphopeptides within the sequence of the parent protein. Column 6: Protein identifier in the UniProt biosequence database available on the World Wide Wide at the website uniprot<<dot>>org Column 7: Name of the protein in the UniProt biosequence database. | | | | | | |

The present invention in particular is directed to the following embodiments:
1. A composition comprising at least or about 1, 2, 3, 4, 5, 6, 7, 8, 9, 10, or more synthetic target peptides, wherein each synthetic target peptide:
   (i) is about or at least 8, 9, 10, 11, 12, 13, 14 or 15 amino acids long; and
   (ii) comprises an amino acid sequence as set forth in any of SEQ ID NOs: 1-193,
   and further wherein said composition optionally stimulates a T cell-mediated immune response to at least one of the synthetic target peptides.
2. The composition of embodiment 1, wherein at least one of the synthetic target peptides comprises a substitution of a serine residue with a homo-serine residue.
3. The composition of embodiment 1, wherein at least one of the synthetic target peptides is a phosphopeptide that comprises a non-hydrolyzable phosphate group.
4. The composition of embodiment 1, wherein the composition is immunologically suitable for at least 60 to 88% of ovarian cancer patients.
5. The composition of embodiment 1, wherein the composition comprises at least 5 different target peptides.
6. The composition of embodiment 1, wherein the composition comprises at least 10 different target peptides.
7. The composition of embodiment 1, wherein the composition comprises at least 15 different target peptides.
8. The composition of embodiment 1, wherein at least one of the synthetic target peptides is capable of binding to an MHC class I molecule of the HLA-A*0201 allele.
9. The composition of embodiment 1, wherein the composition is capable of increasing the 5-year survival rate of ovarian cancer patients treated with the composition by at least 20 percent relative to average 5-year survival rates that could have been expected without treatment with the composition.
10. The composition of embodiment 1, wherein the composition is capable of increasing the survival rate of ovarian cancer patients treated with the composition by at least 20 percent relative to a survival rate that could have been expected without treatment with the composition.
11. The composition of embodiment 1, wherein the composition is capable of increasing the treatment response rate of ovarian cancer patients treated with the composition by at least 20 percent relative to a treatment rate that could have been expected without treatment with the composition.
12. The composition of embodiment 1, wherein the composition is capable of increasing the overall median survival of patients of ovarian cancer patients treated with the composition by at least two months relative to an overall median survival that could have been expected without treatment with the composition.
13. The composition of embodiment 1, further comprising at least one peptide derived from MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein/cyclophilin C-associated protein), TAAL6, TAG72, TLP, and TPS.
14. The composition of embodiment 1, wherein the composition further comprises an adjuvant selected from the group consisting of montanide ISA-51, QS-21, a tetanus helper peptide, GM-CSF, cyclophosamide, bacillus Calmette-Guerin (BCG), corynbacterium parvum, levamisole, azimezone, isoprinisone, dinitrochlorobenezene (DNCB), keyhole limpet hemocyanin (KLH), complete Freunds adjuvant, in complete Freunds adjuvant, a mineral gel, aluminum hydroxide (Alum), lysolecithin, a pluronic polyol, a polyanion, an adjuvant peptide, an oil emulsion, dinitrophenol, and diphtheria toxin (DT), or any combination thereof.
15. An *in vitro* population of dendritic cells comprising the composition of any one of embodiments 1-14 or a composition comprising at least one target peptide comprising an amino acid sequence as set forth in any of SEQ ID NOs: 1-193.
16. An *in vitro* population of CD8⁺ T cells capable of being activated upon being brought into contact with a population of dendritic cells, wherein the dendritic cells comprise a composition of any one of embodiments 1-14 or a composition comprising at least one target peptide comprising an amino acid sequence as set forth in any of SEQ ID NOs: 1-193.
17. An antibody or antibody-like molecule that specifically binds to a complex of an MHC class I molecule and a peptide comprising an amino acid sequence as set forth in one or more of SEQ ID NOs: 1-193.
18. The antibody or antibody-like molecule of embodiment 17, wherein the antibody or antibody-like molecule is a member of the immunoglobulin superfamily.
19. The antibody or antibody-like molecule of embodiment 17, wherein the antibody or antibody-like molecule comprises a binding member selected from the group consisting an Fab, Fab', F(ab')₂, Fv, and a single-chain antibody.
20. The antibody or antibody-like molecule of embodiment 17 conjugated to a therapeutic agent selected from the group consisting of an alkylating agent, an antimetabolite, a mitotic inhibitor, a taxoid, a vinca alkaloid, and an antibiotic.
21. The antibody or antibody-like molecule of embodiment 17, wherein the antibody or antibody-like molecule is a T cell receptor, optionally conjugated to a CD3 agonist.
22. An *in vitro* population of T cells transfected with a nucleic acid encoding a T cell receptor of embodiment 21.
23. A method for treating and/or preventing cancer comprising administering to a subject in need thereof a therapeutically effective dose of a composition of any of embodiments 1-14 or a composition comprising at least one target peptide comprising an amino acid sequence as set forth in any of SEQ ID NOs: 1-193.
24. A method of treating and/or preventing ovarian cancer comprising administering to a subject in need thereof a therapeutically effective dose of a composition of any of embodiments 1-14 or a composition comprising at least one target peptide in combination with a pharmaceutically acceptable carrier.
25. A method for treating and/or preventing cancer comprising administering to a subject in need thereof a therapeutically effective dose of the CD8⁺ T cells of embodiment 16 in combination with a pharmaceutically acceptable carrier.
26. A method for treating and/or preventing cancer comprising administering to a subject in need thereof an *in vitro* population of dendritic cells of embodiment 15 in combination with a pharmaceutically acceptable carrier.
27. A method for treating and/or preventing cancer comprising administering to a subject in need thereof the population of CD8⁺ T cells of embodiment 16 in combination with a pharmaceutically acceptable carrier.
28. A method for making a cancer vaccine comprising combining the composition of any of embodiments 1-14 with an the adjuvant selected from the group consisting of montanide ISA-51, QS-21, a tetanus helper peptide, GM-CSF, cyclophosamide, bacillus Calmette-Guerin (BCG), corynbacterium parvum, levamisole, azimezone, isoprinisone, dinitrochlorobenezene (DNCB), keyhole limpet hemocyanin (KLH), complete Freunds adjuvant, in complete Freunds adjuvant, a mineral gel, aluminum hydroxide (Alum), lysolecithin, a pluronic polyol, a polyanion, an adjuvant peptide, an oil emulsion, dinitrophenol, and diphtheria toxin (DT), or any combination thereof and a pharmaceutically acceptable carrier; and placing the composition, adjuvant, and pharmaceutical carrier into a container, optionally into a syringe.
29. A method for screening target peptides for inclusion in an immunotherapy composition of embodiments 1-14 or for use in the method of using a composition of embodiments 1-14, comprising:
   (a) administering the target peptide to a human;
   (b) determining whether the target peptide is capable of inducing a target peptide-specific memory T cell response in the human; and
   (c) selecting the target peptide for inclusion in an immunotherapy composition if the target peptide elicits a memory T cell response in the human.
30. A method for determining a prognosis of an ovarian cancer patient, the method comprising:
   (a) administering to the patient a target peptide comprising an amino acid sequence as set forth in any of SEQ ID NOs: 1-193, wherein the target peptide is associated with the patient's ovarian cancer;
   (b) determining whether the target peptide is capable of inducing a target peptide-specific memory T cell response in the patient; and
   (c) determining that the patient has a better prognosis if the patient mounts a memory T cell response to the target peptide than if the patient did not mount a memory T cell response to the target peptide.
31. A kit comprising at least one target peptide composition comprising at least one target peptide comprising an amino acid sequence as set forth in any of SEQ ID NOs: 1-193 and a cytokine and/or an adjuvant.
32. The kit of embodiment 31, comprising at least 2, 3, 4, or 5 target peptide compositions.
33. The kit of embodiment 31, wherein the at least one target peptide composition is one of the compositions of embodiments 1-14.
34. The kit of embodiment 31, wherein the cytokine is selected from the group consisting of a transforming growth factor (TGF), optionally TGF-alpha and/or TGF-beta; insulin-like growth factor-I; insulin-like growth factor-II; erythropoietin (EPO); an osteoinductive factor; an interferon, optionally interferon-alpha, interferon-beta, and/or interferon-gamma; and a colony stimulating factor (CSF), optionally macrophage-CSF (M-CSF), granulocyte-macrophage-CSF (GM-CSF), and/or granulocyte-CSF (G-CSF).
35. The kit of embodiment 31, wherein the adjuvant is selected from the group consisting of montanide ISA-51, QS-21, a tetanus helper peptide, GM-CSF, cyclophosphamide, bacillus Calmette-Guerin (BCG), corynbacterium parvum, levamisole, azimezone, isoprinisone, dinitrochlorobenezene (DNCB), a keyhole limpet hemocyanin (KLH), complete Freund's adjuvant, incomplete Freund's adjuvant , a mineral gel, aluminum hydroxide, lysolecithin, a pluronic polyol, a polyanion, an adjuvant peptide, an oil emulsion, dinitrophenol, and diphtheria toxin (DT).
36. The kit of embodiment 31, wherein the cytokine is selected from the group consisting of a nerve growth factor, optionally nerve growth factor (NGF) beta; a platelet-growth factor; a transforming growth factor (TGF), optionally TGF-alpha and/or TGF-beta; insulin-like growth factor-I; insulin-like growth factor-II; erythropoietin (EPO); an osteoinductive factor; an interferon, optionally interferon-a, interferon-β, and/or interferon-y; a colony stimulating factor (CSF), optionally macrophage-CSF (M-CSF), granulocyte-macrophage-CSF (GM-CSF), and/or granulocyte-CSF (G-CSF); an interleukin (IL), optionally IL-1, IL-1α, IL-2, IL-3, IL-4, IL-5, IL-6, IL-7, IL-8, IL-9, IL-10, IL-11, IL-12; IL-13, IL-14, IL-15, IL-16, IL-17, and/or IL-18; LIF; EPO; kit-ligand; fms-related tyrosine kinase 3 (FLT-3; also called CD135); angiostatin; thrombospondin; endostatin; tumor necrosis factor; and lymphotoxin (LT).
37. The kit of embodiment 31, further comprising at least one peptide derived from MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, and TPS.
38. The kit of embodiment 31, wherein the at least one target peptide comprises an amino acid sequence as set forth in any of SEQ ID NOs: 1-193.
39. The composition of embodiment 1, comprising a peptide capable of binding to an MHC class I molecule of the HLA A*0201 allele.

## Claims

1. A composition comprising at least one or more synthetic target peptides, wherein each synthetic target peptide:
(i) is 8 to 50 amino acids long; and
(ii) comprises an amino acid sequence selected from the group consisting of:
SEQ ID NO: 89 wherein the serine at the fourth position is phosphorylated or replaced with a mimetic of phosphoserine;
SEQ ID NO: 94 wherein the serine at the fourth position is phosphorylated or replaced with a mimetic of phosphoserine;
SEQ ID NO: 1 wherein the serine at the fourth position is phosphorylated or replaced with a mimetic of phosphoserine;
SEQ ID NO: 2 wherein the serine at the fifth position is phosphorylated or replaced with a mimetic of phosphoserine;
SEQ ID NO: 4 wherein the threonine at the fifth position is phosphorylated or replaced with a mimetic of phosphothreonine;
SEQ ID NO: 5 wherein the serine at the fourth position is phosphorylated or replaced with a mimetic of phosphoserine;
SEQ ID NO: 8 wherein the serine at the fourth position is phosphorylated or replaced with a mimetic of phosphoserine;
SEQ ID NO: 10 wherein the threonine at the fourth position is phosphorylated or replaced with a mimetic of phosphothreonine;
SEQ ID NO: 11 wherein the threonine at the sixth position is phosphorylated or replaced with a mimetic of phosphothreonine;
SEQ ID NO: 12 wherein the serine at the fifth position is phosphorylated or replaced with a mimetic of phosphoserine;
SEQ ID NO: 13 wherein the serine at the seventh position is phosphorylated or replaced with a mimetic of phosphoserine;
SEQ ID NO: 14 wherein the serine at the fourth position is phosphorylated or replaced with a mimetic of phosphoserine;
SEQ ID NO: 15 wherein the serine at the fifth position is phosphorylated or replaced with a mimetic of phosphoserine;
SEQ ID NO: 16 wherein the threonine at the seventh position is phosphorylated or replaced with a mimetic of phosphothreonine;
SEQ ID NO: 17 wherein the serine at the fourth position is phosphorylated or replaced with a mimetic of phosphoserine;
SEQ ID NO: 18 wherein the serine at the eighth position is phosphorylated or replaced with a mimetic of phosphoserine;
SEQ ID NO: 19 wherein the serine at the eighth position is phosphorylated or replaced with a mimetic of phosphoserine;
SEQ ID NO: 20 wherein the serine at the tenth position is phosphorylated or replaced with a mimetic of phosphoserine;
SEQ ID NO: 22 wherein the serine at the fourth position is phosphorylated or replaced with a mimetic of phosphoserine;
SEQ ID NO: 23 wherein the threonine at the fifth position is phosphorylated or replaced with a mimetic of phosphothreonine;
SEQ ID NO: 24 wherein the serine at the fourth position is phosphorylated or replaced with a mimetic of phosphoserine;
SEQ ID NO: 25 wherein the serine at the eighth position is phosphorylated or replaced with a mimetic of phosphoserine;
SEQ ID NO: 26 wherein the serine at the fourth position is phosphorylated or replaced with a mimetic of phosphoserine;
SEQ ID NO: 27 wherein the serine at the fourth position is phosphorylated or replaced with a mimetic of phosphoserine;
SEQ ID NO: 28 wherein the serine at the fourth position is phosphorylated or replaced with a mimetic of phosphoserine;
SEQ ID NO: 29 wherein the threonine at the fourth position is phosphorylated or replaced with a mimetic of phosphothreonine;
SEQ ID NO: 30 wherein the serine at the fifth position is phosphorylated or replaced with a mimetic of phosphoserine;
SEQ ID NO: 31 wherein the serine at the fifth position is phosphorylated or replaced with a mimetic of phosphoserine;
SEQ ID NO: 33 wherein the serine at the fourth position is phosphorylated or replaced with a mimetic of phosphoserine;
SEQ ID NO: 34 wherein the serine at the fourth position is phosphorylated or replaced with a mimetic of phosphoserine;
SEQ ID NO: 35 wherein the serine at the fourth position is phosphorylated or replaced with a mimetic of phosphoserine;
SEQ ID NO: 36 wherein the serine at the second position is phosphorylated or replaced with a mimetic of phosphoserine;
SEQ ID NO: 37 wherein the serine at the fourth position is phosphorylated or replaced with a mimetic of phosphoserine;
SEQ ID NO: 38 wherein the serine at the fourth position is phosphorylated or replaced with a mimetic of phosphoserine;
SEQ ID NO: 39 wherein the serine at the fourth position is phosphorylated or replaced with a mimetic of phosphoserine;
SEQ ID NO: 40 wherein the threonine at the fourth position is phosphorylated or replaced with a mimetic of phosphothreonine;
SEQ ID NO: 41 wherein the serine at the fifth position is phosphorylated or replaced with a mimetic of phosphoserine;
SEQ ID NO: 42 wherein the serine at the fourth position is phosphorylated or replaced with a mimetic of phosphoserine;
SEQ ID NO: 44 wherein the serine at the fourth position is phosphorylated or replaced with a mimetic of phosphoserine;
SEQ ID NO: 45 wherein the serine at the fourth position is phosphorylated or replaced with a mimetic of phosphoserine;
SEQ ID NO: 46 wherein the serine at the fourth position is phosphorylated or replaced with a mimetic of phosphoserine;
SEQ ID NO: 47 wherein the serine at the fourth position is phosphorylated or replaced with a mimetic of phosphoserine;
SEQ ID NO: 48 wherein the serine at the fourth position is phosphorylated or replaced with a mimetic of phosphoserine;
SEQ ID NO: 53 wherein the serine at the fourth position is phosphorylated or replaced with a mimetic of phosphoserine;
SEQ ID NO: 54 wherein the serine at the fourth position is phosphorylated or replaced with a mimetic of phosphoserine;
SEQ ID NO: 59 wherein the threonine at the eighth position is phosphorylated or replaced with a mimetic of phosphothreonine;
SEQ ID NO: 60 wherein the serine at the fourth position is phosphorylated or replaced with a mimetic of phosphoserine;
SEQ ID NO: 61 wherein the serine at the fourth position is phosphorylated or replaced with a mimetic of phosphoserine;
SEQ ID NO: 62 wherein the serine at the fourth position is phosphorylated or replaced with a mimetic of phosphoserine;
SEQ ID NO: 63 wherein the serine at the sixth position is phosphorylated or replaced with a mimetic of phosphoserine;
SEQ ID NO: 64 wherein the serine at the fourth position is phosphorylated or replaced with a mimetic of phosphoserine;
SEQ ID NO: 66 wherein the threonine at the fifth position is phosphorylated or replaced with a mimetic of phosphothreonine;
SEQ ID NO: 67 wherein the serine at the fourth position is phosphorylated or replaced with a mimetic of phosphoserine;
SEQ ID NO: 68 wherein the serine at the fourth position is phosphorylated or replaced with a mimetic of phosphoserine;
SEQ ID NO: 69 wherein the threonine at the fifth position is phosphorylated or replaced with a mimetic of phosphothreonine;
SEQ ID NO: 70 wherein the serine at the fourth position is phosphorylated or replaced with a mimetic of phosphoserine;
SEQ ID NO: 71 wherein the serine at the fourth position is phosphorylated or replaced with a mimetic of phosphoserine;
SEQ ID NO: 72 wherein the serine at the fourth position is phosphorylated or replaced with a mimetic of phosphoserine;
SEQ ID NO: 73 wherein the serine at the fourth position is phosphorylated or replaced with a mimetic of phosphoserine;
SEQ ID NO: 74 wherein the serine at the fourth position is phosphorylated or replaced with a mimetic of phosphoserine;
SEQ ID NO: 75 wherein the serine at the fourth position is phosphorylated or replaced with a mimetic of phosphoserine;
SEQ ID NO: 76 wherein the serine at the fourth position is phosphorylated or replaced with a mimetic of phosphoserine;
SEQ ID NO: 77 wherein the serine at the fourth position is phosphorylated or replaced with a mimetic of phosphoserine;
SEQ ID NO: 78 wherein the serine at the fourth position is phosphorylated or replaced with a mimetic of phosphoserine;
SEQ ID NO: 80 wherein the serine at the fourth position is phosphorylated or replaced with a mimetic of phosphoserine;
SEQ ID NO: 81 wherein the serine at the fourth position is phosphorylated or replaced with a mimetic of phosphoserine;
SEQ ID NO: 84 wherein the threonine at the fourth position is phosphorylated or replaced with a mimetic of phosphothreonine;
SEQ ID NO: 85 wherein the serine at the fourth position is phosphorylated or replaced with a mimetic of phosphoserine;
SEQ ID NO: 87 wherein the serine at the sixth position is phosphorylated or replaced with a mimetic of phosphoserine;
SEQ ID NO: 88 wherein the serine at the fourth position is phosphorylated or replaced with a mimetic of phosphoserine;
SEQ ID NO: 90 wherein the serine at the fourth position is phosphorylated or replaced with a mimetic of phosphoserine;
SEQ ID NO: 91 wherein the serine at the fourth position is phosphorylated or replaced with a mimetic of phosphoserine;
SEQ ID NO: 92 wherein the serine at the fourth position is phosphorylated or replaced with a mimetic of phosphoserine;
SEQ ID NO: 93 wherein the serine at the fourth position is phosphorylated or replaced with a mimetic of phosphoserine;
SEQ ID NO: 96 wherein the serine at the fourth position is phosphorylated or replaced with a mimetic of phosphoserine;
SEQ ID NO: 97 wherein the serine at the fourth position is phosphorylated or replaced with a mimetic of phosphoserine;
SEQ ID NO: 98 wherein the serine at the fourth position is phosphorylated or replaced with a mimetic of phosphoserine;
SEQ ID NO: 99 wherein the serine at the fourth position is phosphorylated or replaced with a mimetic of phosphoserine;
SEQ ID NO: 100 wherein the threonine at the fourth position is phosphorylated or replaced with a mimetic of phosphothreonine;
SEQ ID NO: 101 wherein the serine at the eighth position is phosphorylated or replaced with a mimetic of phosphoserine;
SEQ ID NO: 102 wherein the serine at the fourth position is phosphorylated or replaced with a mimetic of phosphoserine;
SEQ ID NO: 104 wherein the serine at the fourth position is phosphorylated or replaced with a mimetic of phosphoserine;
SEQ ID NO: 106 wherein the threonine at the fifth position is phosphorylated or replaced with a mimetic of phosphothreonine;
SEQ ID NO: 107 wherein the serine at the fourth position is phosphorylated or replaced with a mimetic of phosphoserine;
SEQ ID NO: 108 wherein the serine at the fourth position is phosphorylated or replaced with a mimetic of phosphoserine;
SEQ ID NO: 109 wherein the serine at the fourth position is phosphorylated or replaced with a mimetic of phosphoserine;
SEQ ID NO: 110 wherein the serine at the fourth position is phosphorylated or replaced with a mimetic of phosphoserine;
SEQ ID NO: 111 wherein the serine at the fourth position is phosphorylated or replaced with a mimetic of phosphoserine;
SEQ ID NO: 115 wherein the serine at the fourth position is phosphorylated or replaced with a mimetic of phosphoserine;
SEQ ID NO: 116 wherein the serine at the fourth position is phosphorylated or replaced with a mimetic of phosphoserine;
SEQ ID NO: 118 wherein the serine at the fourth position is phosphorylated or replaced with a mimetic of phosphoserine;
SEQ ID NO: 119 wherein the serine at the fourth position is phosphorylated or replaced with a mimetic of phosphoserine;
SEQ ID NO: 120 wherein the serine at the fourth position is phosphorylated or replaced with a mimetic of phosphoserine;
SEQ ID NO: 121 wherein the serine at the fourth position is phosphorylated or replaced with a mimetic of phosphoserine;
SEQ ID NO: 122 wherein the serine at the fourth position is phosphorylated or replaced with a mimetic of phosphoserine;
SEQ ID NO: 123 wherein the serine at the fourth position is phosphorylated or replaced with a mimetic of phosphoserine;
SEQ ID NO: 124 wherein the serine at the fourth position is phosphorylated or replaced with a mimetic of phosphoserine;
SEQ ID NO: 128 wherein the serine at the fourth position is phosphorylated or replaced with a mimetic of phosphoserine;
SEQ ID NO: 130 wherein the serine at the fourth position is phosphorylated or replaced with a mimetic of phosphoserine;
SEQ ID NO: 131 wherein the serine at the fourth position is phosphorylated or replaced with a mimetic of phosphoserine;
SEQ ID NO: 132 wherein the serine at the fourth position is phosphorylated or replaced with a mimetic of phosphoserine;
SEQ ID NO: 134 wherein the serine at the fourth position is phosphorylated or replaced with a mimetic of phosphoserine;
SEQ ID NO: 135 wherein the serine at the fourth position is phosphorylated or replaced with a mimetic of phosphoserine;
SEQ ID NO: 136 wherein the serine at the fourth position is phosphorylated or replaced with a mimetic of phosphoserine;
SEQ ID NO: 137 wherein the serine at the fifth position is phosphorylated or replaced with a mimetic of phosphoserine;
SEQ ID NO: 138 wherein the serine at the fourth position is phosphorylated or replaced with a mimetic of phosphoserine;
SEQ ID NO: 139 wherein the serine at the fourth position is phosphorylated or replaced with a mimetic of phosphoserine;
SEQ ID NO: 140 wherein the serine at the fourth position is phosphorylated or replaced with a mimetic of phosphoserine;
SEQ ID NO: 141 wherein the threonine at the fifth position is phosphorylated or replaced with a mimetic of phosphothreonine;
SEQ ID NO: 142 wherein the threonine at the third position is phosphorylated or replaced with a mimetic of phosphothreonine;
SEQ ID NO: 143 wherein the serine at the fourth position is phosphorylated or replaced with a mimetic of phosphoserine;
SEQ ID NO: 144 wherein the serine at the eighth position is phosphorylated or replaced with a mimetic of phosphoserine;
SEQ ID NO: 145 wherein the serine at the fourth position is phosphorylated or replaced with a mimetic of phosphoserine;
SEQ ID NO: 146 wherein the serine at the sixth position is phosphorylated or replaced with a mimetic of phosphoserine;
SEQ ID NO: 147 wherein the serine at the eighth position is phosphorylated or replaced with a mimetic of phosphoserine;
SEQ ID NO: 148 wherein the serine at the fourth position is phosphorylated or replaced with a mimetic of phosphoserine;
SEQ ID NO: 149 wherein the serine at the fourth position is phosphorylated or replaced with a mimetic of phosphoserine;
SEQ ID NO: 150 wherein the serine at the fourth position is phosphorylated or replaced with a mimetic of phosphoserine;
SEQ ID NO: 151 wherein the serine at the fifth position is phosphorylated or replaced with a mimetic of phosphoserine;
SEQ ID NO: 152 wherein the serine at the eighth position is phosphorylated or replaced with a mimetic of phosphoserine;
SEQ ID NO: 153 wherein the serine at the fourth position is phosphorylated or replaced with a mimetic of phosphoserine;
SEQ ID NO: 154 wherein the serine at the fourth position is phosphorylated or replaced with a mimetic of phosphoserine;
SEQ ID NO: 156 wherein the serine at the fourth position is phosphorylated or replaced with a mimetic of phosphoserine;
SEQ ID NO: 157 wherein the serine at the fourth position is phosphorylated or replaced with a mimetic of phosphoserine;
SEQ ID NO: 158 wherein the tyrosine at the sixth position is phosphorylated or replaced with a mimetic of phosphotyrosine;
SEQ ID NO: 159 wherein the serine at the fourth position is phosphorylated or replaced with a mimetic of phosphoserine;
SEQ ID NO: 160 wherein the serine at the fourth position is phosphorylated or replaced with a mimetic of phosphoserine;
SEQ ID NO: 162 wherein the serine at the fourth position is phosphorylated or replaced with a mimetic of phosphoserine, the serine at the fifth position is phosphorylated or replaced with a mimetic of phosphoserine, or any combination thereof;
SEQ ID NO: 163 wherein the serine at the first position is phosphorylated or replaced with a mimetic of phosphoserine;
SEQ ID NO: 164 wherein the serine at the fourth position is phosphorylated or replaced with a mimetic of phosphoserine;
SEQ ID NO: 165 wherein the serine at the fourth position is phosphorylated or replaced with a mimetic of phosphoserine;
SEQ ID NO: 166 wherein the serine at the first position is phosphorylated or replaced with a mimetic of phosphoserine;
SEQ ID NO: 167 wherein the serine at the fourth position is phosphorylated or replaced with a mimetic of phosphoserine;
SEQ ID NO: 168 wherein the serine at the fourth position is phosphorylated or replaced with a mimetic of phosphoserine;
SEQ ID NO: 169 wherein the serine at the fourth position is phosphorylated or replaced with a mimetic of phosphoserine;
SEQ ID NO: 170 wherein the serine at the fifth position is phosphorylated or replaced with a mimetic of phosphoserine;
SEQ ID NO: 171 wherein the serine at the fourth position is phosphorylated or replaced with a mimetic of phosphoserine;
SEQ ID NO: 172 wherein the serine at the fourth position is phosphorylated or replaced with a mimetic of phosphoserine;
SEQ ID NO: 173 wherein the serine at the fourth position is phosphorylated or replaced with a mimetic of phosphoserine;
SEQ ID NO: 174 wherein the serine at the fourth position is phosphorylated or replaced with a mimetic of phosphoserine;
SEQ ID NO: 175 wherein the serine at the fourth position is phosphorylated or replaced with a mimetic of phosphoserine;
SEQ ID NO: 176 wherein the serine at the fourth position is phosphorylated or replaced with a mimetic of phosphoserine;
SEQ ID NO: 177 wherein the serine at the fifth position is phosphorylated or replaced with a mimetic of phosphoserine;
SEQ ID NO: 178 wherein the threonine at the sixth position is phosphorylated or replaced with a mimetic of phosphothreonine;
SEQ ID NO: 181 wherein the tyrosine at the eighth position is phosphorylated or replaced with a mimetic of phosphotyrosine;
SEQ ID NO: 182 wherein the serine at the fourth position is phosphorylated or replaced with a mimetic of phosphoserine;
SEQ ID NO: 183 wherein the serine at the fourth position is phosphorylated or replaced with a mimetic of phosphoserine;
SEQ ID NO: 185 wherein the tyrosine at the first position is phosphorylated or replaced with a mimetic of phosphotyrosine;
SEQ ID NO: 186 wherein the serine at the fourth position is phosphorylated or replaced with a mimetic of phosphoserine;
SEQ ID NO: 187 wherein the serine at the fifth position is phosphorylated or replaced with a mimetic of phosphoserine;
SEQ ID NO: 188 wherein the serine at the fourth position is phosphorylated or replaced with a mimetic of phosphoserine;
SEQ ID NO: 189 wherein the tyrosine at the first position is phosphorylated or replaced with a mimetic of phosphotyrosine;
SEQ ID NO: 190 wherein the serine at the fourth position is phosphorylated or replaced with a mimetic of phosphoserine;
SEQ ID NO: 191 wherein the serine at the fifth position is phosphorylated or replaced with a mimetic of phosphoserine; and
SEQ ID NO: 193 wherein the threonine at the fifth position is phosphorylated or replaced with a mimetic of phosphothreonine.

2. The composition of claim 1, wherein:
(i) at least one of the synthetic target peptides comprises a substitution of a serine residue with a homo-serine residue; or
(ii) at least one of the synthetic target peptides is a phosphopeptide that comprises a non-hydrolyzable phosphate group, and optionally wherein:
(iii) the composition comprises at least 5, at least 10 or at least 15 different target peptides; or
(iv) at least one of the synthetic target peptides is capable of binding to an MHC class I molecule of the HLA-A*0201 allele, and optionally further comprising:
(v) at least one peptide derived from MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, pl85erbB2, p180erbB-3, c-met, nm-23H1, PSA, TAG-72-4, CA 19¬9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein/cyclophilin C-associated protein), TAAL6, TAG72, TLP, and TPS; or
(vi) an adjuvant selected from the group consisting of montanide ISA-51, QS-21, a tetanus helper peptide, GM-CSF, cyclophosamide, bacillus Calmette-Guerin (BCG), corynbacterium parvum, levamisole, azimezone, isoprinisone, dinitrochlorobenezene (DNCB), keyhole limpet hemocyanin (KLH), complete Freunds adjuvant, incomplete Freunds adjuvant, a mineral gel, aluminum hydroxide (Alum), lysolecithin, a pluronic polyol, a polyanion, an adjuvant peptide, an oil emulsion, dinitrophenol, and diphtheria toxin (DT), or any combination thereof.

3. An *in vitro* population of dendritic cells comprising the composition of claim 1 or 2.

4. An *in vitro* population of CD8⁺ T cells capable of being activated upon being brought into contact with a population of dendritic cells, wherein the dendritic cells comprise a composition of claim 1 or 2.

5. An antibody or antibody-like molecule that specifically binds to a complex of an MHC class I molecule and a target peptide as set forth in claim 1.

6. The antibody or antibody-like molecule of claim 5, wherein the antibody or antibody-like molecule is a member of the immunoglobulin superfamily; wherein the antibody or antibody-like molecule comprises a binding member selected from the group consisting an Fab, Fab', F(ab')₂, Fv, and a single-chain antibody; or wherein the antibody or antibody-like molecule is conjugated to a therapeutic agent selected from the group consisting of an alkylating agent, an antimetabolite, a mitotic inhibitor, a taxoid, a vinca alkaloid, and an antibiotic.

7. The antibody or antibody-like molecule of claim 5, wherein the antibody or antibody-like molecule is a T cell receptor, optionally conjugated to a CD3 agonist.

8. An *in vitro* population of T cells transfected with a nucleic acid encoding a T cell receptor of claim 7.

9. A therapeutically effective amount of a composition of claim 1 or 2 for use in a method of treating and/or preventing cancer, optionally ovarian cancer.

10. A composition comprising a therapeutically effective dose of the CD8⁺ T cells of claim 4 in combination with a pharmaceutically acceptable carrier for use in a method for treating and/or preventing cancer.

11. A composition comprising a therapeutically effective amount of an *in vitro* population of dendritic cells of claim 3 in combination with a pharmaceutically acceptable carrier for use in a method for treating and/or preventing cancer.

12. A method for making a cancer vaccine comprising combining the composition of claim 1 or 2 with an adjuvant selected from the group consisting of montanide ISA-51, QS-21, a tetanus helper peptide, GM-CSF, cyclophosamide, bacillus Calmette-Guerin (BCG), corynbacterium parvum, levamisole, azimezone, isoprinisone, dinitrochlorobenezene (DNCB), keyhole limpet hemocyanin (KLH), complete Freunds adjuvant, incomplete Freunds adjuvant, a mineral gel, aluminum hydroxide (Alum), lysolecithin, a pluronic polyol, a polyanion, an adjuvant peptide, an oil emulsion, dinitrophenol, and diphtheria toxin (DT), or any combination thereof and a pharmaceutically acceptable carrier; and placing the composition, adjuvant, and pharmaceutical carrier into a container, optionally into a syringe.

13. A composition of claim 1 or 2 for use in a method for determining a prognosis of an ovarian cancer patient, the method comprising:
(a) administering to the patient a target peptide as set forth in claim 1;
(b) determining whether the target peptide is capable of inducing a target peptide-specific memory T cell response in the patient; and
(c) determining that the patient has a better prognosis if the patient mounts a memory T cell response to the target peptide than if the patient did not mount a memory T cell response to the target peptide.

14. A kit comprising at least one target peptide composition and a cytokine and/or an adjuvant, wherein the at least one target peptide composition is the composition of claim 1 or 2.

15. The kit of claim 14, wherein:
(i) the cytokine is selected from the group consisting of a transforming growth factor (TGF), optionally TGF-alpha and/or TGF-beta; insulin-like growth factor-I; insulin-like growth factor-II; erythropoietin (EPO); an osteoinductive factor; an interferon, optionally interferon-alpha, interferon-beta, and/or interferon-gamma; and a colony stimulating factor (CSF), optionally macrophage-CSF (M-CSF), granulocyte-macrophage-CSF (GM-CSF), and/or granulocyte-CSF (G-CSF); and/or
(ii) the adjuvant is selected from the group consisting of montanide ISA-51, QS-21, a tetanus helper peptide, GM-CSF, cyclophosphamide, bacillus Calmette-Guerin (BCG), Corynbacterium parvum, levamisole, azimezone, isoprinisone, dinitrochlorobenezene (DNCB), a keyhole limpet hemocyanin (KLH), complete Freund's adjuvant, incomplete Freund's adjuvant, a mineral gel, aluminum hydroxide, lysolecithin, a pluronic polyol, a polyanion, an adjuvant peptide, an oil emulsion, dinitrophenol, and diphtheria toxin (DT); and further optionally wherein:
(iii) the cytokine is selected from the group consisting of a nerve growth factor, optionally nerve growth factor (NGF) beta; a platelet-growth factor; a transforming growth factor (TGF), optionally TGF-alpha and/or TGF-beta; insulin-like growth factor-I; insulin-like growth factor-II; erythropoietin (EPO); an osteoinductive factor; an interferon, optionally interferon-a, interferon-n, and/or interferon-y; a colony stimulating factor (CSF), optionally macrophage-CSF (M-CSF), granulocyte-macrophage-CSF (GM-CSF), and/or granulocyte-CSF (G-CSF); an interleukin (IL), optionally IL-1, IL-1α, IL-2, IL-3, IL-4, IL-5, IL-6, IL-7, IL-8, IL-9, IL-10, IL-11, IL-12; IL-13, IL-14, IL-15, IL-16, IL-17, and/or IL-18; LIF; EPO; kit-ligand; fms-related tyrosine kinase 3 (FLT-3; also called CD135); angiostatin; thrombospondin; endostatin; tumor necrosis factor; and lymphotoxin (LT); and/or
(iv) the kit further comprises at least one peptide derived from MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, n-Catenin, CDK4, Mum-1, p16, TAGE, PSMA, PSCA, CT7, telomerase, 43-9F, 5T4, 791Tgp72, alpha-fetoprotein, n-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, and TPS.
